(19) Europäisches Patentamt
European Patent Office
Office européen des brevets

(11) EP 3 640 244 A1

(12) **EUROPEAN PATENT APPLICATION**
published in accordance with Art. 153(4) EPC

(43) Date of publication:
**22.04.2020 Bulletin 2020/17**

(21) Application number: **18817141.7**

(22) Date of filing: **12.06.2018**

(51) Int Cl.:
*C07D 271/12* (2006.01) *A01N 43/836* (2006.01)
*A01P 7/02* (2006.01) *A01P 7/04* (2006.01)
*A61K 31/4245* (2006.01) *A61P 33/14* (2006.01)
*C07D 498/10* (2006.01)

(86) International application number:
**PCT/JP2018/022389**

(87) International publication number:
**WO 2018/230555 (20.12.2018 Gazette 2018/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.06.2017 JP 2017116852**
**28.06.2017 JP 2017125842**
**10.07.2017 JP 2017134971**
**02.08.2017 JP 2017149700**
**25.09.2017 JP 2017183870**
**16.10.2017 JP 2017200523**

(71) Applicant: **Nippon Soda Co., Ltd.**
**Tokyo 100-8165 (JP)**

(72) Inventors:
- **SHIBAYAMA Kotaro**
**Odawara-shi**
**Kanagawa 250-0280 (JP)**
- **SUZUKI Hiroto**
**Odawara-shi**
**Kanagawa 250-0280 (JP)**
- **IWASA Takao**
**Odawara-shi**
**Kanagawa 250-0280 (JP)**
- **HIRATA Koichi**
**Odawara-shi**
**Kanagawa 250-0280 (JP)**
- **KIYOTA Ryutaro**
**Odawara-shi**
**Kanagawa 250-0280 (JP)**

(74) Representative: **Wibbelmann, Jobst**
**Wuesthoff & Wuesthoff**
**Patentanwälte PartG mbB**
**Schweigerstrasse 2**
**81541 München (DE)**

# (54) OXADIAZOLINE COMPOUND AND PEST CONTROL AGENT

(57) The present invention provides a compound represented by formula (I) (in formula (I), $R^1$ represents a substituted or unsubstituted C1-6 alkyl group, $R^2$ represents a substituted or unsubstituted C1-8 alkyl group, $R^3$ represents a hydrogen atom or a substituted or unsubstituted C1-6 alkyl group, A represents a substituted or unsubstituted o-phenylene group, a substituted or unsubstituted bezylene group, or the like, B represents an oxy group, a substituted or unsubstituted oxymethylene group, or the like, and Q represents a substituted or unsubstituted o-phenylene group), or a salt thereof.

EP 3 640 244 A1

[Chem. 1]

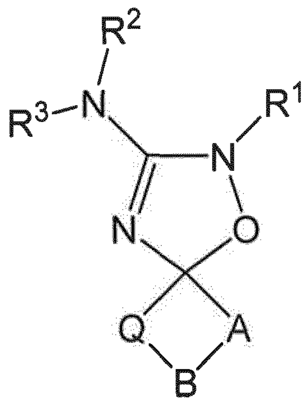

(I)

## Description

### TECHNICAL FIELD

The present invention relates to an oxadiazoline compound and a formulation for controlling harmful organisms. More particularly, the present invention relates to an oxadiazoline compound which has superior insecticidal activity and/or acaricidal activity, exhibits superior safety, and can be advantageously synthesized industrially, and also relates to a formulation for controlling harmful organisms, an insecticidal or acaricidal formulation, a formulation for controlling ectoparasites, or a formulation for controlling Thysanoptera insect pests, which contains the same as an active ingredient.

### BACKGROUND ART

Various compounds having an insecticidal and/or acaricidal activity have been proposed. In order to practically use such compounds as agrochemicals, the compounds are required not only to have a sufficient efficacy, but also to hardly cause chemical resistance, avoid phytotoxicity against plants or soil contamination, and have a low level of toxicity against livestock, fish or the like.

Patent Document 1 discloses compounds represented by Formula (O), and the like.

[Chem. 1]

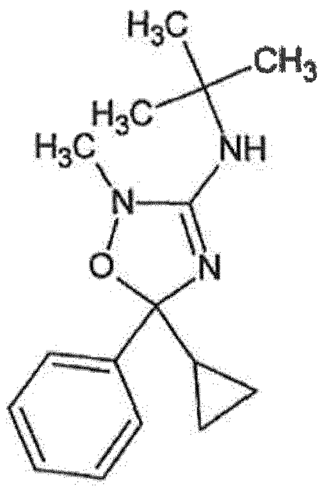

(O)

### PRIOR ART LITERATURE

#### Patent Documents

[Patent Document 1] PCT international Publication No. WO2017/093409 A

### DISCLOSURE OF INVENTION

#### Technical Problem

An object of the present invention is to provide an oxadiazoline compound which has superior activity for controlling harmful organisms, and in particular, superior insecticidal activity and/or acaricidal activity, exhibits superior safety, and can be advantageously synthesized industrially. Another object of the present invention is to provide a formulation for controlling harmful organisms, an insecticide or acaricide, a formulation for controlling ectoparasites, or a formulation for controlling Thysanoptera insect pests, which contains the same oxadiazoline compound as an active ingredient.

#### Solution to Problem

As a result of diligent studies in order to achieve the objects mentioned above, the inventors of the present

application completed the present invention including the following modes.

[1] A compound represented by formula (I) or a salt thereof

[Chem. 2]

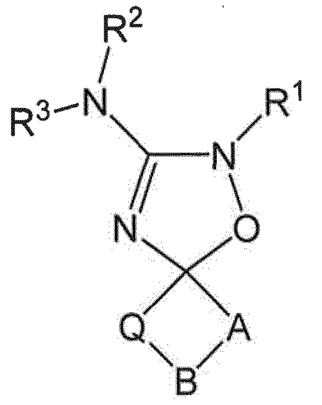

(I)

[in formula (I),

$R^1$ represents a hydrogen atom, a substituted or unsubstituted C1-6 alkyl group, a substituted or unsubstituted C2-6 alkenyl group, a substituted or unsubstituted C6-10 aryl C1-6 alkyl group, or a substituted or unsubstituted C1-6 alkyl carbonyl group,
$R^2$ represents a substituted or unsubstituted C1-8 alkyl group, a substituted or unsubstituted C3-10 cycloalkyl group, a substituted or unsubstituted C6-10 aryl group, a substituted or unsubstituted C6-10 aryl C1-6 alkyl group, or a substituted or unsubstituted C1-6 alkoxy carbonyl group,
$R^3$ represents a hydrogen atom, a substituted or unsubstituted C1-6 alkyl group, a substituted or unsubstituted C2-6 alkenyl group, a substituted or unsubstituted C2-6 alkynyl group, a substituted or unsubstituted C6-10 aryl C1-6 alkyl group, a substituted or unsubstituted C1-6 alkyl carbonyl group, or a substituted or unsubstituted C1-6 alkoxy carbonyl group,
$R^2$ and $R^3$ may bind together to form a substituted or unsubstituted C3-5 alkylene group,
A represents a substituted or unsubstituted o-phenylene group, a substituted or unsubstituted 5- to 6-membered heteroarylene group, a substituted or unsubstituted benzylene group, a substituted or unsubstituted dimethylene group, or a 1,2-cyclopropylene group,
B represents a single bond, an oxy group, a substituted or unsubstituted oxymethylene group, a substituted or unsubstituted methyleneoxy group, a substituted or unsubstituted thiomethylene group, a substituted or unsubstituted methylenethio group, a substituted or unsubstituted methylene group, a substituted or unsubstituted dimethylene group, or a substituted or unsubstituted vinylene group, a thio group, a substituted or unsubstituted sulfonylmethylene group, a substituted or unsubstituted methylenesulfonyl group, a substituted or unsubstituted trimethylene group, a substituted or unsubstituted oxyethylene group, a substituted or unsubstituted ethyleneoxy group, a substituted or unsubstituted propenylene group, a substituted or unsubstituted oxymethyleneoxy group, a group represented by formula: $-NR^a-$, a group represented by formula: $-CH_2-NR^a-$, or a group represented by formula: $-NR^a-CH_2-$,
$R^a$ represents a hydrogen atom or a substituted or unsubstituted C1-6 alkyl group, and
Q represents a substituted or unsubstituted o-phenylene group.]

[2] The compound according to the aforementioned [1], wherein A is a substituted or unsubstituted o-phenylene group, and B is a substituted or unsubstituted oxymethylene group, a substituted or unsubstituted methyleneoxy group, a substituted or unsubstituted dimethylene group, or a substituted or unsubstituted vinylene group.
[3] The compound according to the aforementioned [1], wherein A is a substituted or unsubstituted o-phenylene group, and B is a substituted or unsubstituted oxymethylene group, or a substituted or unsubstituted methyleneoxy group.
[4] A formulation for controlling harmful organisms, containing at least one compound selected from the group consisting of the compounds as recited in any one of [1] to [3] mentioned above, and salts thereof as an active ingredient.

[5] An insecticidal or acaricidal formulation, containing at least one compound selected from the group consisting of the compounds as recited in any one of [1] to [3] mentioned above, and salts thereof as an active ingredient.
[6] A formulation for controlling ectoparasites, containing at least one compound selected from the group consisting of the compounds as recited in any one of [1] to [3] mentioned above, and salts thereof as an active ingredient.
[7] A formulation for controlling Thysanoptera insect pests, containing at least one compound selected from the group consisting of the compounds as recited in any one of [1] to [3] mentioned above, and salts thereof as an active ingredient.

Advantageous Effects of the Invention

The oxadiazoline compounds of the present invention can control harmful organisms which are problematic in view of farm products or for hygiene reasons. The oxadiazoline compounds of the present invention can effectively control various types of agricultural pests and acari with a reduced concentration. In addition, the oxadiazoline compounds of the present invention can effectively control ectoparasites which harm humans and animals. The oxadiazoline compounds of the present invention can effectively control Thysanoptera insect pests.

EMBODIMENTS OF THE INVENTION

[Oxadiazoline compounds]

An oxadiazoline compound of the present invention is a compound represented by formula (I) (hereinafter, referred to as compound (I) in some cases), or a salt of compound (I).
The carbon atom at the 3$^{rd}$ position among the carbon atoms constituting an oxadiazoline ring is an asymmetric carbon atom. The optical isomers derived from the asymmetric carbon correspond to compounds (I) of the present invention regardless of a single isomer, or a mixture of a plurality of isomers.
In the present invention, the term "unsubstituted" means that only a group which is a mother nucleus is present. When only the name of a group as a mother nucleus is described without the term "substituted", it means "unsubstituted" unless otherwise specified.
On the other hand, the term "substituted (= having a substituent)" means that at least one hydrogen atom of a group as a mother nucleus is substituted with a group (substituent) having a structure which is the same as or different from the mother nucleus. Therefore, a "substituted group (substituent)" is another group which is bonded to the group as the mother nucleus. The substituted group may be one, or may be two or more. Two or more substituted groups may be the same as or different from each other.
The term "C1-6" represents that the number of carbon atoms of a group as a mother nucleus is 1 to 6. The number of carbon atoms does not include the number of carbon atoms present in a substituted group. For example, a butyl group having an ethoxy group as a substituted group is classified as a C2 alkoxy C4 alkyl group.
A "substituted group" is not particularly limited as long as it is chemically acceptable and has the effect of the present invention. Hereinafter, as examples of a group which can be a "substituted group", mention may be made of,
a C1-6 alkyl group such as a methyl group, an ethyl group, an n-propyl group, an i-propyl group, an n-butyl group, an s-butyl group, an i-butyl group, a t-butyl group, an n-pentyl group, or an n-hexyl group;
a C2-6 alkenyl group such as a vinyl group, a 1-propenyl group, a 2-propenyl group (an allyl group), a 1-butenyl group, a 2-butenyl group, a 3-butenyl group, a 1-methyl-2-propenyl group, or a 2-methyl-2-propenyl group;
a C2-6 alkynyl group such as an ethynyl group, a 1-propynyl group, a 2-propynyl group, a 1-butynyl group, a 2-butynyl group, a 3-butynyl group, or a 1-methyl-2-propynyl group;
a C3-8 cycloalkyl group such as a cyclopropyl group, a cyclobutyl group, a cyclopentyl group, a cyclohexyl group, or a cubanyl group;
a C6-10 aryl group such as a phenyl group, or a naphthyl group;
a C6-10 aryl C1-6 alkyl group such as a benzyl group, or a phenethyl group;
a 3- to 6-membered heterocyclyl group;
a 3- to 6-membered heterocyclyl C1-6 alkyl group;
a hydroxyl group;
a C1-6 alkoxy group such as a methoxy group, an ethoxy group, an n-propoxy group, an i-propoxy group, an n-butoxy group, an s-butoxy group, an i-butoxy group, or a t-butoxy group;
a C2-6 alkenyloxy group such as a vinyloxy group, an allyloxy group, a propenyloxy group, or a butenyloxy group;
a C2-6 alkynyloxy group such as an ethynyloxy group, or a propargyloxy group;
a C6-10 aryloxy group such as a phenoxy group, or a naphthoxy group;
a C6-10 aryl C1-6 alkoxy group such as a benzyloxy group, or a phenethyloxy group;
a 5- to 6-membered heteroaryloxy group such as a thiazolyloxy group, or a pyridyloxy group;

a 5- to 6-membered heteroaryl C1-6 alkyloxy group such as a thiazolyl methyloxy group, or a pyridyl methyloxy group;
a formyl group;
a C1-6 alkyl carbonyl group such as an acetyl group, or a propionyl group;
a formyloxy group;
a C1-6 alkyl carbonyloxy group such as an acetyloxy group, or a propionyloxy group;
a C6-10 aryl carbonyl group such as a benzoyl group;
a C1-6 alkoxy carbonyl group such as a methoxycarbonyl group, an ethoxycarbonyl group, an n-propoxycarbonyl group, an i-propoxycarbonyl group, an n-butoxycarbonyl group, or a t-butoxycarbonyl group;
a C1-6 alkoxy carbonyloxy group such as a methoxycarbonyloxy group, an ethoxycarbonyloxy group, an n-propoxycarbonyloxy group, an i-propoxycarbonyloxy group, an n-butoxycarbonyloxy group, or a t-butoxycarbonyloxy group;
a carboxyl group;
a halogeno group such as a fluoro group, a chloro group, a bromo group, or an iodo group;
a C1-6 haloalkyl group such as a chloromethyl group, a chloroethyl group, a trifluoromethyl group, a 1,2-dichloro-n-propyl group, a 1-fluoro-n-butyl group, or a perfluoro-n-pentyl group;
a C2-6 haloalkenyl group such as a 2-chloro-1-propenyl group, or a 2-fluoro-1-butenyl group;
a C2-6 haloalkynyl group such as a 4,4-dichloro-1-butynyl group, a 4-fluoro-1-pentynyl group, or a 5-bromo-2-pentynyl group;
a C1-6 haloalkoxy group such as a trifluoromethoxy group, a 2-chloro-n-propoxy group, or a 2,3-dichlorobutoxy group;
a C2-6 haloalkenyloxy group such as a 2-chloropropenyloxy group, or a 3-bromobutenyloxy group;
a C1-6 haloalkyl carbonyl group such as a chloroacetyl group, a trifluoroacetyl group, or a trichloroacetyl group;
an amino group;
a C1-6 alkyl substituted amino group such as a methylamino group, a dimethylamino group, or a diethylamino group;
a C6-10 aryl amino group such as an anilino group, or a naphthyl amino group;
a C6-10 aryl C1-6 alkyl amino group such as a benzylamino group, or a phenethyl amino group;
a formylamino group;
a C1-6 alkyl carbonylamino group such as an acetylamino group, a propanoylamino group, a butyrylamino group, or an i-propyl carbonyl amino group;
a C1-6 alkoxy carbonyl amino group such as a methoxycarbonyl amino group, an ethoxycarbonyl amino group, an n-propoxycarbonyl amino group, or an i-propoxycarbonyl amino group;
a substituted or unsubstituted aminocarbonyl group such as an aminocarbonyl group, a dimethyl aminocarbonyl group, a phenyl aminocarbonyl group, or an N-phenyl-N-methyl aminocarbonyl group;
an imino C1-6 alkyl group such as an iminomethyl group, a (1-imino)ethyl group, or a (1-imino)-n-propyl group;
a substituted or unsubstituted N-hydroxyimino C1-6 alkyl group such as an N-hydroxy-iminomethyl group, a (1-(N-hydroxy)-imino)ethyl group, a (1-(N-hydroxy)-imino)propyl group, an N-methoxy-iminomethyl group, or a (1-(N-methoxy)-imino)ethyl group;
an aminocarbonyloxy group;
a C1-6 alkyl-substituted aminocarbonyloxy group such as an ethyl aminocarbonyloxy group, or a dimethyl aminocarbonyloxy group;
a mercapto group;
a C1-6 alkylthio group such as a methylthio group, an ethylthio group, an n-propylthio group, an i-propylthio group, an n-butylthio group, an i-butylthio group, an s-butylthio group, or a t-butylthio group;
a C1-6 haloalkylthio group such as a trifluoromethylthio group, or a 2,2,2-trifluoroethylthio group;
a C6-10 arylthio group such as a phenylthio group, or a naphthylthio group;
a 5- to 6-membered heteroarylthio group such as a thiazolylthio group, or a pyridylthio group;
a C1-6 alkylsulfinyl group such as a methylsulfinyl group, an ethylsulfinyl group, or a t-butylsulfinyl group;
a C1-6 haloalkyl sulfinyl group such as a trifluoromethyl sulfinyl group, or a 2,2,2-trifluoroethyl sulfinyl group;
a C6-10 arylsulfinyl group such as a phenylsulfinyl group;
a 5- to 6-membered heteroarylsulfinyl group such as a thiazolylsulfinyl group, or a pyridylsulfinyl group;
a C1-6 alkylsulfonyl group such as a methylsulfonyl group, an ethylsulfonyl group, or a t-butylsulfonyl group;
a C1-6 haloalkylsulfonyl group such as a trifluoromethyl sulfonyl group, or a 2,2,2-trifluoroethyl sulfonyl group;
a C6-10 arylsulfonyl group such as a phenylsulfonyl group;
a 5- to 6-membered heteroaryl sulfonyl group such as a thiazolylsulfonyl group, or a pyridylsulfonyl group;
a C1-6 alkylsulfonyloxy group such as a methylsulfonyloxy group, an ethylsulfonyloxy group, or a t-butylsulfonyloxy group;
a C1-6 haloalkyl sulfonyloxy group such as a trifluoromethyl sulfonyloxy group, or a 2,2,2-trifluoroethyl sulfonyloxy group;
a tri C1-6 alkyl-substituted silyl group such as a trimethylsilyl group, a triethylsilyl group, or a t-butyldimethylsilyl group;
a tri C6-10 aryl-substituted silyl group such as a triphenylsilyl group;
a cyano group; and
a nitro group.

In addition, any of the hydrogen atoms in these "substituted groups" may be substituted with other substituted groups having a different structure. In this case, as examples of the "substituted groups", mention may be made of a C1-6 alkyl group, a C1-6 haloalkyl group, a C1-6 alkoxy group, a C1-6 haloalkoxy group, a halogeno group, a cyano group, a nitro group and the like.

In addition, the aforementioned "3- to 6-membered heterocyclyl group" is a group having 1 to 4 heteroatoms selected from the group consisting of a nitrogen atom, an oxygen atom and a sulfur atom as a constitutional atom of the ring. The heterocyclyl group may be a monocyclyl group or a polycyclyl group. As long as at least one ring is a hetero ring in the polyheterocyclyl group, the remaining ring may be a saturated alicyclic ring, an unsaturated alicyclic ring or an aromatic ring. As examples of the "3-to 6-membered heterocyclyl group", mention may be made of a 3- to 6-membered saturated heterocyclyl group, a 5- to 6-membered heteroaryl group, a 5- to 6-membered partially unsaturated heterocyclyl group, and the like.

As examples of the 3- to 6-membered saturated heterocyclyl group, mention may be made of an aziridinyl group, an epoxy group, a pyrrolidinyl group, a tetrahydrofuranyl group, a thiazolidinyl group, a piperidinyl group, a piperazinyl group, a morpholinyl group, a dioxolanyl group, a dioxanyl group, and the like.

As examples of the 5-membered heteroaryl group, mention may be made of a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group, and the like.

As examples of the 6-membered heteroaryl group, mention may be made of a pyridyl group, a pyrazinyl group, a pyrimidinyl group, a pyridazinyl group, a triazinyl group and the like.

[$R^1$]

$R^1$ in formula (I) represents a hydrogen atom, a substituted or unsubstituted C1-6 alkyl group, a substituted or unsubstituted C2-6 alkenyl group, a substituted or unsubstituted C6-10 aryl C1-6 alkyl group, or a substituted or unsubstituted C1-6 alkyl carbonyl group.

The "C1-6 alkyl group" of $R^1$ may be linear or branched in the case where the number of carbon atoms is 3 or more. As examples of the alkyl group, mention may be made of a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group, an i-hexyl group, and the like.

As examples of the preferable "substituted C1-6 alkyl group", mention may be made of a C1-6 haloalkyl group such as a fluoromethyl group, a chloromethyl group, a bromomethyl group, a difluoromethyl group, dichloromethyl group, a dibromomethyl group, a trifluoromethyl group, a trichloromethyl group, a tribromomethyl group, a 1-chloroethyl group, a 2,2,2-trifluoroethyl group, a 2,2,2-trichloroethyl group, a pentafluoroethyl group, a 4-fluorobutyl group, a 4-chlorobutyl group, a 3,3,3-trifluoropropyl group, a 2,2,2-trifluoro-1-trifluoromethylethyl group, a 1,1,1,3,3,3-hexafluoropropan-2-yl group, a perfluoropropan-2-yl group, a perfluorohexyl group, a perchlorohexyl group, a 2,4,6-trichlorohexyl group, or the like;

a hydroxy C1-6 alkyl group such as a hydroxymethyl group, a hydroxyethyl group, or the like;

a C1-6 alkoxy C1-6 alkyl group such as a methoxymethyl group, an ethoxymethyl group, a methoxyethyl group, an ethoxyethyl group, a methoxy-n-propyl group, an n-propoxymethyl group, an i-propoxyethyl group, an s-butoxymethyl group, a t-butoxyethyl group, or the like;

a C3-8 cycloalkyl C1-6 alkyl group such as a cyclopropylmethyl group, a 2-cyclopropylethyl group, a cyclopentylmethyl group, a 2-cyclohexylethyl group, a 2-cyclooctylethyl group, or the like; a C1-6 alkylthio C1-6 alkyl group such as a methylthiomethyl group, an ethylthioethyl group, or the like; a C1-6 alkoxycarbonyl C1-6 alkyl group such as a methoxycarbonylmethyl group, an ethoxycarbonylmethyl group, or the like; a substituted silyloxy C1-6 alkyl group such as a trimethylsilyloxymethyl, a t-butyldimethylsilyloxymethyl group, a t-butyldiphenylsilyloxymethyl group, or the like; and the like.

As examples of the preferable substituted group on the "C1-6 alkyl group" of $R^1$, mention may be made of a halogeno group such as a fluoro group, a chloro group, a bromo group, an iodo group or the like; a C1-6 alkoxy group such as a methoxy group, an ethoxy group, an n-propoxy group, an i-propoxy group, an n-butoxy group, an s-butoxy group, an i-butoxy group, a t-butoxy group, or the like; a C1-6 haloalkoxy group such as a 2-chloro-n-propoxy group, a 2,3-dichlorobutoxy group, a trifluoromethoxy group, or the like; a cyano group; and a C1-6 alkylthio group such as a methylthio group, an ethylthio group, or the like.

As examples of the "C2-6 alkenyl group" of $R^1$, mention may be made of a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, a 5-hexenyl group, and the like.

As examples of the preferable substituted group on the "C2-6 alkenyl group" of $R^1$, mention may be made of

a halogeno group such as a fluoro group, a chloro group, a bromo group, an iodo group or the like; a C1-6 alkoxy group such as a methoxy group, an ethoxy group, an n-propoxy group, an i-propoxy group, an n-butoxy group, an s-butoxy group, an i-butoxy group, a t-butoxy group, or the like; a C1-6 haloalkoxy group such as a 2-chloro-n-propoxy group, a 2,3-dichlorobutoxy group, a trifluoromethoxy group, or the like; and a cyano group.

As examples of the "C6-10 aryl C1-6 alkyl group" of $R^1$, mention may be made of a benzyl group, a phenethyl group, and the like.

As examples of the preferable substituted group on the "C6-10 aryl C1-6 alkyl group" of $R^1$, mention may be made of a halogeno group such as a fluoro group, a chloro group, a bromo group, an iodo group or the like; a C1-6 alkyl group such as a methyl group, an ethyl group, an n-propyl group, an i-propyl group, an n-butyl group, an s-butyl group, an i-butyl group, a t-butyl group, or he like; a C1-6 haloalkyl group such as a 2-chloro-n-propyl group, a 2,3-dichlorobutyl group, a trifluoromethyl group, or the like; a C1-6 alkoxy group such as a methoxy group, an ethoxy group, an n-propoxy group, an i-propoxy group, an n-butoxy group, an s-butoxy group, an i-butoxy group, a t-butoxy group, or the like; a C1-6 haloalkoxy group such as a 2-chloro-n-propoxy group, a 2,3-dichlorobutoxy group, a trifluoromethoxy group, or the like; a cyano group; and a nitro group.

As examples of the "C1-6 alkyl carbonyl group" of $R^1$, mention may be made of an acetyl group, a propionyl group, and the like.

As examples of the preferable substituted group on the "C1-6 alkyl carbonyl group" of $R^1$, mention may be made of a halogeno group such as a fluoro group, a chloro group, a bromo group, an iodo group or the like; a C1-6 alkoxy group such as a methoxy group, an ethoxy group, an n-propoxy group, an i-propoxy group, an n-butoxy group, an s-butoxy group, an i-butoxy group, a t-butoxy group, or the like; a C1-6 haloalkoxy group such as a 2-chloro-n-propoxy group, a 2,3-dichlorobutoxy group, a trifluoromethoxy group, or the like; and a cyano group.

[$R^2$]

$R^2$ in formula (I) represents a substituted or unsubstituted C1-8 alkyl group, a substituted or unsubstituted C3-10 cycloalkyl group, a substituted or unsubstituted C6-10 aryl group, a substituted or unsubstituted C6-10 aryl C1-6 alkyl group, or a substituted or unsubstituted C1-6 alkoxy carbonyl group.

As examples of the "substituted or unsubstituted C6-10 aryl C1-6 alkyl group" of $R^2$, the same examples as those particularly described in $R^1$ may be mentioned.

The "substituted or unsubstituted C1-8 alkyl group" of $R^2$ may be linear or branched in the case where the number of carbon atoms is 3 or more. As examples of the alkyl group, mention may be made of a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a t-pentyl group, a 2-methylbutyl group, a 2,2-dimethylpropyl group, an i-hexyl group, a 2-methylpentan-2-yl group, an n-heptyl group, an n-octyl group, a 2,4,4-trimethylpentan-2-yl group, and the like.

As examples of the preferable "substituted C1-8 alkyl group", mention may be made of a C1-8 haloalkyl group such as a fluoromethyl group, a chloromethyl group, a bromomethyl group, a difluoromethyl group, a dichloromethyl group, a dibromomethyl group, a trifluoromethyl group, a trichloromethyl group, a tribromomethyl group, a 1-chloroethyl group, a 2,2,2-trifluoroethyl group, a 2,2,2-trichloroethyl group, a pentafluoroethyl group, a 4-fluorobutyl group, a 4-chlorobutyl group, a 3,3,3-trifluoropropyl group, a 2,2,2-trifluoro-1-trifluoromethylethyl group, a 1,1,1,3,3,3-hexafluoropropan-2-yl group, a perfluoropropan-2-yl group, a perfluorohexyl group, a perchlorohexyl group, a 2,4,6-trichlorohexyl group, and the like;

a hydroxy C1-8 alkyl group such as a hydroxymethyl group, a hydroxyethyl group, or the like;

a C1-6 alkoxy C1-8 alkyl group such as a methoxymethyl group, an ethoxymethyl group, a methoxyethyl group, an ethoxyethyl group, a methoxy-n-propyl group, an n-propoxymethyl group, an i-propoxyethyl group, an s-butoxymethyl group, a t-butoxyethyl group, or the like;

a C3-8 cycloalkyl Cl-8 alkyl group such as a cyclopropylmethyl group, a 2-cyclopropylethyl group, a cyclopentylmethyl group, a 2-cyclohexylethyl group, a 2-cyclooctylethyl group, or the like; a C1-6 alkylthio C1-8 alkyl group such as a methylthiomethyl group, an ethylthioethyl group, or the like; a C1-6 alkoxy carbonyl C1-8 alkyl group such as a methoxycarbonylmethyl group, an ethoxycarbonylmethyl group, or the like; a substituted silyloxy C1-8 alkyl group such as a trimethylsilyloxymethyl, a t-butyldimethylsilyloxymethyl group, a t-butyldiphenylsilyloxymethyl group, or the like; and the like.

As examples of the preferable substituted group on the "C1-8 alkyl group" of $R^2$, mention may be made of a halogeno group such as a fluoro group, a chloro group, a bromo group, an iodo group or the like; a C1-6 alkoxy group such as a methoxy group, an ethoxy group, an n-propoxy group, an i-propoxy group, an n-butoxy group, an s-butoxy group, an i-butoxy group, a t-butoxy group, or the like; a C1-6 haloalkoxy group such as a 2-chloro-n-propoxy group, a 2,3-dichlorobutoxy group, a trifluoromethoxy group, or the like; a cyano group; and a C1-6 alkylthio group such as a methylthio group, an ethylthio group, or the like.

As examples of the "C3-10 cycloalkyl group" of $R^2$, mention may be made of a monocyclic cycloalkyl group such as a cyclopropyl group, a cyclobutyl group, a cyclopentyl group, a cyclohexyl group, a cubanyl group, or the like; a bicycloalkyl group such as a bicyclooctanyl group; a tricycloalkyl group such as an adamantan-1-yl group, or the like; and the like.

As examples of the "C6-10 aryl group" of $R^2$, mention may be made of a phenyl group, a naphthyl group, and the like.

As examples of the preferable substituted group on the "C3-10 cycloalkyl group" or the "C6-10 aryl group" of $R^2$, mention may be made of a halogeno group such as a fluoro group, a chloro group, a bromo group, an iodo group or the like; a C1-6 alkyl group such as a methyl group, an ethyl group, an n-propyl group, an i-propyl group, an n-butyl group, an s-butyl group, an i-butyl group, a t-butyl group, or the like; a C1-6 haloalkyl group such as a 2-chloro-n-propyl group, a 2,3-dichlorobutyl group, a trifluoromethyl group, or the like; a C1-6 alkoxy group such as a methoxy group, an ethoxy group, an n-propoxy group, an i-propoxy group, an n-butoxy group, an s-butoxy group, an i-butoxy group, a t-butoxy group, or the like; a C1-6 haloalkoxy group such as a 2-chloro-n-propoxy group, a 2,3-dichlorobutoxy group, a trifluoromethoxy group, or the like; a cyano group; and a nitro group.

As examples of the "C1-6 alkoxy carbonyl group" of $R^2$, mention may be made of a methoxycarbonyl group, an ethoxycarbonyl group, an n-propoxycarbonyl group, an i-propoxycarbonyl group, a t-butoxycarbonyl group, and the like.

As examples of the preferable substituted group on the "C1-6 alkoxy carbonyl group" of $R^2$, mention may be made of a halogeno group such as a fluoro group, a chloro group, a bromo group, an iodo group or the like; a C1-6 alkoxy group such as a methoxy group, an ethoxy group, an n-propoxy group, an i-propoxy group, an n-butoxy group, an s-butoxy group, an i-butoxy group, a t-butoxy group, or the like; a C1-6 haloalkoxy group such as a 2-chloro-n-propoxy group, a 2,3-dichlorobutoxy group, a trifluoromethoxy group, or the like; and a cyano group.

[$R^3$]

$R^3$ in formula (I) represents a hydrogen atom, a substituted or unsubstituted C1-6 alkyl group, a substituted or unsubstituted C2-6 alkenyl group, a substituted or unsubstituted C2-6 alkynyl group, a substituted or unsubstituted C6-10 aryl C1-6 alkyl group, a substituted or unsubstituted C1-6 alkyl carbonyl group, or a substituted or unsubstituted C1-6 alkoxy carbonyl group.

As examples of the "substituted or unsubstituted C1-6 alkyl group", the "substituted or unsubstituted C2-6 alkenyl group", the "substituted or unsubstituted C6-10 aryl C1-6 alkyl group", or the "substituted or unsubstituted C1-6 alkyl carbonyl group", mention may be made of the same examples as those particularly described in $R^1$.

As examples of the "substituted or unsubstituted C1-6 alkoxy carbonyl group" of $R^3$, mention may be made of the same examples as those particularly described in $R^2$.

As examples of the "substituted or unsubstituted C2-6 alkynyl group" of $R^3$, mention may be made of an ethynyl group, a 1-propynyl group, a 2-propynyl group, a 1-butynyl group, a 2-butynyl group, a 3-butynyl group, a 1-methyl-2-propynyl group, and the like.

As examples of the preferable substituted group on the "C2-6 alkenyl group" of $R^3$, mention may be made of a halogeno group such as a fluoro group, a chloro group, a bromo group, an iodo group or the like; a C1-6 alkoxy group such as a methoxy group, an ethoxy group, an n-propoxy group, an i-propoxy group, an n-butoxy group, an s-butoxy group, an i-butoxy group, a t-butoxy group, or the like; a C1-6 haloalkyl group such as a 2-chloro-n-propyl group, a 2,3-dichlorobutyl group, a trifluoromethyl group, or the like; and a cyano group.

$R^2$ and $R^3$ may bind together to form a substituted or unsubstituted C3-5 alkylene group.

As examples of the "substituted or unsubstituted C3-5 alkylene group" which $R^2$ and $R^3$ may bind together to form, mention may be made of a trimethylene group, a tetramethylene group, a pentamethylene group, and the like.

As examples of the preferable substituted group on the "C3-5 alkylene group", mention may be made of a halogeno group such as a fluoro group, a chloro group, a bromo group, an iodo group or the like; a CI-6 alkyl group such as a methyl group, an ethyl group, an n-propyl group, an n-butyl group, an s-butyl group, an i-butyl group, a t-butyl group, or the like; and a C1-6 haloalkyl group such as a 2-chloro-n-propyl group, a 2,3-dichlorobutyl group, a trifluoromethyl group, or the like.

[Q]

Q in formula (I) represents a substituted or unsubstituted o-phenylene group.

As examples of the preferable substituted group on the o-phenylene group of the "substituted o-phenylene group", mention may be made of a halogeno group such as a fluoro group, a chloro group, a bromo group, an iodo group or the like; a C1-6 alkyl group such as a methyl group, an ethyl group, an n-propyl group, an i-propyl group, an n-butyl group, an s-butyl group, an i-butyl group, a t-butyl group, or the like; a C2-6 alkenyl group such as a vinyl group, a 1-propenyl group, a 2-propenyl group (an allyl group), a 1-butenyl group, a 2-butenyl group, a 3-butenyl group, a 1-methyl-2-propenyl group, a 2-methyl-2-propenyl group, or the like; a C1-6 haloalkyl group such as a 2-chloro-n-propyl group, a 2,3-dichlorobutyl group, a trifluoromethyl group, or the like; a C1-6 alkoxy group such as a methoxy group, an ethoxy group, an n-propoxy group, an i-propoxy group, an n-butoxy group, an s-butoxy group, an i-butoxy group, a t-butoxy group, or the like; a C1-6 haloalkoxy group such as a 2-chloro-n-propoxy group, a 2,3-dichlorobutoxy group, a trifluoromethoxy group, a 2,2,2-trifluoroethoxy group, a 4,4,4-trifluorobutoxy group, or the like; a benzyloxy group; a cyano group; a nitro group; a C6-10 aryl group such as a phenyl group or the like; a C3-6 cycloalkyl group such as a cyclopropyl group, a cyclobutyl group, a cyclopentyl group, a cyclohexyl group, a cycloheptyl group, or the like; a C1-6 alkylthio group such as a methylthio group, an ethylthio group, an n-propylthio group, an n-butylthio group, an n-pentylthio group, an n-hexylthio group, an i-propylthio group, or the like; a C1-6 alkylsulfinyl group such as a methylsulfinyl group, an ethylsulfinyl group, a t-butylsulfinyl group, or the like; a C1-6 alkoxy carbonyl group such as a methoxycarbonyl group, an ethoxycarbonyl group, or the like; a C1-6 alkoxy carbonyl C1-6 alkyl group such as a methoxycarbonyl methyl group, an ethoxycarbonyl ethyl group, or the like; and the like.

[A]

A in formula (I) represents a substituted or unsubstituted o-phenylene group, a substituted or unsubstituted 5- to 6-membered heteroarylene group, a substituted or unsubstituted benzylene group, a substituted or unsubstituted dimethylene group, or 1,2-cyclopropylene group,

As examples of the substituted group on the o-phenylene group of the "unsubstituted o-phenylene group", mention may be made of the same examples as those particularly described in the substituted groups of the o-phenylene group in "Q" mentioned above.

The "5- to 6-membered heteroarylene group" is a divalent group formed by removing two hydrogen atoms in a heteroaryl compound. The heteroaryl compound is an aromatic compound containing 1 to 4 heteroatoms selected from the group consisting of a nitrogen atom, an oxygen atom and a sulfur atom as a constitutional atom of the ring.

As examples of the 5- to 6-membered heteroarylene group, mention may be made of a 5-membered heteroarylene group such as a thiophendiyl group (more particularly, a 2,3-thiophendiyl group, or a 3,4-thiophendiyl group may be mentioned), a furandiyl group, a pyrroldiyl group, or the like; and a 6-membered heteroarylene group such as a pyridilene group, a pyrimidilene group, a pyridadilene group, or the like.

As examples of the substituted group on the 5- to 6-membered hetroarylene group of the "substituted 5- to 6-membered hetroarylene group", mention may be made of the same examples as those particularly described in the substituted groups of the o-phenylene group mentioned above.

The "bezylene group" is a group represented by the following formula (II).

[Chem. 3]

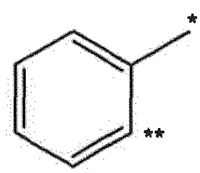

(II)

The carbon atom to which * is added in formula (II) represents a carbon atom binding to the spirocarbon atom in the oxadiazoline ring, and the carbon atom to which ** is added represents a carbon atom binding to "B" in formula (I).

As examples of the substituted group on the benzylene group of the "substituted bezylene group", mention may be made of the same examples as those particularly described in the o-phenylene group mentioned above.

As examples of the substituted group on the dimethylene group of the "substituted dimethylene group", mention may be made of the same examples as those particularly described in the o-phenylene group mentioned above.

In the case where one carbon atom has a plurality of substituted groups, the substituted groups may bind together to form a divalent substituted group. As examples of the divalent substituted group formed, mention may be made of a C2-5 alkylene group such as a dimethylene group, a trimethylene group, a tetramethylene group, or the like.

[B]

B represents a single bond, an oxy group, a substituted or unsubstituted oxymethylene group, a substituted or unsubstituted methyleneoxy group, a substituted or unsubstituted thiomethylene group, a substituted or unsubstituted methylenethio group, a substituted or unsubstituted methylene group, a substituted or unsubstituted dimethylene group, or a substituted or unsubstituted vinylene group, a thio group, a substituted or unsubstituted sulfonylmethylene group, a substituted or unsubstituted methylenesulfonyl group, a substituted or unsubstituted trimethylene group, a substituted or unsubstituted oxyethylene group, a substituted or unsubstituted ethyleneoxy group, a substituted or unsubstituted propenylene group, a substituted or unsubstituted oxymethyleneoxy group, a group represented by formula: $-NR^a-$, a group represented by formula: $-CH_2-NR^a-$, or a group represented by formula: $-NR^a-CH_2-$.

The "unsubstituted oxymethylene group" is, in particular, a group represented by a formula: $*O-CH_2**$.

The "unsubstituted methyleneoxy group" is a group represented by a formula: $*CH_2-O**$.

The "unsubstituted thiomethylene group" is, in particular, a group represented by a formula: $*S-CH_2**$.

The "unsubstituted methylenethio group" is a group represented by a formula: $*CH_2-S**$.

The "unsubstituted sulfonylmethylene group" is, in particular, a group represented by a formula: $*SO_2-CH_2**$.

The "unsubstituted methylenesulfonyl group" is a group represented by a formula: $*CH_2-SO_2**$.

The "unsubstituted oxyethylene group" is, in particular, a group represented by a formula: $*O-CH_2CH_2**$.

The "unsubstituted ethyleneoxy group" is a group represented by a formula: $*CH_2CH_2-O**$.

The "unsubstituted oxyethylene group" is a group represented by a formula: $*O-CH_2-O**$.

The atom with * in each formula represents an atom binding to "A" in formula (I). The atom with ** represents an atom binding to "Q" in formula (I).

$R^a$ represents a hydrogen atom or a substituted or unsubstituted C1-6 alkyl group.

As examples of the "C1-6 alkyl group" of $R^a$, mention may be made of the same examples as those particularly described in $R^1$ mentioned above.

As examples of the substituted group on each group of the "substituted oxymethylene group", the "substituted methyleneoxy group", the "substituted thiomethylene group", the "substituted methylenethio group", the "substituted methylene group", the "substituted dimethylene group", the " substituted vinylene group", the "substituted trimethylene group", the "substituted oxyethylene group", the "substituted ethyleneoxy group", the "substituted propenylene group", and the "substituted oxymethyleneoxy group", mention may be made of the same examples as those particularly described in the o-phenylene group mentioned above. In the case where one carbon atom has a plurality of substituted groups, they may bind together to form a divalent substituted group. As examples of the divalent substituted group formed, mention may be made of a C2-5 alkylene group such as a dimethylene group, a trimethylene group, a tetramethylene group, or the like.

As examples of the preferable substituted group on each group, mention may be made of a halogeno group such as a fluoro group, a chloro group, a bromo group, an iodo group or the like; a C1-6 alkyl group such as a methyl group, an ethyl group, an n-propyl group, an i-propyl group, an n-butyl group, an s-butyl group, an i-butyl group, a t-butyl group, or the like; a cyano group; and the like.

Among the oxadiazoline compounds according to the present invention, a compound (a compound represented by formula (1-2)) is preferable, in which each of Q and A represents an o-phenylene group, and B is an unsubstituted oxymethylene group, an unsubstituted methyleneoxy group, a substituted or unsubstituted dimethylene group, or a substituted or unsubstituted vinylene group in formula (I).

[Chem. 4]

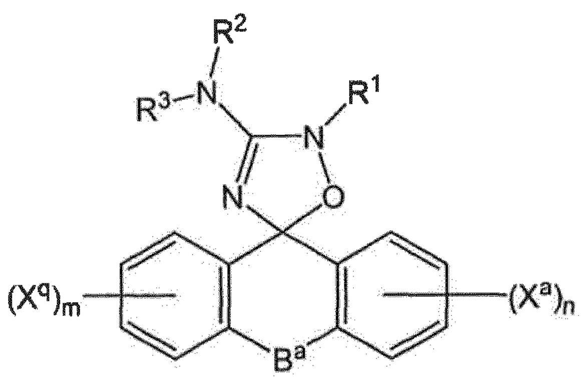

(I-2)

In formula (1-2), $R^1$ to $R^3$ represent the same meanings as those in formula (I),

$X^q$ represents a substituted group on the o-phenylene group,

m represents the number of the substituted groups, and represents any integer ranging from 0 to 4,

$X^a$ represents a substituted group on the o-phenylene group,
n represents the number of the substituted groups, and represents any integer ranging from 0 to 4, and
$B^a$ represents a substituted or unsubstituted oxymethylene group, a substituted or unsubstituted methyleneoxy group, a substituted or unsubstituted dimethylene group, or a substituted or unsubstituted vinylene group.

As examples of the substituted groups $X^q$ and $X^a$, mention may be made of the same examples as those described as the substituted group on the o-phenylene group of [Q] mentioned above.

Among the oxadiazoline compounds according to the present invention, a compound (a compound represented by formula (I-1)) is preferable, in which each of Q and A represents an o-phenylene group, and B is an unsubstituted oxymethylene group or an unsubstituted methyleneoxy group in formula (I).

[Chem. 5]

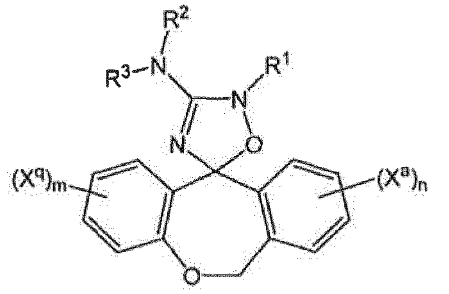

(I-1)

In formula (I-1), $R^1$ to $R^3$ represent the same meanings as those in formula (I),
$X^q$ represents a substituted group on the o-phenylene group,
m represents the number of the substituted groups, and represents any integer ranging from 0 to 4,
$X^a$ represents a substituted group on the o-phenylene group, and
n represents the number of the substituted groups, and represents any integer ranging from 0 to 4.

As examples of the substituted groups $X^q$ and $X^a$, mention may be made of the same examples as those described as the substituted group on the o-phenylene group of [Q] mentioned above.

A salt of compound (I) is not particularly limited as long as the salt is an agriculturally and horticulturally acceptable salt. As examples of the salt of compound (I), mention may be made of a salt of an inorganic acid such as hydrochloric acid, sulfuric acid or the like; a salt of an organic acid such as acetic acid, lactic acid or the like; a salt of an alkaline metal such as lithium, sodium, potassium or the like; a salt of an alkaline earth metal such as calcium, magnesium or the like; a salt of a transition metal such as iron, copper or the like; a salt of an organic base such as ammonia, triethylamine, tributylamine, pyridine, hydrazine or the like; and the like.

The compound (I) or a salt of the compound (I) is not particularly limited by the preparation method thereof. For example, the compound (I) or a salt thereof can be obtained by means of the well-known preparation methods described in the Examples and the like. In addition, the salts thereof can be obtained from the compound (I) by means of well-known methods.

The oxadiazoline compound of the present invention has a superior effect for controlling harmful organisms such as various agricultural pests affecting the plant growth, and acari.

In addition, the oxadiazoline compound of the present invention has a reduced phytotoxicity against plants and has a low level of toxicity against fish or warm-blooded animals, and for this reason, the compound of the present invention is a compound with high safety. For this reason, the compound of the present invention is useful as an active ingredient of a pesticide or an acaricide.

In addition, in recent years, many pests such as diamondback moths, planthoppers, leafhoppers and aphids have developed a resistance to various types of conventional agrochemicals, and for this reason, a problem occurs in which the efficacy of the conventional agrochemicals has become insufficient. Therefore, agrochemicals that are effective even for the resistant strains of pests are desired. The oxadiazoline compounds of the present invention exhibit superior effects for controlling not only the sensitive strains of pests, but also various resistant strains of pests and acaricide-resistant strains of acari. The oxadiazoline compounds of the present invention exhibit superior effects for controlling Thysanoptera insect pests.

The compounds of the present invention have a superior effect for controlling the ectoparasites harmful for humans and animals. In addition, the compounds of the present invention have a low level of toxicity to the fish or warm-blooded animals, and for this reason, the oxadiazoline compounds are highly safe compounds. For this reason, the

compounds of the present invention are useful as an active ingredient of a formulation for controlling ectoparasites.

In addition, the oxadiazoline compounds of the present invention are effective for controlling the targeted organisms in any development stages, and exhibit superior effects of controlling, for example, acari and insects in the stages of eggs, nymphs, larvae, pupae and adults.

[Formulation for controlling harmful organisms, insecticidal formulation, or formulation for controlling Thysanoptera insect pests]

The formulation for controlling harmful organisms, the insecticidal formulation, or the formulation for controlling Thysanoptera insect pests of the present invention contains at least one compound selected from the oxadiazoline compounds of the present invention as an active ingredient. The amount of the oxadiazoline compound contained in the formulation for controlling harmful organisms, the insecticide or the acaricide of the present invention is not particularly limited as long as an effect of controlling harmful organisms, agricultural pests, or acari is exhibited.

The formulation for controlling harmful organisms, the insecticidal formulation, or the formulation for controlling Thysanoptera insect pests of the present invention is preferably used for crops; vegetables; edible roots; tuber crops; flowers; fruit trees; trees of tea, coffee, cacao or foliage plants; grasses for pastures; grasses for lawns; plants such as cotton; or the like.

As for the application to the plants, the formulation for controlling harmful organisms, the insecticidal formulation or the formulation for controlling Thysanoptera insect pests of the present invention may be applied on any one part of the plants, such as leaf, stem, stalk, flower, bud, fruit, seed, sprout, root, tuber, tuberous root, shoot, cutting and the like.

In addition, the plant varieties for which the formulation for controlling harmful organisms, the insecticidal formulation or the formulation for controlling Thysanoptera insect pests of the present invention is applicable are not particularly limited. As examples of the plant varieties, mention may be made of originals, varieties, improved varieties, cultivated varieties, mutant plants, hybrid plants, genetically modified organisms (GMO) and the like.

The formulations for controlling harmful organisms of the present invention can be used for controlling various agricultural pests and acari by seed treatment, foliar spraying, soil application, water surface application and the like.

Specific examples of the various agricultural pests and acari which can be controlled by the formulations for controlling harmful organisms of the present invention are shown below.

(1) Lepidoptera Butterflies and Moths

(a) Arctiidae moths, for example, Hyphantria cunea and Lemyra imparilis;
(b) Bucculatricidae moths, for example, Bucculatrix pyrivorella;
(c) Carposinidae, for example, Carposina sasakii;
(d) Crambidae moths, for example, Diaphania indica and Diaphania nitidalis of Diaphania spp.; Ostrinia fumacalis, Ostrinia nubilalis and Ostrinia scapulalis of Ostrinia spp.; and others such as Chilo suppressalis, Cnaphalocrocis medinalis, Conogethes punctiferalis, Diatraea grandiosella, Glyphodes pyloalis, Hellula undalis and Parapediasia teterrella;
(e) Gelechiidae moths, for example, Helcystogramma triannulella, Pectinophora gossypiella, Phthorimaea operculella and Sitotroga cerealella;
(f) Geometridae moths, for example, Ascotis selenaria;
(g) Gracillariidae moths, for example, Caloptilia theivora, Phyllocnistis citrella and Phyllonorycter ringoniella;
(h) Hesperiidae butterflies, for example, Parnara guttata;
(i) Lasiocampidae moths, for example, Malacosoma neustria;
(j) Lymantriidae moths, for example, Lymantria dispar and Lymantria monacha of Lymantria spp.; and others such as Euproctis pseudoconspersa and Orgyia thyellina;
(k) Lyonetiidae moths, for example, Lyonetia clerkella and Lyonetia prunifoliella malinella of Lyonetia spp.;
(l) Noctuidae moths, for example, Spodoptera depravata, Spodoptera eridania, Spodoptera exigua, Spodoptera frugiperda, Spodoptera littoralis and Spodoptera litura of Spodoptera spp.; Autographa gamma and Autographa nigrisigna of Autographa spp.; Agrotis ipsilon and Agrotis segetum of Agrotis spp.; Helicoverpa armigera, Helicoverpa assulta and Helicoverpa zea of Helicoverpa spp.; Heliothis armigera and Heliothis virescens of Heliothis spp.; and others such as Aedia leucomelas, Ctenoplusia agnata, Eudocima tyrannus, Mamestra brassicae, Mythimna separata, Naranga aenescens, Panolis japonica, Peridroma saucia, Pseudoplusia includens and Trichoplusiani;
(m) Nolidae moths, for example, Earias insulana;
(n) Pieridae butterflies, for example, Pieris brassicae and Pieris rapae crucivora of Pieris spp.;
(o) Plutellidae moths, for example, Acrolepiopsis sapporensis and Acrolepiopsis suzukiella of Acrolepiopsis spp.; and others such as Plutella xylostella;

(p) Pyralidae moths, for example, Cadra cautella, Elasmopalpus lignosellus, Etiella zinckenella and Galleria mellonella;
(q) Sphingidae moths, for example, Manduca quinquemaculata and Manduca sexta of Manduca spp.;
(r) Stathmopodidae moths, for example, Stathmopoda masinissa;
(s) Tineidae moths, for example, Tinea translucens;
(t) Tortricidae moths, for example, Adoxophyes honmai and Adoxophyes orana of Adoxophyes spp.; Archips breviplicanus and Archips fuscocupreanus of Archips spp.; and others such as Choristoneura fumiferana, Cydia pomonella, Eupoecilia ambiguella, Grapholitha molesta, Homona magnanima, Leguminivora glycinivorella, Lobesia botrana, Matsumuraeses phaseoli, Pandemis heparana and Sparganothis pilleriana; and (u) Yponomeutidae moths, for example, Argyresthia conjugella.

(2) Thysanoptera Insect Pests

(a) Phlaeothripidae, for example, Ponticulothrips diospyrosi; and
(b) Thripidae, for example, Frankliniella intonsa and Frankliniella occidentalis of Frankliniella spp.; Thrips palmi and Thrips tabaci of Thrips spp.; and others such as Heliothrips haemorrhoidalis and Scirtothrips dorsalis.

(3) Hemiptera Insect Pests

(A) Archaeorrhyncha

(a) Delphacidae, for example, Laodelphax striatella, Nilaparvata lugens, Perkinsiella saccharicida and Sogatella furcifera.

(B) Clypeorrhyncha

(a) Cicadellidae, for example, Empoasca fabae, Empoasca nipponica, Empoasca onukii and Empoasca sakaii of Empoasca spp.; and others such as Arboridia apicalis, Balclutha saltuella, Epiacanthus stramineus, Macrosteles striifrons and Nephotettix cinctinceps.

(C) Heteroptera

(a) Alydidae, for example, Riptortus clavatus;
(b) Coreidae, for example, Cletus punctiger and Leptocorisa chinensis;
(c) Lygaeidae, for example, Blissus leucopterus, Cavelerius saccharivorus and Togo hemipterus;
(d) Miridae, for example, Halticus insularis, Lygus lineolaris, Psuedatomoscelis seriatus, Stenodema sibiricum, Stenotus rubrovittatus and Trigonotylus caelestialium;
(e) Pentatomidae, for example, Nezara antennata and Nezara viridula of Nezara spp.; Eysarcoris aeneus, Eysarcoris lewisi and Eysarcoris ventralis of Eysarcoris spp.; and others such as Dolycoris baccarum, Eurydema rugosum, Glaucias subpunctatus, Halyomorpha halys, Piezodorus hybneri, Plautia crossota and Scotinophora lurida;
(f) Pyrrhocoridae, for example, Dysdercus cingulatus;
(g) Rhopalidae, for example, Rhopalus msculatus;
(h) Scutelleridae, for example, Eurygaster integriceps; and
(i) Tingidae, for example, Stephanitis nashi.

(D) Sternorrhyncha

(a) Adelgidae, for example, Adelges laricis;
(b) Aleyrodidae, for example, Bemisia argentifolii and Bemisia tabaci of Bemisia spp.; and others such as Aleurocanthus spiniferus, Dialeurodes citri and Trialeurodes vaporariorum;
(c) Aphididae, for example, Aphis craccivora, Aphis fabae, Aphis forbesi, Aphis gossypii, Aphis pomi, Aphis sambuci and Aphis spiraecola of Aphis spp.; Rhopalosiphum maidis and Rhopalosiphum padi of Rhopalosiphum spp.; Dysaphis plantaginea and Dysaphis radicola of Dysaphis spp.; Macrosiphum avenae and Macrosiphum euphorbiae of Macrosiphum spp.; Myzus cerasi, Myzus persicae and Myzus varians of Myzus spp.; and others such as Acyrthosiphon pisum, Aulacorthum solani, Brachycaudus helichrysi, Brevicoryne brassicae, Chaetosiphon fragaefolii, Hyalopterus pruni, Hyperomyzus lactucae, Lipaphis erysimi, Megoura viciae, Metopolophium dirhodum, Nasonovia ribis-nigri, Phorodon humuli, Schizaphis graminum, Sitobion

avenae and Toxoptera aurantii;

(d) Coccidae, for example, Ceroplastes ceriferus and Ceroplastes rubens of Ceroplastes spp.;

(e) Diaspididae, for example, Pseudaulacaspis pentagona and Pseudaulacaspis prunicola of Pseudaulacaspis spp.; Unaspis euonymi and Unaspis yanonensis of Unaspis spp.; and others such as Aonidiella aurantii, Comstockaspis pemiciosa, Fiorinia theae and Pseudaonidia paeoniae;

(f) Margarodidae, for example, Drosicha corpulenta and Icerya purchasi;

(g) Phylloxeridae, for example, Viteus vitifolii;

(h) Pseudococcidae, for example, Planococcus citri and Planococcus kuraunhiae of Planococcus spp.; and others such as Phenacoccus solani and Pseudococcus comstocki; and

(i) Psyllidae, for example, Psylla mali and Psylla pyrisuga of Psylla spp.; and others such as Diaphorina citri.

(4) Polyphaga Insect Pests

(a) Anobiidae, for example, Lasioderma serricorne;

(b) Attelabidae, for example, Byctiscus betulae and Rhynchites heros;

(c) Bostrichidae, for example, Lyctus brunneus;

(d) Brentidae, for example, Cylas formicarius;

(e) Buprestidae, for example, Agrilus sinuatus;

(f) Cerambycidae, for example, Anoplophora malasiaca, Monochamus alternatus, Psacothea hilaris and Xylotrechus pyrrhoderus;

(g) Chrysomelidae, for example, Bruchus pisorum and Bruchus rufimanus of Bruchus spp.; Diabrotica barberi, Diabrotica undecimpunctata and Diabrotica virgifera of Diabrotica spp.; Phyllotreta nemorum and Phyllotreta striolata of Phyllotreta spp.; and others such as Aulacophora femoralis, Callosobruchus chinensis, Cassida nebulosa, Chaetocnema concinna, Leptinotarsa decemlineata, Oulema oryzae and Psylliodes angusticollis;

(h) Coccinellidae, for example, Epilachna varivestis and Epilachna vigintioctopunctata of Epilachna spp.;

(i) Curculionidae, for example, Anthonomus grandis and Anthonomus pomorum of Anthonomus spp.; Sitophilus granarius and Sitophilus zeamais of Sitophilus spp.; and others such as Echinocnemus squameus, Euscepes postfasciatus, Hylobius abietis, Hypera postica, Lissohoptrus oryzophilus, Otiorhynchus sulcatus, Sitona lineatus and Sphenophorus venatus;

(j) Elateridae, for example, Melanotus fortnumi and Melanotus tamsuyensis of Melanotus spp.;

(k) Nitidulidae, for example, Epuraea domina;

(l) Scarabaeidae, for example, Anomala cuprea and Anomala rufocuprea of Anomala spp.; and others such as Cetonia aurata, Gametis jucunda, Heptophylla picea, Melolontha and Popillia japonica;

(m) Scolytidae, for example, Ips typographus;

(n) Staphylinidae, for example, Paederus fuscipes;

(o) Tenebrionidae, for example, Tenebrio molitor and Tribolium castaneum; and

(p) Trogossitidae, for example, Tenebroides mauritanicus.

(5) Diptera Insect Pests

(A) Brachycera

(a) Agromyzidae, for example, Liriomyza bryoniae, Liriomyza chinensis, Liriomyza sativae and Liriomyza trifolii of Liriomyza spp.; and others such as Chromatomyia horticola and Agromyza oryzae;

(b) Anthomyiidae, for example, Delia platura and Delia radicum of Delia spp.; and others such as Pegomya cunicularia;

(c) Drosophilidae, for example, Drosophila melanogaster and Drosophila suzukii of Drosophila spp.;

(d) Ephydridae, for example, Hydrellia griseola;

(e) Psilidae, for example, Psila rosae; and

(f) Tephritidae, for example, Bactrocera cucurbitae and Bactrocera dorsalis of Bactrocera spp.; Rhagoletis cerasi and Rhagoletis pomonella of Rhagoletis spp.; and others such as Ceratitis capitata and Dacus oleae.

(B) Nematocera

(a) Cecidomyiidae, for example, Asphondylia yushimai, Contarinia sorghicola, Mayetiola destructor and Sitodiplosis mosellana.

(6) Orthoptera Insect Pests

(a) Acrididae, for example, Schistocerca americana and Schistocerca gregaria of Schistocerca spp.; and others such as Chortoicetes terminifera, Dociostaurus maroccanus, Locusta migratoria, Locustana pardalina, Nomadacris septemfasciata and Oxya yezoensis;
(b) Gryllidae, for example, Acheta domestica and Teleogryllus emma;
(c) Gryllotalpidae, for example, Gryllotalpa orientalis; and
(d) Tettigoniidae, for example, Tachycines asynamorus.

(7) Acari

(A) Acaridida of Astigmata

(a) Acaridae mites, for example, Rhizoglyphus echinopus and Rhizoglyphus robini of Rhizoglyphus spp.; Tyrophagus neiswanderi, Tyrophagus perniciosus, Tyrophagus putrescentiae and Tyrophagus similis of Tyrophagus spp.; and others such as Acarus siro, Aleuroglyphus ovatus and Mycetoglyphus fungivorus;

(B) Actinedida of Prostigmata

(a) Tetranychidae mites, for example, Bryobia praetiosa and Bryobia rubrioculus of Bryobia spp.; Eotetranychus asiaticus, Eotetranychus boreus, Eotetranychus celtis, Eotetranychus geniculatus, Eotetranychus kankitus, Eotetranychus pruni, Eotetranychus shii, Eotetranychus smithi, Eotetranychus suginamensis and Eotetranychus uncatus of Eotetranychus spp.; Oligonychus hondoensis, Oligonychus ilicis, Oligonychus karamatus, Oligonychus mangiferus, Oligonychus orthius, Oligonychus perseae, Oligonychus pustulosus, Oligonychus shinkajii and Oligonychus ununguis of Oligonychus spp.; Panonychus citri, Panonychus mori and Panonychus ulmi of Panonychus spp.; Tetranychus cinnabarinus, Tetranychus evansi, Tetranychus kanzawai, Tetranychus ludeni, Tetranychus quercivorus, Tetranychus phaselus, Tetranychus urticae and Tetranychus viennensis of Tetranychus spp.; Aponychus corpuzae and Aponychus firmianae of Aponychus spp.; Sasanychus akitanus and Sasanychus pusillus of Sasanychus spp.; Shizotetranychus celarius, Shizotetranychus longus, Shizotetranychus miscanthi, Shizotetranychus recki and Shizotetranychus schizopus of Shizotetranychus spp.; and others such as Tetranychina harti, Tuckerella pavoniformis and Yezonychus sapporensis;
(b) Tenuipalpidae mites, for example, Brevipalpus lewisi, Brevipalpus obovatus, Brevipalpus phoenicis, Brevipalpus russulus and Brevipalpus californicus of Brevipalpus spp.; Tenuipalpus pacificus and Tenuipalpus zhizhilashviliae of Tenuipalpus spp.; and others such as Dolichotetranychus floridanus;
(c) Eriophyidae mites, for example, Aceria diospyri, Aceria ficus, Aceria japonica, Aceria kuko, Aceria paradianthi, Aceria tiyingi, Aceria tulipae and Aceria zoysiea of Aceria spp.; Eriophyes chibaensis and Eriophyes emarginatae of Eriophyes spp.; Aculops lycopersici and Aculops pelekassi of Aculops spp.; Aculus fockeui and Aculus schlechtendali of Aculus spp.; and others such as Acaphylla theavagrans, Calacarus carinatus, Colomerus vitis, Calepitrimerus vitis, Epitrimerus pyri, Paraphytoptus kikus, Paracalacarus podocarpi and Phyllocotruta citri;
(d) Tarsonemidae mites, for example, Tarsonemus bilobatus and Tarsonemus waitei of Tarsonemus spp.; and others such as Phytonemus pallidus and Polyphagotarsonemus latus; and
(e) Penthaleidae mites, for example, Penthaleus erythrocephalus and Penthaleus major of Penthaleus spp.

The formulation for controlling harmful organisms of the present invention may be mixed or used in combination with other active constituents such as fungicides, insecticides / acaricides, nematicides and soil pesticides; and/or plant regulators, herbicides, synergists, fertilizers, soil conditioners and animal feed.
Combinations of the oxadiazoline compound of the present invention with other active constituents can be expected to provide synergistic effects in terms of insecticidal / acaricidal / nematicidal activity. The synergistic effect can be confirmed in accordance with a conventional method by means of an equation defined by Colby (Colby. S. R.; Calculating Synergistic and Antagonistic Responses of Herbicide Combinations; Weeds, 15, pages 20 - 22, 1967).
Examples of the insecticides / acaricides, nematocides, soil pesticides, vermicides and the like which can be mixed or used together with the formulation for controlling harmful organisms according to the present invention are described below.

(1) Acetylcholine esterase inhibitor:

(a) Carbamate-based agents: alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb,

methomyl, oxamyl, pirimicarb, propoxur, thiodicarb, thiofanox, triazamate, trimethacarb, XMC, xylylcarb, fenothiocarb, MIPC, MPMC, MTMC, aldoxycarb, allyxycarb, aminocarb, bufencarb, cloethocarb, metam-sodium, and promecarb; and

(b) Organic phosphorus-based agents: acephate, azamethiphos, azinphos-ethyl, azinphos-methyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos / DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion; bromophos-ethyl, BRP, carbophenothion, cyanofenphos, CYAP, demeton-S-methyl sulfone, dialifos, dichlofenthion, dioxabenzofos, etrimfos, fensulfothion, flupyrazofos, fonofos, formothion, fosmethilan, isazofos, jodfenphos, methacrifos, pirimiphos-ethyl, phosphocarb, propaphos, prothoate, and sulprofos.

(2) GABA-gated chloride ion channel antagonists: acetoprole, chlordane, endosulfan, ethiprole, fipronil, pyrafluprole, pyriprole, camphechlor, heptachlor, and dienochlor.

(3) Sodium channel modulators: acrinathrin, d-cis-trans-allethrin, d-trans allethrin, bifenthrin, bioallethrin, bioallethrin S-cyclopentyl isomer, bioresmethrin, cycloprothrin, cyfluthrin, β-cyfluthrin, cyhalothrin, λ-cyhalothrin, γ-cyhalothrin, cypermethrin, α-cypermethrin, β-cypermethrin, θ-cypermethrin, ξ-cypermethrin, cyphenothrin [(1R)-trans isomer], δ-methrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, permethrin, phenothrin [(1R)-trans isomer], prallethrin, pyrethrum, resmethrin, silafluofen, tefluthrin, tetramethrin [(1R)-isomer], tralomethrin, transfluthrin; allethrin, pyrethrins, pyrethrin I, pyrethrin II, profluthrin, dimefluthrin, bioethanomethrin, biopermethrin, transpermethirn, fenfluthrin, fenpirithrin, flubrocythrinate, flufenprox, metofluthrin, protrifenbute, pyresmethrin, and terallethrin.

(4) Nicotinic acetylcholine receptor agonists: acetamiprid, clothianidin, dinotefuran, imidacloprid, nitenpyram, nithiazine, thiacloprid, thiamethoxam, sulfoxaflor, nicotine, and flupyradifurone.

(5) Nicotinic acetylcholine receptor allosteric modulators: spinetoram, and spinosad.

(6) Chloride channel activators: abamectin, emamectin-benzoate, lepimectin, milbemectin, ivermectin, selamectin, doramectin, eprinomectin, moxidectin, milbemycin; milbemycin oxime, and nemadectin.

(7) Juvenile hormone-like substances: hydroprene, kinoprene, methoprene, fenoxycarb, pyriproxyfen, diofenolan, epofenonane, and triprene.

(8) Other nonspecific inhibitors: methyl bromide, chloropicrin, sulfuryl fluoride, borax, and tartar emetic.

(9) Homoptera selective antifeedants: flonicamid, pymetrozine, and pyrifluquinazon.

(10) Acari growth inhibitors: clofentezine, diflovidazin, hexythiazox, and etoxazole.

(11) Microorganism-derived insect midgut inner membrane distrupting agents: Bacillus thuringiensis subsp. Israelensis, Bacillus sphaericus, Bacillus thuringiensis subsp. Aizawai, Bacillus thuringiensis subsp. Kurstaki, Bacillus thuringiensis subsp. Tenebrionis, Bt crop protein: Cry1Ab, Cry1Ac, Cry1Fa, Cry1A. 105, Cry2Ab, Vip3A, mCry3A, Cry3Ab, Cry3Bb, and Cry34Ab1/Cry35Ab1.

(12) Mitochondria ATP biosynthesis enzyme inhibitors: diafenthiuron, azocyclotin, cyhexatin, fenbutatin oxide, propargite, and tetradifon.

(13) Oxidative phosphorylation decouplers: chlorfenapyr, sulfluramid, DNOC; binapacryl, dinobuton, and dinocap.

(14) Nicotinic acetylcholine receptor channel blockers: bensultap, cartap hydrochloride; nereistoxin; thiosultap-sodium, and thiocyclam.

(15) Chitin synthesis inhibitors: bistrifluron, chlorfluazuron, diflubenzuron, flucycloxuron, flufenoxuron, hexaflumuron, lufenuron, novaluron, noviflumuron, teflubenzuron, triflumuron, buprofezin, and fluazuron.

(16) Diptera molting disruptors: cyromazine.

(17) Molting hormone receptor agonists: chromafenozide, halofenozide, methoxyfenozide, and tebufenozide.

(18) Octopamine receptor agonists: amitraz, demiditraz, and chlordimeform.

(19) Mitochondria electron transfer chain complex III inhibitors: acequinocyl, fluacrypyrim, hydramethylnon, and bifenazate.

(20) Mitochondria electron transfer chain complex I inhibitors: fenazaquin, fenpyroximate, pyrimidifen, pyridaben, tebufenpyrad, tolfenpyrad, and rotenone.

(21) Voltage-dependent sodium channel blockers: indoxacarb, and metaflumizone.

(22) Acetyl CoA carboxylase inhibitors: spirodiclofen, spiromesifen, spirotetramat, and spiropidion.

(23) Mitochondria electron transfer chain complex IV inhibitors: aluminum phosphide, calcium phosphide, phosphine, zinc phosphide, and cyanide.

(24) Mitochondria electron transfer chain complex II inhibitors: cyenopyrafen, cyflumetofen, and pyflubumide.
(25) Ryanodine receptor modulators: chlorantraniliprole, cyantraniliprole, flubendiamide, cyclaniliprole, and tetraniliprole.
(26) Mixed function oxidase inhibitor compounds: piperonyl butoxide.
(27) Latrophilin receptor agonists: depsipeptide, cyclodepsipeptide, 24 membered cyclodepsipeptide, and emodepside.
(28) Others (action mechanism is unknown): azadirachtin, benzoximate, bifenazate, bromopropylate, quinomethionate, cryolite, dicofol, pyridalyl, benclothiaz, sulfur, amidoflumet, 1, 3-dichloropropene, DCIP, phenisobromolate, benzomate, metaldehyde, chlorobenzilate, chlothiazoben, dicyclanil, fenoxacrim, fentrifanil, flubenzimine, fluphenazine, gossyplure, japonilure, metoxadiazone, oil, sodium oleate, tetrasul, triarathene; afidopyropen, flometoquin, flufiprole, fluensulfone, meperfluthrin, tetramethylfluthrin, tralopyril, dimefluthrin, methylneodecanamide; fluralaner, afoxolaner, and fluxametamide, 5-[5-(3,5-dichlorophenyl)-5-trifluoromethyl-4,5-dihydroisoxazol- 3-yl]-2-(1H-1,2,4-triazol-1-yl)benzonitrile (CAS: 943137-49-3), broflanilide, triflumezopyrim, dicloromezotiaz, oxazosulfyl, and other meta-diamides.
(29) Parasiticide:

(a) Benzimidazole-based agents: fenbendazole, albendazole, triclabendazole, oxibendazole, mebendazole, oxfendazole, parbendazole, flubendazole; febantel, netobimin, thiophanate; thiabendazole, and cambendazole;
(b) Salicylanilide-based agents: closantel, oxyclozanide, rafoxanide, and niclosamide;
(c) Substituted phenol-based agents: nitroxinil, and nitroscanate;
(d) Pyrimidine-based agents: pyrantel, and morantel;
(e) Imidazothiazole-based agents: levamisole, and tetramisole;
(f) Tetrahydropyrimidine-based agents: praziquantel, and epsiprantel; and
(g) Other antiparasitic agents: cyclodien, ryania, clorsulon, metronidazole, demiditraz; piperazine, diethylcarbamazine, dichlorophen, monepantel, tribendimidine, amidantel; thiacetarsamide, melarsomine, and arsenamide.

Specific examples of the fungicides which can be mixed or used together with the formulation for controlling harmful organisms according to the present invention are described below.

(1) Nucleic acid biosynthesis inhibitors:

(a) RNA polymerase I inhibitors: benalaxyl, benalaxyl-M, furalaxyl, metalaxyl, metalaxyl-M; oxadixyl; clozylacon, and ofurace;
(b) Adenosine deaminase inhibitors: bupirimate, dimethirimol, and ethirimol;
(c) DNA/RNA synthesis inhibitors: hymexazol, and octhilinone; and
(d) DNA topoisomerase II inhibitors: oxolinic acid.

(2) Karyokinesis inhibitor and cell division inhibitors:

(a) β-Tubulin polymerization inhibitors: benomyl, carbendazim, chlorfenazole, fuberidazole, thiabendazole; thiophanate, thiophanate-methyl; diethofencarb; zoxamide; and ethaboxam;
(b) Cell division inhibitors: pencycuron; and
(c) Delocalization inhibitors of spectrin-like protein: fluopicolide.

(3) Respiration inhibitors:

(a) Complex I NADH oxidoreductase inhibitors: diflumetorim; and tolfenpyrad;
(b) Complex II succinic acid dehydrogenase inhibitors: benodanil, flutolanil, mepronil; isofetamid; fluopyram; fenfuram, furmecyclox; carboxin, oxycarboxin; thifluzamide; benzovindiflupyr; bixafen, fluxapyroxad, furametpyr, isopyrazam, penflufen, penthiopyrad, sedaxane; boscalid, and pyraziflumid;
(c) Complex III ubiquinol oxidase Qo inhibitors: azoxystrobin, coumoxystrobin, coumethoxystrobin, enoxastrobin, flufenoxystrobin, picoxystrobin, pyraoxystrobin; pyraclostrobin, pyrametostrobin, triclopyricarb; kresoxim-methyl, trifloxystrobin; dimoxystrobin, fenaminstrobin, metominostrobin, orysastrobin; famoxadone; fluoxastrobin; fenamidone; pyribencarb, and mandestrobin;
(d) Complex III ubiquinol reductase Qi inhibitors: cyazofamid, and amisulbrom;
(e) Oxidative phosphorylation uncoupling agents: binapacryl, meptyldinocap, dinocap; fluazinam; and ferimzone;
(f) Oxidative phosphorylation inhibitors (ATP synthase inhibitors): fentin acetate, fentin chloride, and fentin

hydroxide;
(g) ATP production inhibitors: silthiofam; and
h) Complex III: cytochrome bcl (ubiquinone reductase) Qx (unknown) inhibitors: ametoctradin.

(4) Amino acid and protein synthesis inhibitors:

(a) Methionine biosynthesis inhibitors: andoprim, cyprodinil, mepanipyrim, and pyrimethanil; and
(b) Protein synthesis inhibitors: blasticidin S; kasugamycin, kasugamycin hydrochloride; streptomycin; and oxytetracycline.

(5) Signal transduction inhibitors:

(a) Signal transduction inhibitors: quinoxyfen and proquinazid; and
(b) MAP/histidine kinase inhibitors in osmotic pressure signal transduction: fenpiclonil, fludioxonil, chlozolinate, iprodione, procymidone, and vinclozolin.

(6) Lipid and cell membrane synthesis inhibitors:

(a) Phospholipid biosynthesis and methyltransferase inhibitors: edifenphos, iprobenfos, pyrazophos; and isoprothiolane;
(b) Lipid peroxidation agents: biphenyl, chloroneb, dichloran, quintozene, tecnazene, tolclofos-methyl; and etridiazole;
(c) Agents that act upon cell membranes: iodocarb, propamocarb, propamocarb-hydrochloride, propamocarb-fosetylate, and prothiocarb;
(d) Microorganisms that disturb pathogen cell membranes: Bacillus subtilis, Bacillus subtilis strain QST713, Bacillus subtilis strain FZB24, Bacillus subtilis strain MBI600, and Bacillus subtilis strain D747; and
(e) Agents that disturb cell membranes: Melaleuca alternifolia (tea tree) extract.

(7) Cell membrane sterol biosynthesis inhibitors:

(a) C14-position demethylation inhibitors in sterol biosynthesis: triforine; pyrifenox, pyrisoxazole; fenarimol, flurprimidol, nuarimol; imazalil, imazalil-sulfate, oxpoconazole, pefurazoate, prochloraz, triflumizole, viniconazole;
azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, penconazole, propiconazole, fluquinconazole, simeconazole, tebuconazole, tetraconazole, triadimefon, triadimenol, triticonazole; prothioconazole, voriconazole, and mefentrifluconazole;
(b) $\Delta$14 reductase and $\Delta 8 \rightarrow \Delta 7$-isomerase inhibitors in sterol biosynthesis: aldimorph, dodemorph, dodemorph acetate, fenpropimorph, tridemorph; fenpropidin, piperalin; and spiroxamine;
(c) 3-keto reductase inhibitors in C4-position demethylation in sterol biosynthesis systems: fenhexamid; and fenpyrazamine; and
(d) Squalene epoxidase inhibitors in sterol biosynthesis systems: pyributicarb; naftifene, and terbinafine.

(8) Cell wall synthesis inhibitors:

(a) Trehalase inhibitors: validamycin;
(b) Chitin synthase inhibitors: polyoxins and polyoxorim; and
(c) Cellulose synthase inhibitors: dimethomorph, flumorph, pyrimorph; benthiavalicarb, iprovalicarb, tolprocarb, valifenalate; and mandipropamide.

(9) Melanin biosynthesis inhibitors:

(a) Reductase inhibitors in melanin biosynthesis: fthalide; pyroquilon; and tricyclazole; and
(b) Anhydrase inhibitors in melanin biosynthesis: carpropamid; diclocymet; and fenoxanil.

(10) Host plant resistance-inducing agents:

(a) Agent that acts on salicylic acid biosynthetic pathway: acibenzolar-S-methyl; and

(b) Others: probenazole; tiadinil; isotianil; laminarin; and Reynoutria sachalinensis extract.

(11) Agents for which the mode of activity is unclear: cymoxanil, fosetyl-aluminum, phosphoric acid (phosphate), tecloftalam, triazoxide, flusulfamide, diclomezine, methasulfocarb, cyflufenamid, metrafenone, pyriofenone, dodine, dodine free base, and flutianil.

(12) Agents having multiple activities: copper (copper salts), bordeaux mixture, copper hydroxide, copper naphthalate, copper oxide, copper oxychloride, copper sulfate, sulfur, sulfur products, calcium polysulfide, ferbam, mancozeb, maneb, mancopper, metiram, polycarbamate, propineb, thiram, zineb, ziram; captan, captafol, folpet; chlorothalonil; dichlofluanid, tolylfluanid; guazatine, iminoctadine triacetate, iminoctadine trialbesilate; anilazine; dithianon; quinomethionate; and fluoroimide.

(13) Other agents: DBEDC, fluor folpet, guazatine acetate, bis(8-quinolinolato) copper (II), propamidine, chloropicrin, cyprofuram, agrobacterium, bethoxazin, diphenylamine, methyl isothiocyanate (MITC), mildiomycin, capsaicin, curfraneb, cyprosulfamide, dazomet, debacarb, dichlorophen, difenzoquat, difenzoquat methylsulfonate, flumetover, fosetyl calcium, fosetyl sodium, irumamycin, natamycin, nitrothal isopropyl, oxamocarb, propanosine sodium, pyrrolnitrin, tebufloquin, tolnifanide, zarilamide, algophase, amicarthiazol, oxathiapiprolin, metiram zinc, benthiazole, trichlamide, uniconazole, mildiomycin, oxyfenthiin, picarbutrazox, fenpicoxamid, dichlobentiazox, and quinofumelin.

Specific examples of plant growth regulators that can be mixed or used in combination with the formulation for controlling harmful organisms of the present invention are listed below.

1-Methylcyclopropane, 2,3,5-triiodobenzoic acid, IAA, IBA, MCPA, 4-CPA, 5-aminolevulinic acid, 6-benzylaminopurine, abscisic acid, aviglycine hydrochloride, ancymidol, butralin, calcium carbonate, calcium chloride, calcium formate, calcium peroxide, lime sulfur, calcium sulfate, chlormequat chloride, chlorpropham, choline chloride, cloprop, cyanamide, cyclanilide, daminozide, decyl alcohol, dichlorprop, dikegulac, dimethipin, diquat, ethephon, ethychlozate, flumetralin, flurprimidol, forchlorfenuron, gibberellin A, gibberellin A3, hymexazol, inabenfide, isoprothiolane, kinetin, maleic acid hydrazide, mefluidide, mepiquat chloride, oxidation type glutathione, paclobutrazol, pendimethalin, prohexadione calcium, prohydrojasmon, pyraflufen-ethyl, sintofen, sodium 1-naphthalene acetate, sodium cyanate, streptomycin, thidiazuron, triapenthenol, tribufos, trinexapac-ethyl, uniconazole P, and 1-nathtylacetamide.

In order to easily mix or use together with the insecticide mentioned above and the like, a composition is preferably prepared by mixing the formulation for controlling harmful organisms according to the present invention is mixed with the insecticide and the like.

Such a composition is a composition which contains a formulation for controlling harmful organisms containing at least one compound selected from the group consisting of compounds represented by formula (I) or salts thereof, as an active ingredient, and at least one active ingredient selected from the group consisting of insecticidal / acaricidal agents, nematicides, soil pesticides, and fungicides.

More particularly, the composition contains the formulation for controlling harmful organisms containing at least one compound selected from the group consisting of the compounds represented by formula (I) or salts thereof, as an active ingredient, and at least one active ingredient selected from the group consisting of (1) acetylcholine esterase inhibitor, (2) GABA-gated chloride ion channel antagonists, (3) sodium channel modulators, (4) nicotinic acetylcholine receptor agonists, (5) nicotinic acetylcholine receptor allosteric modulators, (6) chloride channel activators, (7) juvenile hormone-like substances, (8) other nonspecific inhibitors, (9) homoptera selective antifeedants, (10) acari growth inhibitors, (11) microorganism-derived insect midgut inner membrane distrupting agents, (12) mitochondria ATP biosynthesis enzyme inhibitors, (13) oxidative phosphorylation decouplers, (14) nicotinic acetylcholine receptor channel blockers, (15) chitin synthesis inhibitors, (16) diptera molting disruptors, (17) molting hormone receptor agonists, (18) octopamine receptor agonists, (19) mitochondria electron transfer chain complex III inhibitors, (20) mitochondria electron transfer chain complex I inhibitors, (21) voltage-dependent sodium channel blockers, (22) acetyl CoA carboxylase inhibitors, (23) mitochondria electron transfer chain complex IV inhibitors, (24) mitochondria electron transfer chain complex II inhibitors, (25) ryanodine receptor modulators, (26) mixed function oxidase inhibitor compounds, (27) latrophilin receptor agonists, and (28) others (action mechanism is unknown).

[Formulation for controlling ectoparasites]

The formulation for controlling ectoparasites according to the present invention contains at least one compound selected from the oxadiazoline compounds of the present invention as an active ingredient. The amount of the oxadiazoline compounds contained in the formulation for controlling ectoparasites of the present invention is not particularly limited, as long as effects of controlling ectoparasites are exhibited.

As examples of the host animals for which the formulation for controlling ectoparasites of the present invention

is applicable, mention may be made of warm-blooded animals such as a pet animal such as a dog or a cat; a pet bird; a farm animal such as cattle, horse, pig, or sheep; domestic fowl; and the like. In addition, fish such as salmon, trout, puffer, carp, and the like; and insects such as honey-bees, stag beetles, unicorn beetles, and the like may be mentioned.

The formulation for controlling ectoparasites of the present invention can be applied by a known veterinary method (topical, oral, parenteral or subcutaneous administration). Examples of the method include a method for orally administering tablets, capsules and drinks mixed with the formulation for controlling ectoparasites to the animals; a method for administering to the animals by using an immersion liquid, suppository or injection (intramuscular, subcutaneous, intravenous, intraabdominal or the like); a method for topically administering an oil-based or aqueous liquid preparation by spraying, pouring on, spotting on or the like; a method for topically administering by attaching a collar, an ear tag or the like made by molding a mixture obtained by kneading the formulation for controlling ectoparasites with a resin to the animals; and the like.

The ectoparasites live on the host animals, especially live inside the body or on the skin of warm-blooded animals or fish. More specifically, the ectoparasites are parasitic in the back, armpit, underbelly, inner thigh and the like of the host animals and obtain nutritional sources such as blood, dandruff from the animals to live.

As examples of ectoparasites, mention may be made of mites, lice, fleas, mosquitoes, stable flies, flesh flies, Japanese fishlouse (*Argulus japonicas*) and the like.

Specific examples of the ectoparasites which can be prevented by the formulation for controlling ectoparasites according to the present invention are described below.

(1) Acari

Acari belonging to the Dermanyssidae family, acari belonging to the Macronyssidae family, acari belonging to the Laelapidae family, acari belonging to the Varroidae family, acari belonging to the Argasidae family, acari belonging to the Ixodidae family, acari belonging to the Psoroptidae family, acari belonging to the Sarcoptidae family, acari belonging to the Knemidokoptidae family, acari belonging to the Demodixidae family, acari belonging to the Trombiculidae family, and insect-parasitic acari such as Coleopterophagus berlesei.

(2) Phthiraptera order

Lice belonging to the Haematopinidae family, lice belonging to the Linognathidae family, biting lice belonging to the Menoponidae family, biting lice belonging to the Philopteridae family, and biting lice belonging to the Trichodectidae family.

(3) Siphonaptera order

Fleas belonging to the Pulicidae family, for example, Ctenocephalides canis and Ctenocephalides felis of Ctenocephalides spp.;
Fleas belonging to the Tungidae family, fleas belonging to the Ceratophyllidae family, and fleas belonging to the Leptopsyllidae family.

(4) Hemiptera order.

(5) Harmful organisms of Diptera order

Mosquitoes belonging to the Culicidae family, black flies belonging to the Simuliidae family, punkie belonging to the Ceratopogonidae family, horseflies belonging to the Tabanidae family, flies belonging to the Muscidae family, tsetse flies belonging to the Glossinidae family, flesh flies belonging to the Sarcophagidae family, flies belonging to the Hippoboscidae family, flies belonging to the Calliphoridae family, and flies belonging to the Oestridae family.

[Formulation for Controlling Other Harmful Organisms]

In addition, the oxadiazoline compound of the present invention exhibits a superior effect for controlling other pests that have a sting or venom that can harm humans and animals, pests carrying various pathogens / pathogenic bacteria, and pests that impart a discomforting sensation to humans (such as toxic pests, sanitary insect pests, unpleasant insect pests).

Specific examples thereof are listed below.

(1) Hymenoptera insect pests

Sawflies of the Argidae family, wasps of the Cynipidae family, sawflies of the Diprionidae family, ants of the Formicidae family, wasps of the Mutillidae family, and wasps of the Vespidae family.

(2) Other insect pests

Blattodea, termites, Araneae, centipedes, millipedes, crustacea and Cimex lectularius.

EXAMPLES

[Formulation Examples]

Some examples of the formulations for controlling harmful organisms, insecticidal or acaricidal formulations, formulations for controlling Thysanoptera insect pests of the present invention are described below. The additives and the addition ratios are not limited to those in the formulation examples and can be modified over a wide range. The term "part" in the formulation examples indicates "part by weight".
The formulation examples for agricultural and horticultural use and for paddy rice are described below.

(Formulation Example 1: Wettable powder)

40 parts of the oxadiazoline compound of the present invention, 53 parts of diatomaceous earth, 4 parts of a higher alcohol sulfuric ester, and 3 parts of an alkylnaphthalene sulfonic acid salt were uniformly mixed and finely pulverized to obtain a wettable powder including 40% of an active ingredient.

(Formulation Example 2: Emulsion)

30 parts of the oxadiazoline compound of the present invention, 33 parts of xylene, 30 parts of dimethylformamide and 7 parts of a polyoxyethylene alkyl aryl ether were mixed and dissolved to obtain an emulsion including 30% of an active ingredient.

(Formulation Example 3: Granules)

5 parts of the oxadiazoline compound of the present invention, 40 parts of talc, 38 parts of clay, 10 parts of bentonite and 7 parts of sodium alkylsulfate were uniformly mixed and finely pulverized, followed by granulating into a granular shape having a diameter of 0.5 to 1.0 mm to obtain granules containing 5% of an active ingredient.

(Formulation Example 4: Granules)

5 parts of the oxadiazoline compound of the present invention, 73 parts of clay, 20 parts of bentonite, 1 part of sodium dioctyl sulfosuccinate and 1 part of potassium phosphate were thoroughly pulverized and mixed. Water was added thereto, and the mixture was kneaded well, followed by granulating and drying to obtain granules containing 5% of an active ingredient.

(Formulation Example 5: Suspension)

10 parts of the oxadiazoline compound according to the present invention, 4 parts of polyoxyethylene alkyl allyl ether, 2 parts of sodium polycarboxylate, 10 parts of glycerol, 0.2 parts of xanthan gum and 73.8 parts of water were mixed and wet-pulverized so as to have a grain size of 3 microns or less. Thereby, a suspension containing 10% of an active ingredient was obtained.
The formulation examples of the formulation for controlling ectoparasites are described below.

(Formulation Example 6: Granulated powder)

5 parts of the oxadiazoline compound of the present invention was dissolved in an organic solvent to obtain a solution. The solution mentioned above was sprayed on 94 parts of kaolin and 1 part of white carbon, followed by evaporating the solvent under reduced pressure. This type of granulated powder may be mixed with animal food.

(Formulation Example 7: Impregnating formulation)

0.1 to 1 part of the oxadiazoline compound of the present invention and 99 to 99.9 parts of peanut oil were uniformly mixed, and then filter-sterilized by means of a sterilizing filter.

(Formulation Example 8: Pour-on formulation)

5 parts of the oxadiazoline compound of the present invention, 10 parts of a myristic ester and 85 parts of isopropanol were uniformly mixed to obtain a pour-on formulation.

(Formulation Example 9: Spot-on formulation)

10 to 15 parts of the oxadiazoline compound of the present invention, 10 parts of a palmitic ester and 75 to 80 parts of isopropanol were uniformly mixed to obtain a spot-on formulation.

(Formulation Example 10: Spray formulation)

1 part of the oxadiazoline compound of the present invention, 10 parts of propylene glycol and 89 parts of isopropanol were uniformly mixed to obtain a spray formulation.

Next, Examples of compounds are described to explain the present invention more specifically. It should be understood that the present invention is not limited to the following examples of compounds.

[Reference Example 1]

Synthesis of 1-(tert-butyl)-3-(6,11-dihydrodibenzo[b,e]oxepin-11-yl)thiourea

[Chem. 6]

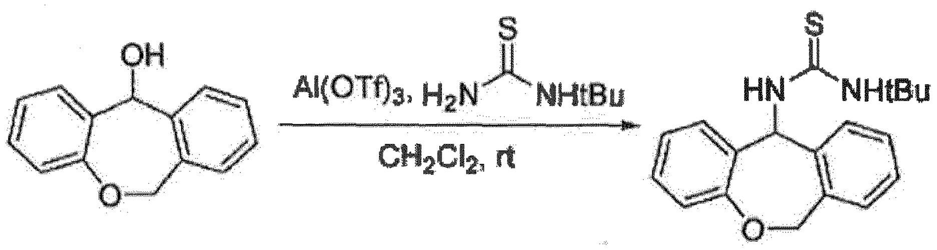


6,11-Dihydrodibenzo[b,e]oxepin-11-ol was synthesized in accordance with the method described in Chemical and Pharmaceutical Bulletin, 1991, 39, 0, 2564.

6,11-Dihydrodibenzo[b,e]oxepin-11-ol (4.2 g) was dissolved in dichloromethane (100 mL), and subsequently, 1-(t-butyl)thiourea (2.8 g) and aluminum trifluoromethane sulfonate (0.10 g) were added thereto. The mixture was stirred for 25 hours. Subsequently, the reaction solvent was distilled off. Subsequently, the residue was purified by column chromatography with silica gel. Thereby, 6.1 g of the objective product was obtained.
Melting point: 167 - 168°C.

[Reference Example 2]

Synthesis of N-tert-butyl-N-(6,11-dihydrodibenzo[b,e]oxepin-11-yl) methanediimine

[Chem. 7]

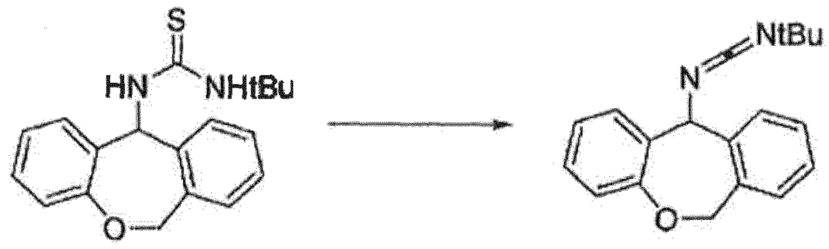

Triethylamine (6.8 ml) and 2-chloro-1-methylpyridinium iodide (6.3 g) were added to a solution of the thiourea (6.7 g) obtained in Reference Example 1 dissolved in acetonitrile (54 ml), and the mixture was stirred for 4 hours at room temperature. The insoluble material was removed by filtration, and the filtrate was concentrated under reduced pressure. The residue was purified by column chromatography with silica gel. Thereby, 4.7 g of the objective product was obtained.
$^1$H-NMR ($CDCl_3$) δ ppm: 1.06 (s, 9H), 4.94 (d, 1H), 5.51 (s, 1H), 5.87 (d, 1H), 6.90 (m, 2H), 7.20 (m, 1H), 7.33 (m, 5H).

[Example 1]

Synthesis of N-(tert-butyl)-2'-methyl-2'H,6H-spiro[dibenzo[b,e]oxepine-11,5'-[1,2,4] oxadiazol]-3'-amine (Compound No. a-11)

[Chem. 8]

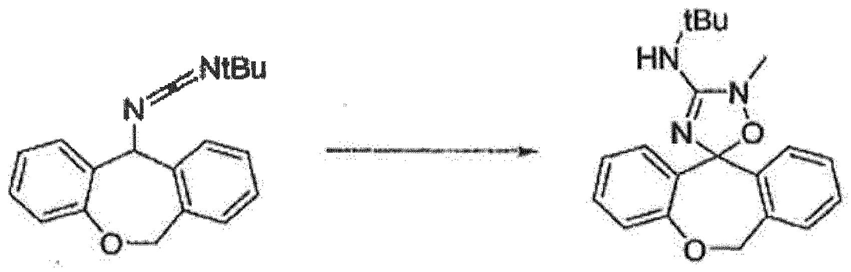

N-Methylhydroxyamine hydrochloride (1.6 g) and triethylamine (2.6 ml) were added to a solution of carbodiimide (4.7 g) obtained in Reference Example 2 dissolved in acetonitrile (53 ml), and the mixture was stirred for 3 hours at room temperature. The reaction solution was concentrated under reduced pressure, and subsequently, dichloromethane (80 ml) and manganese dioxide (1.4 g) were added thereto. The mixture was stirred for 4 hours at room temperature. The reaction solution was filtered with celite. The insoluble material was washed with ethyl acetate. The filtrate was concentrated under reduced pressure. The crude product was washed with hexane. Thereby, 4.6 g of the objective product was obtained.
$^1$H-NMR ($CDCl_3$) δ ppm: 1.58 (s, 9H), 2.82 (s, 3H), 3.78 (s, 1H), 5.05 (d, 1H), 6.02 (d, 1H), 6.83 (d, 1H), 6.90 (t, 1H), 7.18 (t, 1H), 7.28 (m, 3H), 7.77 (m, 2H).

[Example 2]

Synthesis of N-(tert-butyl)-N-ethyl-2'-methyl-2'H,6H-spiro [dibenzo[b,e]oxepine-11,5'-[1,2,4]oxadiazol]-3'-amine (Compound No. a-10)

[Chem. 9]

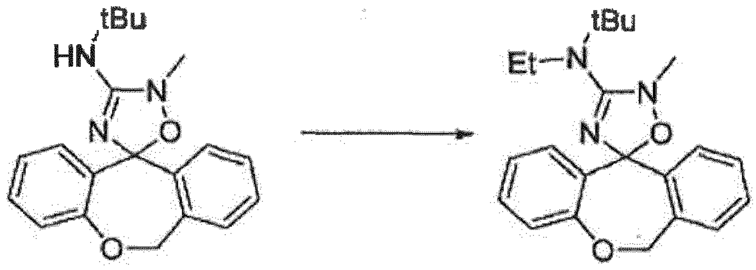

Sodium hydride (0.8 g) was added to a solution of the amine (4.6 g) obtained in Example 1 dissolved in DMF (45 ml) at 0°C, and the mixture was stirred for 30 minutes. Ethyl iodide (2.1 ml) was added thereto, and the mixture was stirred for 3 hours at 0°C. The reaction solution was added to water with ice. The mixture was subjected to extraction with ethyl acetate. The organic layer was washed with a saturated aqueous solution of sodium chloride, dried over anhydrous magnesium sulfate, and filtered. Subsequently, the filtrate was concentrated under reduced pressure. The obtained crude product was purified by column chromatography with silica gel. Thereby, 2.5g of the objective product was obtained.
$^1$H-NMR ($CDCl_3$) $\delta$ ppm: 1.17 (t, 3H), 1.62 (s, 9H), 2.78 (s, 3H), 3.36 (q, 2H), 5.12 (d, 1H), 6.02 (d, 1H), 6.85 (m, 2H), 7.19 (t, 1H), 7.28 (m, 3H), 7.77 (m, 2H).

[Reference Example 3]

Synthesis of 1-(tert-butyl)-3-(10,11-dihydrodibenzo[b,f]oxepin-10-yl)thiourea

[Chem. 10]

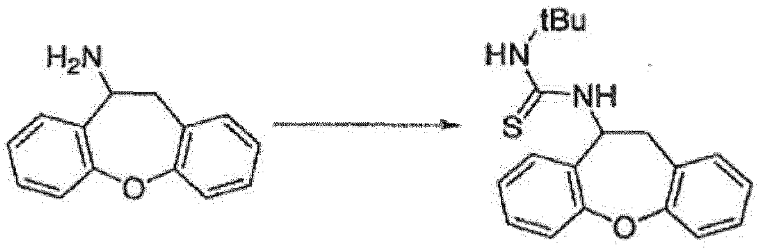

10,11-Dihydro-dibenz[b,f]oxepin-10-ylamine was synthesized in accordance with the method described in Angewandte Chemie - International Edition, 2015, 54, 17, 5049.
10,11-Dihydro-dibenz[b,f]oxepin-10-ylamine (0.59 g) was dissolved in tetrahydrofuran (10 ml), and subsequently, t-butyl isothiocyanate (0.55 g) was added thereto. The mixture was stirred for 17 hours at room temperature, and subsequently, the solvent was distilled off therefrom. The residue was purified by column chromatography with silica gel. Thereby, 0.10 g of the objective product was obtained.

[Example 3]

Synthesis of N-(tert-butyl)-2'-methyl-2'H,11H-spiro[dibenzo[b,f] oxepine-10,5'-[1,2,4]oxadiazol]-3'-amine (Compound No. a-22)

[Chem. 11]

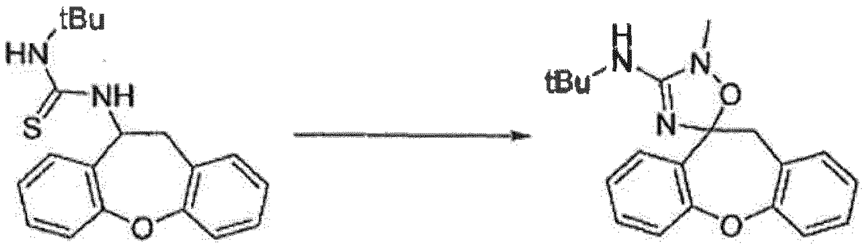

The thiourea (0.10 g) obtained in Reference Example 3 was dissolved in acetonitrile (3 ml). Subsequently, triethylamine (88 mg) and 2-chloro-1-methylpyridinium iodide (0.11 g) were added thereto, and the mixture was stirred for 4 hours at room temperature. N-methylhydroxyamine hydrochloride (48 mg) and triethylamine (58 mg) were added to the reaction solution, and the mixture was stirred for 18 hours at room temperature. The reaction solution was concentrated under reduced pressure. Subsequently, the residue was purified by column chromatography with silica gel. Thereby, 0.06 g of the objective product was obtained.

[Reference Example 4]

Synthesis of 2'-methyl-2'H,6H-spiro[dibenzo[b,e]oxepine-11,5'-[1,2,4]oxadiazol]-3'-amine

[Chem. 12]

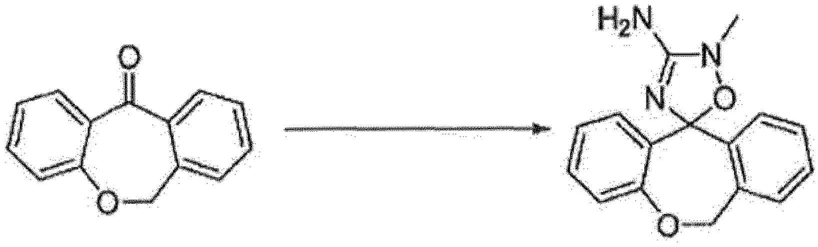

Dibenzo[b,e]oxepin-11(6H)-one (3.2 g) was dissolved in dichloromethane (30 ml), and subsequently, titanium tetrachloride (5.7 g) was added thereto. N,N-bis(trimethylsilyl)carbodiimide (5.6 g) was dropwise added thereto, and subsequently, the mixture was stirred for one hour at room temperature. Subsequently, water was added thereto to quench the reaction. The organic layer of the reaction mixture was extracted with dichloromethane, and the solvent thereof was distilled off under reduced pressure. The obtained crude product was dissolved in tetrahydrofuran (30 ml). Subsequently, N-hydroxyamine hydrochloride (2.5 g) and triethylamine (3.0 g) were added thereto, and the mixture was stirred for one hour at 60 °C. The solid material was removed therefrom by filtration. Subsequently, the solvent was distilled off under reduced pressure. The residue was purified by column chromatography with silica gel. Thereby, 2.9 g of the objective product was obtained.
$^1$H-NMR ($CDCl_3$) δ ppm: 2.98 (s, 3H), 5.22 (d, 1H), 5.82 (d, 1H), 6.87 (d, 1H), 6.94 (m, 1H), 7.24 - 7.30 (m, 4H), 7.76 - 7.82 (m, 2H).

[Example 4]

Synthesis of N-(tert-butyl)-2'-methyl-2'H,6H-spiro[dibenzo[b,e] oxepine-11,5'-[1,2,4]oxadiazol]-3'-amine (Compound No. a-11)

[Chem. 13]

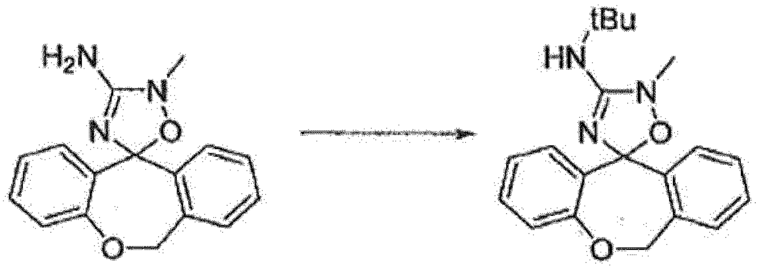

t-Butyl 2,2,2-trichloroacetimidate (0.13 ml) and boron trifluoride ethyl ether complex (46 μl) were added to a solution of the amine (0.1 g) obtained in Reference Example 4 dissolved in 1,2-dichloroethane (1.8 ml), and the mixture was stirred for 2 hours under refluxing by heating. The reaction solution was added to a saturated aqueous solution of sodium hydrogen carbonate, and the mixture was subjected to extraction with ethyl acetate. The organic layer was dried over anhydrous magnesium sulfate, and filtered. Subsequently, the filtrate was concentrated under reduced pressure. The obtained crude product was purified by column chromatography with silica gel. Thereby, 2.5 mg (yield 2%) of the objective product was obtained.

$^1$H-NMR ($CDCl_3$) δ ppm: 1.58 (s, 9H), 2.82 (s, 3H), 3.78 (s, 1H), 5.05 (d, 1H), 6.02 (d, 1H), 6.83 (d, 1H), 6.90 (t, 1H), 7.18 (t, 1H), 7.28 (m, 3H), 7.77 (m, 2H).

[Reference Example 5]

Synthesis of 1-(tert-butyl)-3-(6-fluoro-2,2-dimethylchroman-4-yl)thiourea

[Chem. 14]

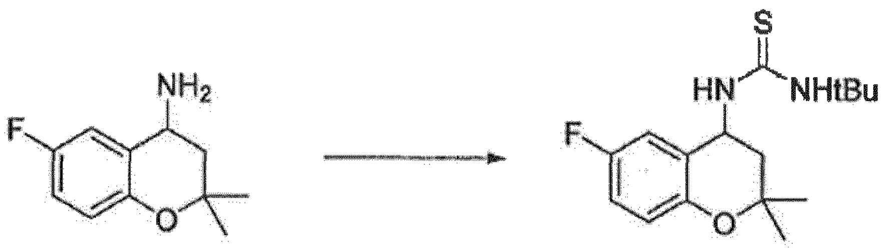

t-Butyl isothiocyanate (1.9 g) was added to a solution of 6-fluoro-2,2-dimethylchroman-4-amine (3.2 g) dissolved in dichloromethane (54 ml), and the mixture was stirred for 6 hours under refluxing by heating. The reaction solution was concentrated. The obtained solid was washed with ether. Thereby, 3.9 g of the objective product was obtained.

[Reference Example 6]

Synthesis of 2-(tert-butyl)-3-(6-fluoro-2,2-dimethylchroman-4-yl)-1-hydroxy-1-methylguanidine

[Chem. 15]

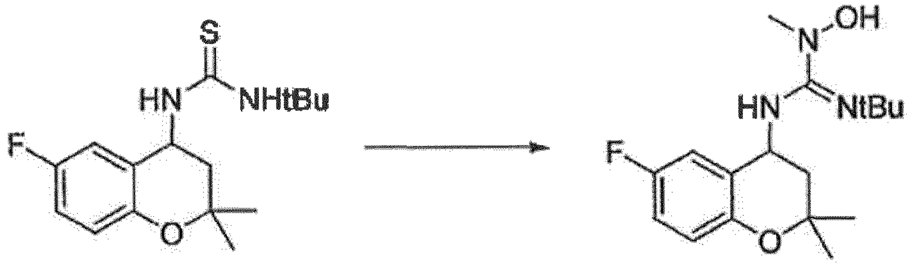

Triethylamine (2.0 ml) and 2-chloro-1-methylpyridinium iodide (1.8 g) were added to a solution of the thiourea (1.84 g) obtained in Reference Example 5 dissolved in acetonitrile (20 ml), and the mixture was stirred for 18 hours at room temperature. N-methylhydroxyamine hydrochloride (0.6 g) and pyridine (1.2 ml) were added to the reaction solution, and the mixture was stirred for 14 hours at room temperature. The reaction solution was added to water, and the mixture was subjected to extraction with ethyl acetate. The organic layer was washed with a saturated aqueous solution of sodium chloride, dried over anhydrous magnesium sulfate, and filtered. Subsequently, the filtrate was concentrated under reduced pressure. The obtained crude product was purified by column chromatography with silica gel. Thereby, 0.4 g of the objective product was obtained.

[Example 5]

Synthesis of N-(tert-butyl)-6-fluoro-2,2,2'-trimethyl-2'H-spiro[chromane-4,5'-[1,2,4]oxadiazol]-3'-amine (Compound No. a-20)

[Chem. 16]

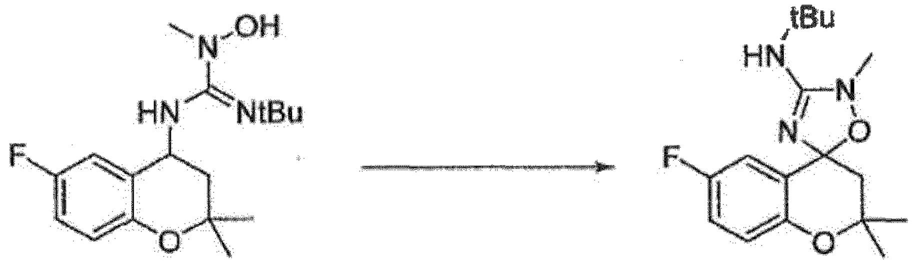

Manganese dioxide (0.4 g) was added to a solution of guanidine (0.4 g) obtained in Reference Example 6 dissolved in chloroform (10 ml), and the mixture was stirred for 3 days at room temperature. The reaction solution was filtered with celite, and the insoluble material was washed with ethyl acetate. The filtrate was concentrated under reduced pressure. The obtained crude product was purified by column chromatography with silica gel. Thereby, 0.15 g of the objective product was obtained.

Some of the oxadiazoline compounds according to the present invention (Compound Nos. a-1 to a-43) which were produced by the same methods as those described in the Examples mentioned above are shown below together with the physical properties (melting point or NMR).

[Chem. 17]

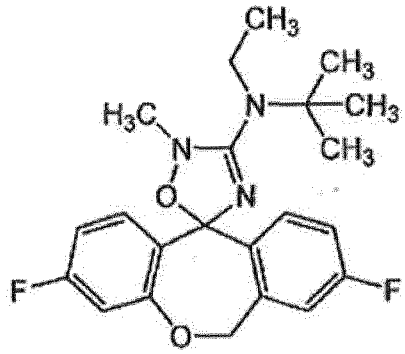

(a-1)

m.p. 95 - 96°C

[Chem. 18]

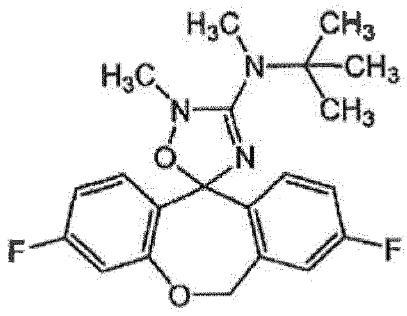

(a-2)

m.p. 113 - 114°C

[Chem. 19]

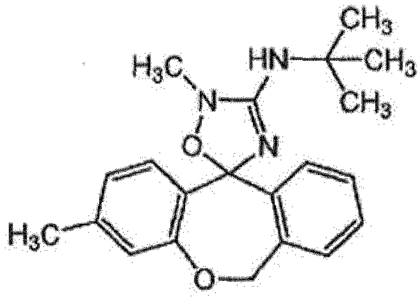

(a-3)

m.p. 154 - 156°C

[Chem. 20]

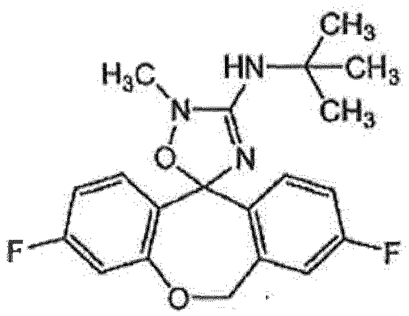

(a-4)

m.p. 130 - 132°C

[Chem. 21]

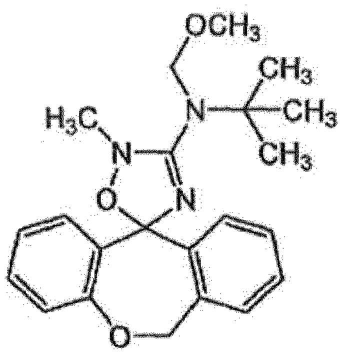

(a-5)

$^1$H-NMR (400 MHz, $CDCl_3$) δ ppm: 1.67 (s, 9H), 2.80 (s, 3H), 3.24 (s, 3H), 4.69 (d, 2H), 5.15 (d, 1H), 5.99 (d, 1H), 6.82 - 6.88 (m, 2H), 7.18 (m, 1H), 7.25 - 7.30 (m, 3H), 7.73 (m, 1H).

[Chem. 22]

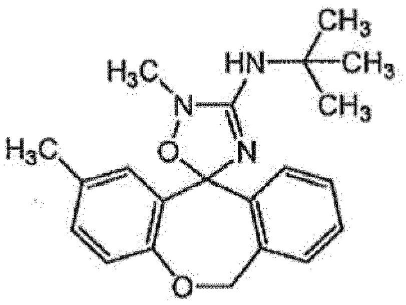

(a-6)

m.p. 175 - 177°C

[Chem. 23]

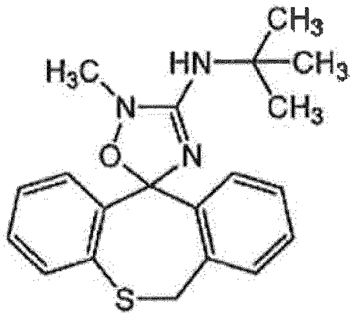

(a-7)

m.p.: 148 - 150°C

[Chem. 24]

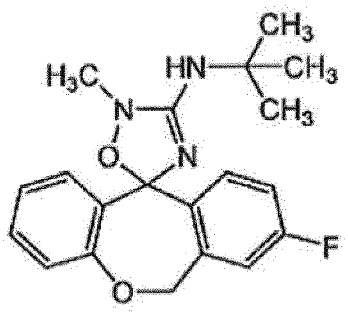

(a-8)

m.p.: 163 - 166°C

[Chem. 25]

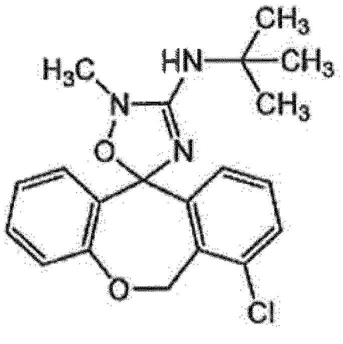

(a-9)

m.p.: 181 - 183°C

[Chem. 26]

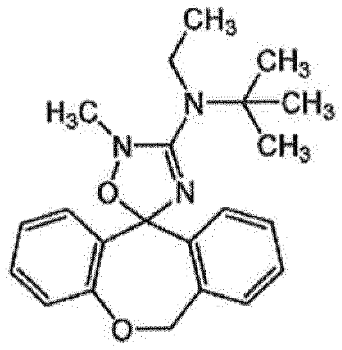

(a-10)

m.p.: 85 - 86°C

[Chem. 27]

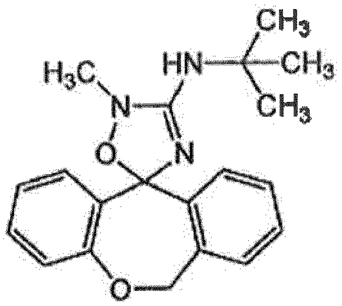

(a-11)

m.p.: 196 - 198°C

[Chem. 28]

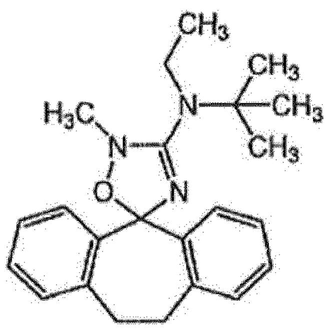

(a-12)

$^1$H-NMR (400 MHz, $CDCl_3$) $\delta$ ppm: 1.13 (t, 3H), 1.52 (s, 9H), 2.75 (s, 3H), 3.10 (m, 2H), 3.32 (q, 2H), 3.69 (m, 2H), 7.09 - 7.13 (m, 6H), 7.78 - 7.81 (m, 2H).

[Chem. 29]

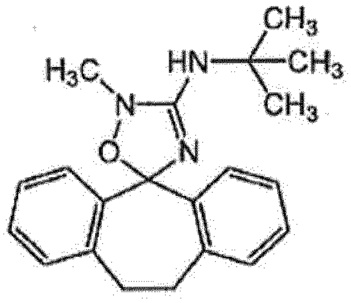

(a-13)

m.p.: 130 - 131°C

[Chem. 30]

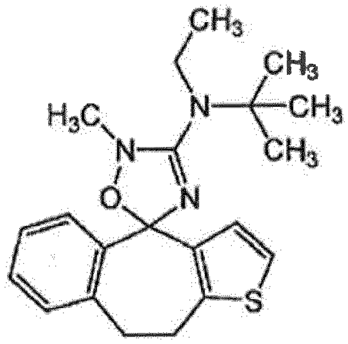

(a-14)

m.p.: 68 - 71°C

[Chem. 31]

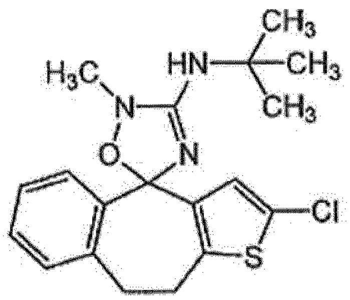

(a-15)

m.p.: 149 -151°C

[Chem. 32]

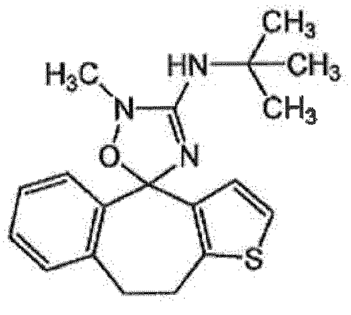

(a-16)

m.p.: 168 - 170°C

[Chem. 33]

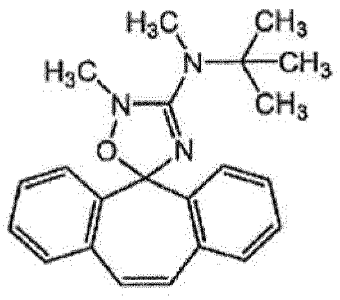

(a-17)

m.p.: 144 - 146°C

[Chem. 34]

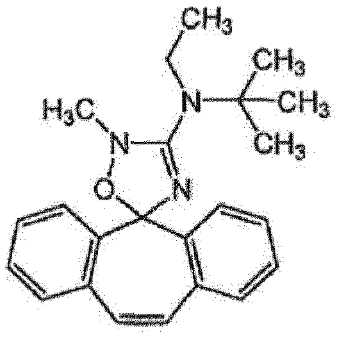

(a-18)

$^1$H-NMR (400 MHz, $CDCl_3$) δ ppm: 1.09 (t, 3H), 1.63 (s, 9H), 2.49 (s, 3H), 3.27 (q, 2H), 7.10 (s, 2H), 7.35 (m, 6H), 7.97 (d, 2H).

[Chem. 35]

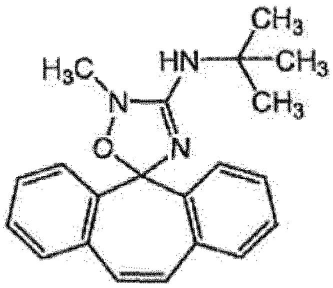

(a-19)

m.p.: 156 - 158°C

[Chem. 36]

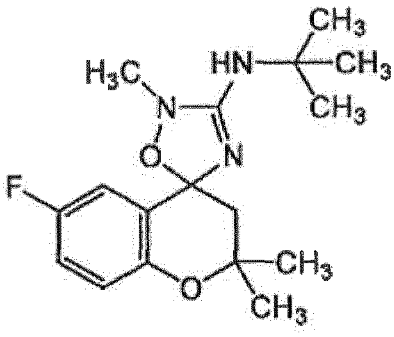

(a-20)

m.p.: 94 - 97°C

[Chem. 37]

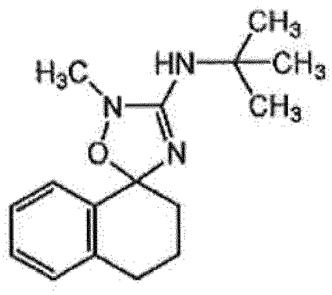

(a-21)

m.p.: 94.8 - 97.5°C

[Chem. 38]

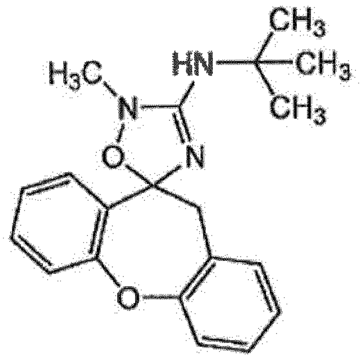

(a-22)

m.p.: 132 - 135°C

[Chem. 39]

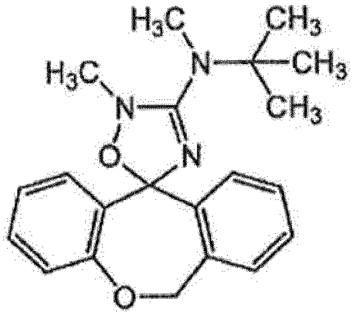

(a-23)

m.p.: 122 - 124°C

[Chem. 40]

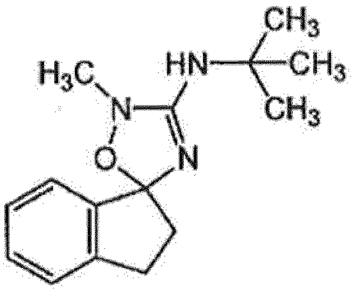

(a-24)

m.p.: 82 - 84°C

[Chem. 41]

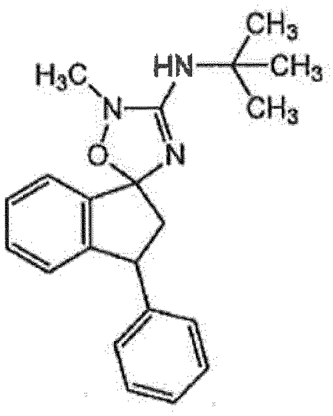

(a-25)

m.p.: 118 - 120°C

[Chem. 42]

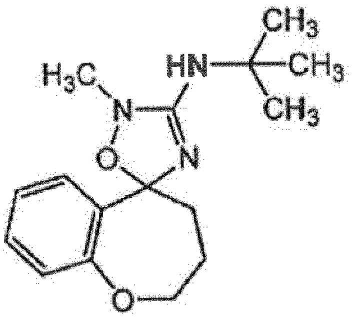

(a-26)

m.p.: 85 - 87°C

[Chem. 43]

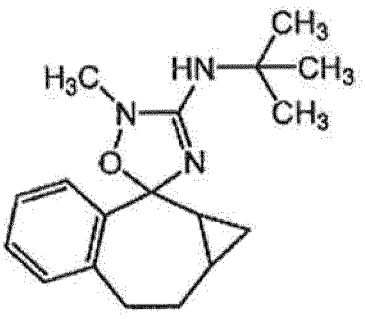

(a-27)

m.p.: 100 - 102°C

[Chem. 44]

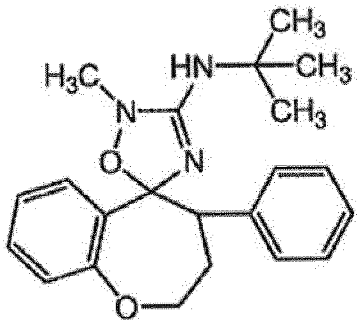

(a-28)

m.p.: 143 - 145°C

[Chem. 45]

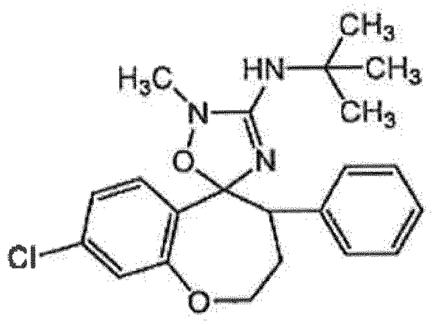

(a-29)

m.p.: 151 - 153°C

[Chem. 46]

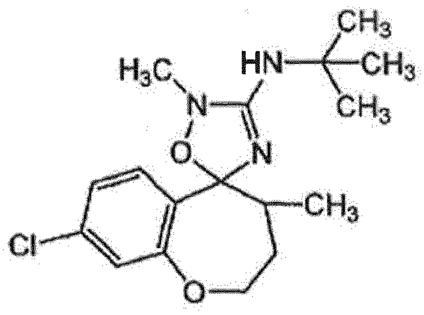

(a-30)

m.p.: 107 - 109°C

[Chem. 47]

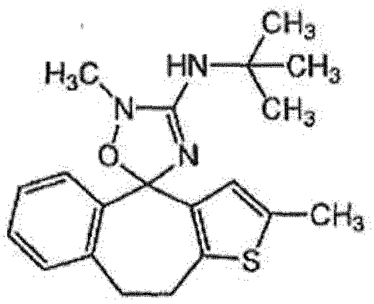

(a-31)

m.p.: 142 - 143°C

[Chem. 48]

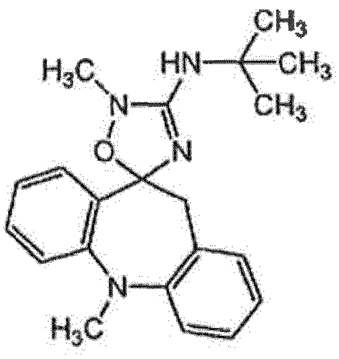

(a-32)

$^1$H-NMR (400 MHz, $CDCl_3$) δ ppm: 1.40 (s, 9H), 3.02 (s, 3H), 3.27 (d, 1H), 3.40 (s, 3H), 3.56 (d, 1H), 6.91 (t, 1H), 7.01 (m, 1H), 7.08 - 7.25 (m, 5H), 7.42 (d, 1H).

[Chem. 49]

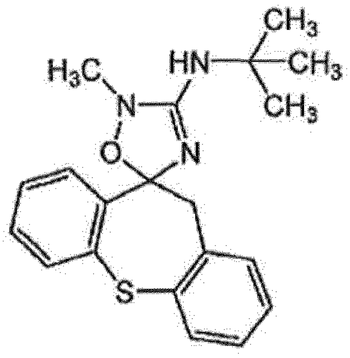

(a-33)

$^1$H-NMR (400 MHz, $CDCl_3$) δ ppm: 1.40 (s, 9H), 3.04 (s, 3H), 3.72 (d, 1H), 3.84 (d, 1H), 7.09 - 7.15 (m, 3H), 7.19 (m, 1H), 7.32 (d, 1H), 7.41 (d, 1H), 7.48 - 7.53 (m, 2H).

[Chem. 50]

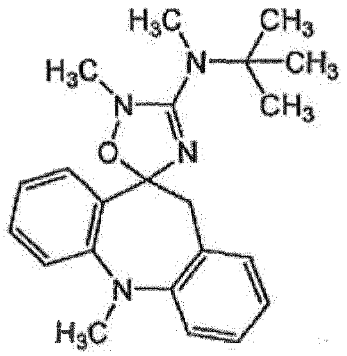

(a-34)

$^{1}H$-NMR (400 MHz, $CDCl_3$) δ ppm: 1.40 (s, 9H), 2.89 (s, 3H), 3.07 (s, 3H), 3.35 (d, 1H), 3.40 (s, 3H), 3.55 (d, 1H), 6.92 (t, 1H), 7.01 - 7.25 (m, 6H), 7.42 (d, 1H).

[Chem. 51]

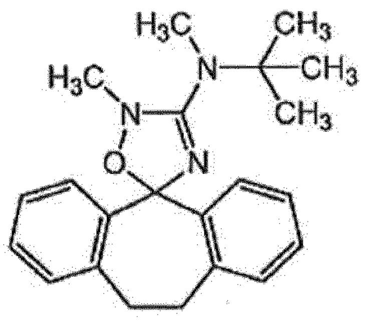

(a-35)

m.p.: 94 - 96°C

[Chem. 52]

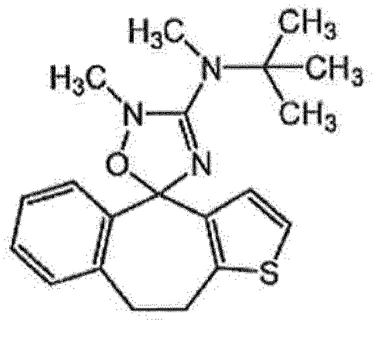

(a-36)

m.p.: 153 - 155°C

[Chem. 53]

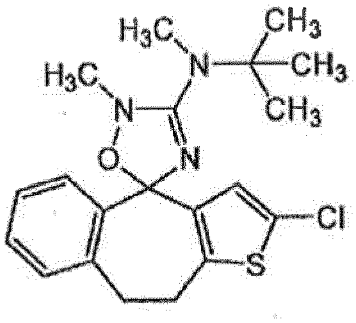

(a-37)

m.p.: 141 - 143°C

[Chem. 54]

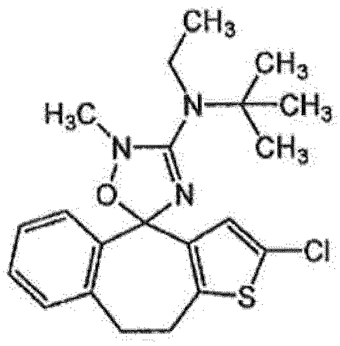

(a-38)

m.p.: 75 - 77°C

[Chem. 55]

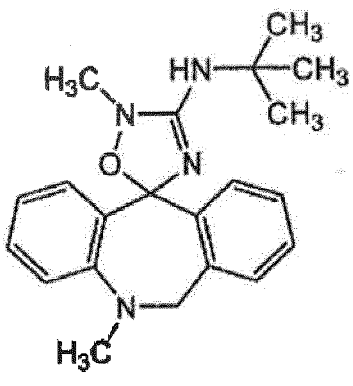

(a-39)

m.p.: 149 - 151°C

[Chem. 56]

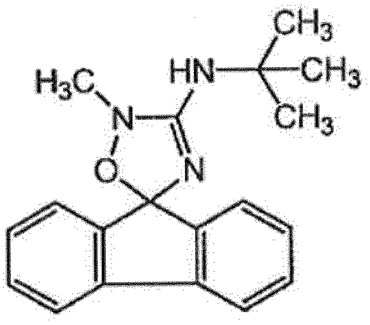

(a-40)

m.p.: 67 - 69°C

[Chem. 57]

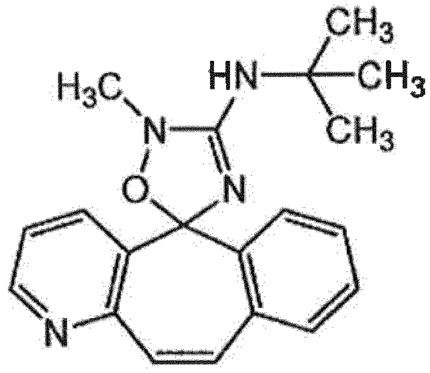

(a-41)

m.p.: 148 - 150°C

[Chem. 58]

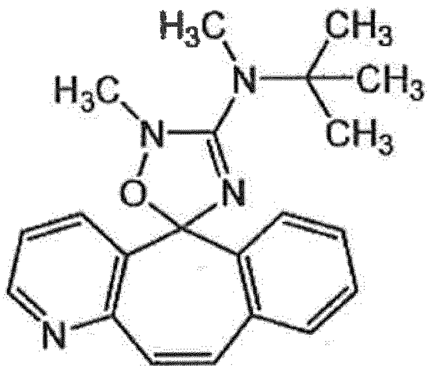

(a-42)

m.p.: 159 - 161°C

[Chem. 59]

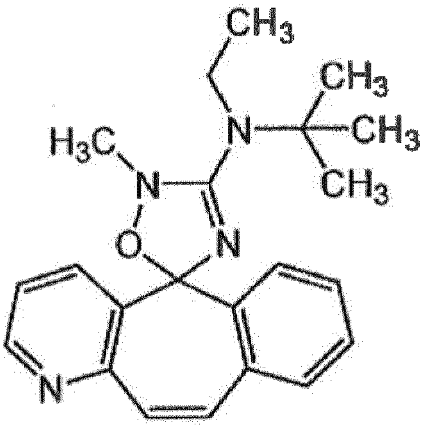

(a-43)

m.p.: 133 - 135°C

In addition, among the oxadiazoline compounds according to the present invention, some compounds represented by formula (II-a) are shown in Table 1, together with the physical properties (melting point, refractive index, or characteristic state).

In Table 1, Me represents a methyl group, Et represents an ethyl group, n-Pr represents an n-propyl group, t-Bu represents a t-butyl group, Bn represents a benzyl group, Ac represents an acetyl group, BOC represents a t-butoxycarbonyl group, Ph represents a phenyl group, and c-Pr represents a cyclopropyl group.

[Chem. 60]

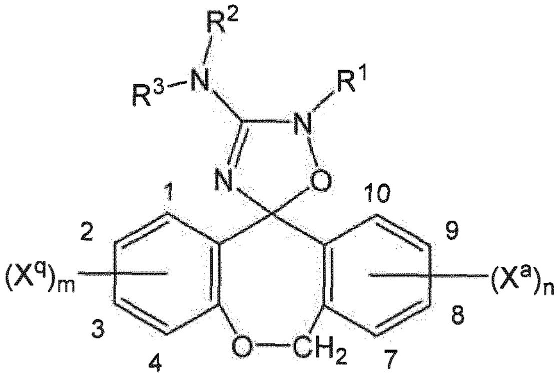

(II-a)

[Table 1]

| Table 1 | | | | | | |
|---|---|---|---|---|---|---|
| Compound No. | $R^1$ | $R^2$ | $R^3$ | $(X^q)m$ | $(X^a)n$ | Physical property |
| b-1 | Me | t-Bu | H | 2-F | - | m.p. 195-198°C |
| b-2 | Me | t-Bu | Et | 2-F | - | m.p. 105-107°C |
| b-3 | Me | t-Bu | H | 4-F | - | m.p. 175-177°C |
| b-4 | Me | t-Bu | Et | 4-F | - | m.p. 102-104°C |
| b-5 | Me | t-Bu | H | 2-Cl | - | m.p. 171-173°C |
| b-6 | Me | t-Bu | Et | 2-Cl | - | m.p. 68-70°C |
| b-7 | Me | t-Bu | H | 2-MeO | - | m.p. 145-146°C |
| b-8 | Me | t-Bu | Et | 2-MeO | | m.p. 76-78°C |

(continued)

| Table 1 | | | | | | |
|---|---|---|---|---|---|---|
| Compound No. | $R^1$ | $R^2$ | $R^3$ | $(X^q)m$ | $(X^a)n$ | Physical property |
| b-9 | Me | t-Bu | H | 3-F | - | m.p. 146-148°C |
| b-10 | Me | t-Bu | Et | 3-F | - | m.p. 90-92°C |
| b-11 | Me | t-Bu | H | 3-Cl | - | m.p. 135-137°C |
| b-12 | Me | t-Bu | Et | 3-Cl | - | m.p. 99-101 °C |
| b-13 | Me | t-Bu | H | 2-$CF_3O$ | - | m.p. 117-119°C |
| b-14 | Me | t-Bu | H | 3-$CF_3O$ | - | m.p. 138-140°C |
| b-15 | Me | t-Bu | H | - | 8-Cl | m.p. 174-176°C |
| b-16 | Me | t-Bu | H | 1,2-$F_2$ | - | m.p. 160-163 °C |
| b-17 | Me | t-Bu | H | 2-Et | - | m.p. 142-144°C |
| b-18 | Me | BOC | BOC | - | - | m.p. 150-152°C |
| b-19 | Me | BOC | H | - | - | m.p. 194-196°C |
| b-20 | Me | t-Bu | H | 3-Ph | - | m.p. 183-185°C |

[Table 2]

| Table 1 (continued) | | | | | | |
|---|---|---|---|---|---|---|
| Compound No. | $R^1$ | $R^2$ | $R^3$ | $(X^q)m$ | $(X^a)n$ | Physical property |
| b-21 | Me | t-Bu | H | 3-(c-Pr) | - | m.p. 78-80°C |
| b-22 | Me | t-Bu | H | 2-MeS | - | amorphous |
| b-23 | Me | t-Bu | H | 2-MeOC(=O)$CH_2$ | - | m.p. 120-122°C |
| b-24 | Me | t-Bu | H | 3-CN | - | m.p. 96-98°C |
| b-25 | Et | t-Bu | H | - | - | m.p. 188-190°C |
| b-26 | Et | t-Bu | Me | - | - | m.p. 112-114°C |
| b-27 | Et | t-Bu | Et | - | - | amorphous |
| b-28 | Me | t-Bu | Et | 3-$CF_3CH_2O$ | - | amorphous |
| b-29 | Me | t-Bu | H | 3-Br | - | m.p. 144-146°C |
| b-30 | Me | t-Bu | Et | 3-Br | - | amorphous |
| b-31 | Me | t-Bu | H | 4-Br | - | m.p. 192-194°C |
| b-32 | Me | t-Bu | H | 4-Me | - | m.p. 168-170°C |
| b-33 | Me | t-Bu | H | 4-Ph | - | amorphous |
| b-34 | Me | t-Bu | H | 3-MeS | - | m.p. 80-82°C |
| b-3 5 | Me | t-Bu | Et | 3-MeS | - | amorphous |
| b-36 | allyl | t-Bu | H | - | - | m.p. 148-150°C |
| b-37 | H | t-Bu | H | - | - | m.p. 200°C up |
| b-38 | Me | t-Bu | H | 4-CN | - | m.p. 179-181°C |
| b-39 | Me | t-Bu | H | 4-Cl | - | m.p. 188-191°C |
| b-40 | Me | t-Bu | Et | 3-MeS(=O) | - | amorphous |

(continued)

| Table 1 (continued) | | | | | | |
|---|---|---|---|---|---|---|
| Compound No. | $R^1$ | $R^2$ | $R^3$ | $(X^q)m$ | $(X^a)n$ | Physical property |
| b-41 | allyl | t-Bu | Me | - | - | m.p. 72-74°C |
| b-42 | allyl | t-Bu | Et | - | - | nD(20.9) 1.6231 |
| b-43 | H | t-Bu | Me | - | - | m.p. 43-45°C |
| b-44 | H | t-Bu | Et | - | - | nD(20.8) 1.5831 |
| b-45 | Me | t-Bu | H | 2,4-$Me_2$ | - | m.p. 196-198°C |
| b-46 | Me | t-Bu | Me | 2,4-$Me_2$ | - | m.p. 130-132°C |
| b-47 | Me | t-Bu | Et | 2,4-$Me_2$ | - | m.p. 143-145°C |
| b-48 | Me | t-Bu | Me | - | - | m.p. 122-124°C |
| b-49 | Me | t-Bu | Bn | - | - | m.p. 153-155°C |
| b-50 | Me | t-Bu | Me | 3-$CF_3O$ | - | amorphous |

[Table 3]

| Table 1 (continued) | | | | | | |
|---|---|---|---|---|---|---|
| Compound No. | $R^1$ | $R^2$ | $R^3$ | $(X^q)m$ | $(X^a)n$ | Physical property |
| b-51 | Me | t-Bu | Et | 3-$CF_3O$ | - | amorphous |
| b-52 | Me | t-Bu | Et | - | 7-Cl | m.p. 110-112°C |
| b-53 | Me | t-Bu | Me | - | 8-F | m.p. 118-119°C |
| b-54 | Me | t-Bu | Et | - | 8-F | m.p. 107-109°C |
| b-55 | Me | t-Bu | Me | 2-Me | - | m.p. 108-110°C |
| b-56 | Me | t-Bu | Et | 2-Me | - | amorphous |
| b-57 | Me | t-Bu | H | 3-F | - | m.p. 142-144°C |
| b-58 | Me | t-Bu | allyl | - | - | amorphous |
| b-59 | Me | t-Bu | n-Pr | - | - | amorphous |
| b-60 | Me | Me | Me | - | - | m.p. 150-154°C |
| b-61 | Me | t-Bu | H | 2-Br | - | m.p. 148-151°C |
| b-62 | Me | t-Bu | H | 2-Ph | - | m.p. 175-177°C |
| b-63 | Me | t-Bu | H | 2-MeOC(=O) | - | amorphous |
| b-64 | Me | t-Bu | H | 2-CN | - | m.p. 204-206°C |
| b-65 | Me | $-CH_2CH_2CH_2CH_2-$ | | - | - | m.p. 171-173°C |
| b-66 | Bn | t-Bu | H | - | - | m.p. 214-216°C |
| b-67 | Ac | t-Bu | Me | - | - | amorphous |
| b-68 | Me | $C(Me)_2Et$ | H | - | - | m.p. 140-142°C |
| b-69 | Me | $C(Me)_2Et$ | Me | - | - | m.p. 136-138°C |
| b-70 | Me | $C(Me)_2Et$ | Et | - | - | viscous oil |
| b-71 | Me | 1-Me-c-Pr | H | - | - | m.p. 160-162°C |
| b-72 | Me | $C(Me)_2CF_3$ | H | - | - | m.p. 166-168°C |

(continued)

| Table 1 (continued) | | | | | | |
|---|---|---|---|---|---|---|
| Compound No. | $R^1$ | $R^2$ | $R^3$ | $(X^q)m$ | $(X^a)n$ | Physical property |
| b-73 | Me | $C(Me)_2CF_3$ | Me | - | - | nD(21.9) 1.6192 |
| b-74 | Me | t-Bu | $EtOCH_2$ | - | - | nD(21.8) 1.6391 |
| b-75 | Me | t-Bu | $MeOCH_2CH_2OCH_2$ | - | - | nD(21.8) 1.6411 |
| b-76 | Me | $4\text{-}CF_3OPh$ | H | - | - | amorphous |
| b-77 | Me | 4-ClPh | H | - | - | amorphous |
| b-78 | Me | t-Bu | Me | 3-F | - | m.p. 110-112°C |
| b-79 | Me | t-Bu | t-Bu | - | - | m.p. 150-152°C |
| b-80 | Me | t-Bu | MeOCO | - | - | m.p. 128-130°C |

[Table 4]

| Table 1 (continued) | | | | | | |
|---|---|---|---|---|---|---|
| Compound No. | $R^1$ | $R^2$ | $R^3$ | $(X^q)m$ | $(X^a)n$ | Physical property |
| b-81 | Me | t-Bu | Me | 4-F | - | m.p. 166-167°C |
| b-82 | Me | t-Bu | H | $1,3\text{-}F_2$ | - | m.p. 114-116°C |
| b-83 | Me | t-Bu | Et | $1,3\text{-}F_2$ | - | amorphous |
| b-84 | Me | t-Bu | H | $3,4\text{-}F_2$ | - | m.p. 152-154°C |
| b-85 | Me | t-Bu | Me | $3,4\text{-}F_2$ | - | m.p. 135-136°C |
| b-86 | Me | t-Bu | Et | $3,4\text{-}F_2$ | - | m.p. 102-104°C |
| b-87 | Me | t-Bu | $MeSCH_2$ | - | - | nD(21.3) 1.6314 |
| b-88 | Me | i-Pr | H | - | - | m.p. 178-180°C |
| b-89 | Me | i-Pr | Me | - | - | m.p. 165-167°C |
| b-90 | Me | i-Pr | Et | - | - | m.p. 103-105°C |
| b-91 | Me | i-Pr | $MeOCH_2$ | - | - | nD(21.4) 1.6219 |
| b-92 | Me | t-Bu | Me | 4-Cl | - | m.p. 161-163°C |
| b-93 | Me | t-Bu | Et | 4-Cl | - | m.p. 97-99°C |
| b-94 | Me | t-Bu | Me | 4-Br | - | m.p. 148-150°C |
| b-95 | Me | t-Bu | Et | 4-Br | - | amorphous |
| b-96 | Me | t-Bu | Me | 4-Me | - | m.p. 150-152°C |
| b-97 | Me | t-Bu | Et | 4-Me | - | amorphous |
| b-98 | Me | t-Bu | H | - | 7-F | m.p. 168-170°C |
| b-99 | Me | t-Bu | H | 4-F | 7-F | m.p. 189-191°C |
| b-100 | Me | t-Bu | Me | - | 7-F | m.p. 136-138°C |
| b-101 | Me | t-Bu | Me | 4-F | 7-F | m.p. 148-150°C |
| b-102 b-103 | Me | t-Bu | Et | - | 7-F | m.p. 108-110°C |
| | Me | t-Bu | Et | 4-F | 7-F | nD(21.1) 1.6192 |
| b-104 | Me | t-Bu | $MeOCH_2$ | - | 7-F | nD(21.2) 1.6322 |

(continued)

| Table 1 (continued) | | | | | | |
|---|---|---|---|---|---|---|
| Compound No. | $R^1$ | $R^2$ | $R^3$ | $(X^q)m$ | $(X^a)n$ | Physical property |
| b-105 | Me | t-Bu | $MeOCH_2$ | 4-F | 7-F | nD(21.1) 1.6387 |
| b-106 | Me | t-Bu | Me | 4-Vinyl | - | m.p. 107-110°C |
| b-107 | Me | t-Bu | Me | 4-Et | - | amorphous |
| b-108 | Me | t-Bu | Propargyl | 4-F | - | nD(20.4) 1.6128 |
| b-109 | Me | t-Bu | Et | 3-Et | - | amorphous |
| b-110 | Me | t-Bu | H | 3-MeO | - | amorphous |

[Table 5]

| Table 1 (continued) | | | | | | |
|---|---|---|---|---|---|---|
| Compound No. | $R^1$ | $R^2$ | $R^3$ | $(X^q)m$ | $(X^a)n$ | Physical property |
| b-111 | Me | t-Bu | Me | 3-MeO | - | amorphous |
| b-112 | Me | t-Bu | Et | 3-MeO | - | amorphous |
| b-113 | Me | t-Bu | H | 4-MeO | - | m.p. 194-196°C |
| b-114 | Me | t-Bu | Me | 4-MeO | - | m.p. 183-185°C |
| b-115 | Me | t-Bu | Et | 4-MeO | - | m.p. 55-57°C |
| b-116 | Me | t-Bu | $MeOCH_2$ | 4-MeO | - | nD(21.1) 1.6187 |
| b-117 | Me | t-Bu | n-Pr | 4-F | - | nD(20.8) 1.6042 |
| b-118 | Me | t-Bu | n-Pr | - | 7-F | m.p. 96-98°C |
| b-119 | Me | t-Bu | H | - | 7-Br | m.p. 196-198°C |
| b-120 | Me | t-Bu | Me | - | 7-Br | nD(20.9) 1.6151 |
| b-121 | Me | t-Bu | Et | - | 7-Br | nD(20.9) 1.6204 |
| b-122 | Me | t-Bu | H | - | 7-Me | m.p. 200-202°C |
| b-123 | Me | t-Bu | Me | - | 7-Me | m.p. 120-122°C |
| b-124 | Me | t-Bu | Et | - | 7-Me | nD(21.1) 1.6198 |
| b-125 | Me | t-Bu | H | - | 7-Et | m.p. 172-174°C |
| b-126 | Me | t-Bu | Me | - | 7-Et | nD(21.1) 1.6231 |
| b-127 | Me | t-Bu | Et | - | 7-Et | nD(21) 1.6291 |
| b-128 | Me | t-Bu | H | - | 7-Vinyl | m.p. 195-197°C |
| b-129 | Me | t-Bu | Me | - | 7-Vinyl | nD(21.3) 1.6135 |
| b-130 | Me | t-Bu | Et | - | 7-Vinyl | nD(21.2) 1.6187 |
| b-131 | Me | t-Bu | H | - | 10-F | m.p. 144-146°C |
| b-132 | Me | t-Bu | Me | - | 10-F | m.p. 116-118°C |
| b-133 | Me | t-Bu | Et | - | 10-F | nD(20.1) 1.6142 |
| b-134 | Me | t-Bu | n-Pr | - | 10-F | m.p. 97-99°C |
| b-135 | Me | $C(Me)_2Et$ | H | 4-F | - | m.p. 121-122°C |
| b-136 | Me | $C(Me)_2Et$ | H | 4-Me | - | m.p. 119-120°C |

(continued)

| Table 1 (continued) | | | | | | |
|---|---|---|---|---|---|---|
| Compound No. | $R^1$ | $R^2$ | $R^3$ | $(X^q)m$ | $(X^a)n$ | Physical property |
| b-137 | Me | t-Bu | Me | 3-Me | - | m.p. 58-60°C |
| b-138 | Me | t-Bu | Et | 3-Me | - | amorphous |
| b-139 | Me | t-Bu | n-Pr | 3-Me | - | amorphous |
| b-140 | Me | $C(Me)_2Et$ | Me | 4-F | - | m.p. 109-111°C |

[Table 6]

| Table 1 (continued) | | | | | | |
|---|---|---|---|---|---|---|
| Compound No. | $R^1$ | $R^2$ | $R^3$ | $(X^q)m$ | $(X^a)n$ | Physical property |
| b-141 | Me | $C(Me)_2Et$ | Et | 4-F | - | amorphous |
| b-142 | Me | $C(Me)_2Et$ | Me | 4-Me | - | m.p. 115-117°C |
| b-143 | Me | $C(Me)_2Et$ | Et | 4-Me | - | amorphous |
| b-144 | Me | $C(Et)_2Me$ | H | - | - | m.p. 52-54°C |
| b-145 | Me | $C(Me)_2Ph$ | H | - | - | m.p. 197-198°C |
| b-146 | Me | $C(Me)_2CH_2$(t-Bu) | H | - | - | m.p. 136-137°C |
| b-147 | Me | $C(Me)_2$(nPr) | H | - | - | m.p. 142-144°C |
| b-148 | Me | $C(Me)_2CH_2$(t-Bu) | Me | - | - | m.p. 116-117°C |
| b-149 | Me | $C(Me)_2CH_2$(t-Bu) | Et | - | - | amorphous |
| b-150 | Me | $C(Me)_2Ph$ | Me | - | - | m.p. 167-168°C |
| b-151 | Me | $C(Me)_2Ph$ | Et | - | - | m.p. 160-161°C |
| b-152 | Me | $C(Me)_2Ph$ | n-Pr | - | - | m.p. 154-155°C |
| b-153 | Me | $C(Et)_2Me$ | Me | - | - | nD(21.9) 1.6031 |
| b-154 | Me | $C(Et)_2Me$ | Et | - | - | nD(21.9) 1.6124 |
| b-155 | Me | $C(Et)_2Me$ | n-Pr | - | - | nD(21.9) 1.6193 |
| b-156 | Me | $C(Me)_2$(n-Pr) | Me | - | - | nD(22.1) 1.6138 |
| b-157 | Me | $C(Me)_2$(n-Pr) | Et | - | - | m.p. 85-87°C |
| b-158 | Me | $C(Me)_2$(n-Pr) | n-Pr | - | - | nD(22) 1.6211 |
| b-159 | Me | t-Bu | H | 4-Et | - | m.p. 152-153°C |
| b-160 | Me | $C(Me)_2Et$ | H | 4-Et | - | m.p. 142-143°C |
| b-161 | Me | t-Bu | Me | 2-Cl | - | m.p. 125-127°C |
| b-162 | Me | $C(Me)_2Et$ | Me | 2-Cl | - | m.p. 112-114°C |
| b-163 | Me | $C(Me)_2Et$ | Et | 2-Cl | - | amorphous |
| b-164 | Me | $C(Me)_2Et$ | H | 2-Cl | - | m.p. 169-171°C |
| b-165 | Me | t-Bu | Et | 4-Et | - | amorphous |
| b-166 | Me | $C(Me)_2Et$ | Me | 4-Et | - | m.p. 73-75°C |
| b-167 | Me | $C(Me)_2Et$ | Et | 4-Et | - | amorphous |
| b-168 | Me | $C(Me)_2Et$ | H | - | 8-F | m.p. 137-138°C |

(continued)

| Table 1 (continued) | | | | | | |
|---|---|---|---|---|---|---|
| Compound No. | $R^1$ | $R^2$ | $R^3$ | $(X^q)m$ | $(X^a)n$ | Physical property |
| b-169 | Me | $C(Me)_2Et$ | H | 3-F | 8-F | amorphous |
| b-170 | Me | $C(Me)_2Et$ | H | 3-Me | - | m.p. 74-76°C |

[Table 7]

| Table 1 (continued) | | | | | | |
|---|---|---|---|---|---|---|
| Compound No. | $R^1$ | $R^2$ | $R^3$ | $(X^q)m$ | $(X^a)n$ | Physical property |
| b-171 | Me | $C(Me)_2$(n-Pr) | H | 3-Me | - | m.p. 60-62°C |
| b-172 | Me | $C(Me)_2Et$ | H | 3-Cl | - | m.p. 139-141°C |
| b-173 | Me | $C(Me)_2$(n-Pr) | H | 3-Cl | - | m.p. 116-118°C |
| b-174 | Me | $C(Me)_2Et$ | Me | 3-Me | - | amorphous |
| b-175 | Me | $C(Me)_2$(n-Pr) | Me | 3-Me | - | amorphous |
| b-176 | Me | $C(Me)_2Et$ | Et | 3-Me | - | amorphous |
| b-177 | Me | $C(Me)_2$(n-Pr) | Et | 3-Cl | - | m.p. 90-92°C |
| b-178 | Me | $C(Me)_2Et$ | Me | 3-Me | - | amorphous |
| b-179 | Me | $C(Me)_2$(n-Pr) | Me | 3-Cl | - | amorphous |
| b-180 | Me | $C(Me)_2Et$ | Et | 3-Cl | - | amorphous |
| b-181 | Me | $C(Me)_2Et$ | H | 3-F | - | m.p. 135-137°C |
| b-182 | Me | $C(Me)_2Et$ | Me | 3-F | - | m.p. 69-71°C |
| b-183 | Me | t-Bu | H | 4-(n-Pr) | - | m.p. 133-135°C |
| b-184 | Me | t-Bu | H | 4-(n-Bu) | - | m.p. 104-106°C |
| b-185 | Me | $C(Me)_2Et$ | Et | 3-F | - | nD(22.1) 1.6014 |
| b-186 | Me | $C(Me)_2Et$ | n-Pr | 3-F | - | nD(22.2) 1.6219 |
| b-187 | Me | $C(Me)_2$(n-Pr) | H | 3-F | - | m.p. 111-113°C |
| b-188 | Me | $C(Me)_2$(n-Pr) | Me | 3-F | - | nD(21.8) 1.6017 |
| b-189 | Me | $C(Me)_2$(n-Pr) | Et | 3-F | - | nD(22.3) 1.6113 |
| b-190 | Me | $C(Me)_2Et$ | H | - | 7-Cl | m.p. 100-102°C |
| b-191 | Me | $C(Me)_2Et$ | Me | - | 7-Cl | m.p. 138-140°C |
| b-192 | Me | $C(Me)_2Et$ | Et | - | 7-Cl | nD(21.9) 1.5918 |
| b-193 | Me | t-Bu | H | 3-Et | - | m.p. 113-115°C |
| b-194 | Me | t-Bu | Me | 3-Et | - | amorphous |
| b-195 | Me | t-Bu | Et | 3-Et | - | amorphous |
| b-196 | Me | $C(Me)_2Et$ | H | - | 7-F | m.p. 132-134°C |
| b-197 | Me | $C(Me)_2Et$ | Me | - | 7-F | m.p. 140-142°C |
| b-198 | Me | $C(Me)_2Et$ | Et | - | 7-F | nD(20.5) 1.5536 |
| b-199 | Me | $C(Me)_2Et$ | n-Pr | - | 7-F | nD(20.8) 1.5793 |

Among the compounds described above, with respect to the compounds which are in the form of an amorphous

or a viscous oil, the NMR data thereof are shown below.

Compound No. (b-22): $^1$H-NMR (400 MHz, $CDCl_3$) δ ppm: 1.59 (s, 9H), 2.41 (s, 3H), 2.82 (s, 3H), 3.83 (s, 1H), 5.05 (d, 1H), 5.99 (d, 1H), 6.79 (d, 1H), 7.15 (d, 1H), 7.31 (m, 3H), 7.77 (m, 2H).

Compound No. (b-27): $^1$H-NMR (400 MHz, $CDCl_3$) δ ppm: 1.02 (t, 3H), 1.31 (t, 3H), 1.60 (s, 9H), 2.87 (q, 2H), 3.15 (q, 2H), 5.01 (d, 1H), 6.15 (d, 1H), 6.81 (m, 2H), 7.15 (dd, 1H), 7.27 (m, 3H), 7.72 (m, 2H).

Compound No. (b-28): $^1$H-NMR (400 MHz, $CDCl_3$) δ ppm: 1.17 (t, 3H), 1.62 (s, 9H), 2.77 (s, 3H), 3.35 (q, 2H), 4.24 (q, 2H), 5.10 (d, 1H), 6.05 (d, 1H), 6.37 (d, 1H), 6.50 (d, 1H), 7.27 (m, 3H), 7.72 (m, 2H).

Compound No. (b-30): $^1$H-NMR (400 MHz, $CDCl_3$) δ ppm: 1.17 (t, 3H), 1.59 (s, 9H), 2.78 (s, 3H), 3.37 (q, 2H), 5.18 (d, 1H), 6.00 (d, 1H), 7.20 (m, 2H), 7.29 (m, 2H), 7.47 (d, 1H), 7.72 (m, 2H).

Compound No. (b-33): $^1$H-NMR (400 MHz, $CDCl_3$) δ ppm: 1.59 (s, 9H), 2.85 (s, 3H), 3.82 (s, 1H), 4.98 (d, 1H), 5.84 (d, 1H), 7.00 (t, 1H), 7.12 (m, 1H), 7.29 - 7.40 (m, 8H), 7.81 (m, 2H).

Compound No. (b-35): $^1$H-NMR (400 MHz, $CDCl_3$) δ ppm: 1.16 (t, 3H), 1.61 (s, 9H), 2.40 (s, 3H), 2.81 (s, 3H), 3.32 (q, 2H), 5.05 (d, 1H), 6.01 (d, 1H), 6.70 (d, 1H), 6.80 (dd, 1H), 7.28 (m, 3H), 7.68 (d, 1H), 7.75 (dd, 1H).

Compound No. (b-40): $^1$H-NMR (400 MHz, $CDCl_3$) δ ppm: 1.18 (t, 3H), 1.59 (s, 9H), 2.60 (s, 3H), 2.82 (s, 3H), 3.38 (q, 2H), 5.23 (dd, 1H), 6.11 (dd, 1H), 7.04 (d, 1H), 7.18 (dd, 1H), 7.29 (m, 3H), 7.78 (d, 1H), 7.95 (dd, 1H).

Compound No. (b-50): $^1$H-NMR (400 MHz, $CDCl_3$) δ ppm: 1.59 (s, 9H), 2.74 (s, 3H), 2.90 (s, 3H), 5.11 (d, 1H), 6.07 (d, 1H), 6.68 (m, 2H), 7.30 (m, 3H), 7.72 (m, 1H), 7.80 (d, 1H).

Compound No. (b-51): $^1$H-NMR (400 MHz, $CDCl_3$) δ ppm: 1.15 (t, 3H), 1.60 (s, 9H), 2.77 (s, 3H), 3.35 (q, 2H), 5.15 (d, 1H), 6.06 (d, 1H), 6.70 (m, 2H), 7.30 (m, 3H), 7.72 (m, 1H), 7.79 (d, 1H).

Compound No. (b-56): $^1$H-NMR (400 MHz, $CDCl_3$) δ ppm: 1.15 (t, 3H), 1.61 (s, 9H), 2.20 (s, 3H), 2.79 (s, 3H), 3.35 (q, 2H), 5.15 (d, 1H), 5.96 (d, 1H), 6.73 (d, 1H), 6.96 (m, 1H), 7.28 (m, 3H), 7.56 (d, 1H), 7.73 (m, 1H).

Compound No. (b-58): $^1$H-NMR (400 MHz, $CDCl_3$) δ ppm: 1.61 (s, 9H), 2.77 (s, 3H), 3.96 (m, 2H), 5.11 (d, 2H), 5.19 (d, 1H), 5.90 (m, 1H), 6.03 (d, 1H), 6.83 (m, 2H), 7.18 (t, 1H), 7.25 - 7.30 (m, 3H), 7.73 (m, 2H).

Compound No. (b-59): $^1$H-NMR (400 MHz, $CDCl_3$) δ ppm: 0.81 (t, 3H), 1.58 (m, 2H), 1.61 (s, 9H), 2.77 (s, 3H), 3.21 (m, 2H), 5.13 (d, 1H), 6.03 (d, 1H), 6.83 (m, 2H), 7.18 (t, 1H), 7.25 - 7.30 (m, 3H), 7.73 (m, 2H).

Compound No. (b-63): $^1$H-NMR (400 MHz, $CDCl_3$) δ ppm: 1.61 (s, 9H), 2.84 (s, 3H), 3.82 (s, 3H), 5.15 (d, 1H), 6.11 (d, 1H), 6.84 (d, 1H), 7.30 (m, 3H), 7.73 (m, 1H), 7.82 (dd, 1H), 8.59 (d, 1H).

Compound No. (b-67): $^1$H-NMR (400 MHz, $CDCl_3$) δ ppm: 1.67 (s, 9H), 1.73 (s, 3H), 2.96 (s, 3H), 4.97 (d, 1H), 6.18 (d, 1H), 6.91 (m, 2H), 7.30 (m, 4H), 7.69 (d, 1H), 7.81 (d, 1H).

Compound (b-70): $^1$H-NMR (400 MHz, $CDCl_3$) δ ppm: 0.94 (t, 3H), 1.17 (t, 3H), 1.56 (m, 6H), 2.05 (q, 1H), 2.21 (q, 1H), 2.78 (s, 3H), 3.31 (q, 2H), 5.05 (d, 1H), 6.08 (d, 1H), 6.83 (m, 2H), 7.15 (t, 1H), 7.25 (t, 3H), 7.75 (m, 2H).

Compound (b-76): $^1$H-NMR (400 MHz, $CDCl_3$) δ ppm: 2.75 (s, 3H), 5.17 (d, 1H), 5.85 (d, 1H), 6.85 (m, 1H), 6.92 (m, 1H), 7.21 - 7.32 (m, 8H), 7.61 (m, 1H), 7.66 (m, 1H).

Compound (b-77): $^1$H-NMR (400 MHz, $CDCl_3$) δppm: 2.74 (s, 3H), 5.15 (d, 1H), 5.86 (d, 1H), 6.83 (m, 1H), 6.91 (m, 1H), 7.15 - 7.20 (m, 4H), 7.27 - 7.35 (m, 4H), 7.60 (m, 1H), 7.64 (m, 1H).

Compound (b-83): $^1$H-NMR (400 MHz, $CDCl_3$) δ ppm: 1.16 (t, 3H), 1.54 (s, 9H), 2.81 (s, 3H), 3.32 (q, 2H), 5.25 (d, 1H), 5.60 (d, 1H), 6.37 - 6.40 (m, 2H), 7.25 (m, 1H), 7.29 - 7.30 (m, 2H), 7.86 (m, 1H).

Compound (b-95): $^1$H-NMR (400 MHz, $CDCl_3$) δ ppm: 1.15 (t, 3H), 1.60 (s, 9H), 2.79 (s, 3H), 3.33 (q, 2H), 5.33 (d, 1H), 6.05 (d, 1H), 6.75 (t, 1H), 7.25 - 7.28 (m, 3H), 7.46 (m, 1H), 7.70 - 7.75 (m, 2H).

Compound (b-97): $^1$H-NMR (400 MHz, $CDCl_3$) δ ppm: 1.16 (t, 3H), 1.61 (s, 9H), 2.19 (s, 3H), 2.78 (s, 3H), 3.33 (q, 2H), 5.16 (d, 1H), 5.97 (d, 1H), 6.79 (t, 1H), 7.07 (m, 1H), 7.20 - 7.26 (m, 3H), 7.59 (m, 1H), 7.75 (m, 1H).

Compound (b-107): $^1$H-NMR (400 MHz, $CDCl_3$) δ ppm: 1.16 (t, 3H), 1.60 (s, 9H), 2.63 (m, 2H), 2.74 (s, 3H), 2.89 (s, 3H), 5.10 (d, 1H), 5.94 (d, 1H), 6.64 (t, 1H), 7.09 (m, 1H), 7.16 - 7.26 (m, 3H), 7.59 (m, 1H), 7.76 (m, 1H).

Compound (b-109): $^1$H-NMR (400 MHz, $CDCl_3$) δ ppm: 1.15 - 1.17 (m, 6H), 1.61 (s, 9H), 2.53 (q, 2H), 2.78 (s, 3H), 3.34 (q, 2H), 5.08 (d, 1H), 6.01 (d, 1H), 6.66 (s, 1H), 6.73 (d, 1H), 7.24 - 7.26 (m, 3H), 7.65 (d, 1H), 7.74 (m, 1H).

Compound (b-110): $^1$H-NMR (400 MHz, $CDCl_3$) δ ppm: 1.58 (s, 9H), 2.81 (s, 3H), 3.72 (s, 3H), 5.01 (d, 1H), 6.03 (d, 1H), 6.37 (d, 1H), 6.45 (m, 1H), 7.25 - 7.28 (m, 3H), 7.68 - 7.70 (m, 2H).

Compound (b-111): $^1$H-NMR (400 MHz, $CDCl_3$) δ ppm: 1.60 (s, 9H), 2.74 (s, 3H), 2.90 (s, 3H), 3.72 (s, 3H), 5.02 (d, 1H), 6.04 (d, 1H), 6.36 (d, 1H), 6.44 (m, 1H), 7.25 - 7.28 (m, 3H), 7.66 (d, 1H), 7.73 (m, 1H).

Compound (b-112): $^1$H-NMR (400 MHz, $CDCl_3$) δ ppm: 1.16 (t, 3H), 1.61 (s, 9H), 2.77 (s, 3H), 3.34 (q, 2H), 3.72 (s, 3H), 5.04 (d, 1H), 6.04 (d, 1H), 6.36 (d, 1H), 6.46 (m, 1H), 7.25 - 7.28 (m, 3H), 7.67 (d, 1H), 7.73 (m, 1H).

Compound (b-138): $^1$H-NMR (400 MHz, $CDCl_3$) δ ppm: 1.34 (t, 3H), 1.59 (s, 9H), 2.24 (s, 3H), 2.63 (m, 2H), 2.74 (s, 3H), 5.11 (d, 1H), 5.98 (d, 1H), 6.64 (t, 1H), 6.79 (m, 1H), 7.16 - 7.26 (m, 3H), 7.57 (m, 1H), 7.76 (m, 1H).

Compound (b-139): $^1$H-NMR (400 MHz, $CDCl_3$) δ ppm: 1.21 (t, 3H), 1.61 (s, 9H), 1.89 (m, 2H), 2.21 (s, 3H), 2.73 (m, 2H), 2.71 (s, 3H), 5.15 (d, 1H), 5.81 (d, 1H), 6.61 (d, 1H), 6.78 (m, 1H), 7.11-7.31 (m, 3H), 7.61 (m, 1H), 7.88 (m, 1H).

Compound (b-141): $^1$H-NMR (400 MHz, $CDCl_3$) δ ppm: 0.91 (t, 3H), 1.16 (t, 3H), 1.53 (s, 6H), 2.03 (m, 1H), 2.16

(m, 1H), 2.79 (s, 3H), 3.31 (m, 2H), 5.24 (d, 1H), 6.03 (d, 1H), 6.77 (m, 1H), 7.01 (m, 1H), 7.25 - 7.29 (m, 3H), 7.50 (m, 1H), 7.72 (m, 1H).

Compound (b-143): $^1$H-NMR (400 MHz, $CDCl_3$) δ ppm: 0.92 (t, 3H), 1.16 (t, 3H), 1.53 (s, 6H), 2.18 (s, 3H), 2.78 (s, 3H), 3.31 (q, 2H), 5.11 (d, 1H), 6.02 (d, 1H), 6.79 (t, 1H), 7.05 (m, 1H), 7.20 - 7.26 (m, 3H), 7.58 (m, 1H), 7.72 (m, 1H).

Compound (b-149): $^1$H-NMR (400 MHz, $CDCl_3$) δ ppm: 1.07 (s, 9H), 1.18 (t, 3H), 1.65 (s, 3H), 1.68 (s, 3H), 2.12 (d, 1H), 2.30 (d, 1H), 2.78 (s, 3H), 3.34 (q, 2H), 5.09 (d, 1H), 6.07 (d, 1H), 6.80 - 6.86 (m, 2H), 7.16 (m, 1H), 7.25 - 7.28 (m, 3H), 7.76 - 7.79 (m, 2H).

Compound (b-161): $^1$H-NMR (400 MHz, $CDCl_3$) δ ppm: 1.60 (s, 9H), 2.77 (s, 3H), 2.91 (s, 3H), 5.09 (d, 1H), 6.98 (d, 1H), 6.75 (d, 1H), 7.10 (m, 1H), 7.24 - 7.28 (m, 3H), 7.72 - 7.75 (m, 2H).

Compound (b-163): $^1$H-NMR (400 MHz, $CDCl_3$) δ ppm: 0.94 (t, 3H), 1.18 (t, 3H), 1.56 (m, 6H), 2.15 (m, 2H), 2.80 (s, 3H), 3.34 (q, 2H), 5.06 (d, 1H), 6.03 (d, 1H), 6.75 (d, 1H), 7.10 (m, 1H), 7.25 - 7.30 (m, 3H), 7.69 - 7.72 (m, 2H).

Compound (b-165): $^1$H-NMR (400 MHz, $CDCl_3$) δ ppm: 84-0310 1.15 - 1.18 (m, 6H), 1.61 (s, 9H), 2.61 (m, 2H), 2.77 (s, 3H), 3.33 (q, 2H), 5.14 (d, 1H), 5.95 (d, 1H), 6.84 (m, 1H), 7.08 (m, 1H), 7.21 - 7.26 (m, 3H), 7.60 (m, 1H), 7.75 (m, 1H).

Compound (b-167): $^1$H-NMR (400 MHz, $CDCl_3$) δ ppm: 84-0312 0.93 (t, 3H), 1.15 - 1.18 (m, 6H), 1.53 (s, 6H), 2.08 (m, 1H), 2.10 (m, 1H), 2.62 (m, 2H), 2.77 (s, 3H), 3.32 (q, 2H), 5.09 (d, 1H), 5.98 (d, 1H), 6.83 (t, 1H), 7.08 (m, 1H), 7.21 - 7.26 (m, 3H), 7.60 (m, 1H), 7.73 (m, 1H).

Compound (b-174): $^1$H-NMR (400 MHz, $CDCl_3$) δ ppm: 0.99 (t, 3H), 1.56 (s, 6H), 2.18 (q, 2H), 2.27 (s, 3H), 2.80 (s, 3H), 2.93 (s, 3H), 5.07 (d, 1H), 6.09 (d, 1H), 6.70 (s, 1H), 6.73 (d, 1H), 7.29 (m, 3H), 7.67 (d, 1H), 7.77 (m, 1H).

Compound (b-175): $^1$H-NMR (400 MHz, $CDCl_3$) δ ppm: 1.00 (t, 3H), 1.41 (m, 2H), 1.55 (s, 6H), 2.09 (q, 2H), 2.24 (s, 3H), 2.77 (s, 3H), 2.90 (s, 3H), 5.06 (d, 1H), 6.05 (d, 1H), 6.67 (s, 1H), 6.71 (d, 1H), 7.27 (m, 3H), 7.64 (d, 1H), 7.73 (m, 1H).

Compound (b-176): $^1$H-NMR (400 MHz, $CDCl_3$) δ ppm: 0.87 (t, 3H), 1.16 (t, 3H), 1.54 (s, 6H), 2.13 (m, 2H), 2.22 (s, 3H), 2.78 (s, 3H), 3.32 (m, 2H), 5.04 (d, 1H), 6.04 (d, 1H), 6.64 (s, 1H), 6.69 (d, 1H), 7.25 (m, 3H), 7.61 (d, 1H), 7.70 (m, 1H).

Compound (b-178): $^1$H-NMR (400 MHz, $CDCl_3$) δ ppm: 0.94 (t, 3H), 1.53 (s, 6H), 2.09 - 2.14 (m, 2H), 2.76 (s, 3H), 2.89 (s, 3H), 5.06 (d, 1H), 6.05 (d, 1H), 7.81 - 7.83 (m, 2H), 7.26 (m, 3H), 7.67 - 7.70 (m, 2H).

Compound (b-179): $^1$H-NMR (400 MHz, $CDCl_3$) δ ppm: 0.97 (t, 3H), 1.52 (s, 6H), 2.03 - 2.06 (m, 2H), 2.75 (s, 3H), 2.88 (s, 3H), 5.04 (d, 1H), 6.03 (d, 1H), 6.82 (m, 2H), 7.24 - 7.27 (m, 3H), 7.66 - 7.71 (m, 2H).

Compound (b-180): $^1$H-NMR (400 MHz, $CDCl_3$) δ ppm: 0.91 (t, 3H), 1.16 (t, 3H), 1.53 (s, 3H), 1.55 (s, 3H), 2.00 - 2.05 (m, 1H), 2.17 - 2.21 (m, 2H), 2.78 (s, 3H), 3.32 (q, 2H), 5.09 (d, 1H), 6.05 (d, 1H), 6.82 - 6.84 (m, 2H), 7.27 (m, 3H), 7.66 - 7.71 (m, 2H).

Compound (b-194): $^1$H-NMR (400 MHz, $CDCl_3$) δ ppm: 1.15 (t, 3H), 1.60 (s, 9H), 2.53 (q, 2H), 2.75 (s, 3H), 2.89 (s, 3H), 5.04 (d, 1H), 6.02 (d, 1H), 6.66 (s, 1H), 6.73 (d, 1H), 7.25 (m, 3H), 7.65 (d, 1H), 7.73 (m, 1H).

In addition, among the oxadiazoline compounds according to the present invention, some of the compounds represented by formula (II-b) are shown in Table 2, together with the physical properties (melting point, refractive index, or characteristic state).

In Table 2, Me represents a methyl group, Et represents an ethyl group, t-Bu represents a t-butyl group, Bn represents a benzyl group, and Ph represents a phenyl group.

[Chem. 61]

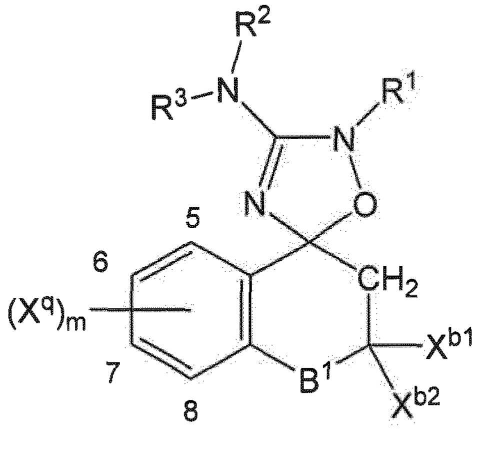

(II-b)

[Table 8]

| Table 2 | | | | | | | | |
|---|---|---|---|---|---|---|---|---|
| Compound No. | $R^1$ | $R^2$ | $R^3$ | $(X^q)m$ | $B^1$ | $X^{b1}$ | $X^{b2}$ | Physical property |
| c-1 | Me | t-Bu | H | 7-MeO | $CH_2$ | H | H | m.p. 120-122°C |
| c-2 | Me | t-Bu | H | - | O | Me | Me | m.p. 79-82°C |
| c-3 | Me | t-Bu | H | - | O | H | H | m.p. 90-92°C |
| c-4 | Me | t-Bu | H | - | O | $-CH_2CH_2CH_2CH_2-$ | $CH_2-$ | m.p. 102-104°C |
| c-5 | Me | t-Bu | H | 6-Cl | O | Me | Me | nD(23.3) 1.5269 |
| c-6 | Me | t-Bu | Et | 6-Cl | O | Me | Me | nD(22.6) 1.5229 |
| c-7 | Me | t-Bu | H | 6-Br | O | Me | Me | nD(22.8) 1.536 |
| c-8 | Me | t-Bu | H | 6-Cl,7-Me | O | Me | Me | nD(22.2) 1.5299 |
| c-9 | Me | t-Bu | H | 6-F | O | Me | Me | m.p. 86-88°C |
| c-10 | Me | t-Bu | H | 6-F | O | H | H | m.p. 85-86°C |
| c-11 | Me | t-Bu | H | 7-MeO | O | Me | Me | m.p. 95-97°C |
| c-12 | Me | t-Bu | H | - | O | Ph | H | m.p. 139-141°C |
| c-13 | Me | t-Bu | H | 6-Me | O | Me | Me | m.p. 104-107°C |
| c-14 | Me | t-Bu | H | - | O | 4-ClPh | H | m.p. 155-156°C |
| c-15 | Me | t-Bu | H | - | O | 4-FPh | H | m.p. 143-145°C |
| c-16 | Me | t-Bu | H | - | O | 4-MePh | H | m.p. 135-137°C |

[Table 9]

| Table 2 (continued) | | | | | | | | |
|---|---|---|---|---|---|---|---|---|
| Compound No. | $R^1$ | $R^2$ | $R^3$ | $(X^q)m$ | $B^1$ | $X^{b1}$ | $X^{b2}$ | Physical property |
| c-17 | Me | t-Bu | H | 6-Cl | O | 4-ClPh | H | m.p. 199-201°C |
| c-18 | Me | t-Bu | H | 6-F | $SO_2$ | H | H | m.p. 171-173°C |
| c-19 | Me | t-Bu | H | $6-CF_3CH_2CH_2CH_2O$ | O | Me | Me | m.p. 111-113°C |
| c-20 | Me | t-Bu | Et | $6-CF_3CH_2CH_2CH_2O$ | O | Me | Me | m.p. 75-77°C |
| c-21 | Me | t-Bu | allyl | $6-CF_3CH_2CH_2CH_2O$ | O | Me | Me | m.p. 60-62°C |
| c-22 | Me | t-Bu | H | 6-MeO | O | Me | Me | m.p. 90-91°C |
| c-23 | Me | t-Bu | Et | 6-MeO | O | Me | Me | amorphous |
| c-24 | Me | t-Bu | H | 7-Cl | O | Me | Me | m.p. 111-113°C |
| c-25 | Me | t-Bu | Et | 7-Cl | O | Me | Me | m.p. 114-116°C |
| c-26 | Me | t-Bu | Bn | 7-Cl | O | Me | Me | viscous oil |
| c-27 | Me | t-Bu | H | 7-BnO | O | Me | Me | m.p. 120-122°C |
| c-28 | Me | t-Bu | H | $6,7-(MeO)_2$ | O | Me | Me | m.p. 134-136°C |
| c-29 | Me | t-Bu | H | 8-Cl | O | Me | Me | m.p. 152-153°C |
| c-30 | Me | t-Bu | Et | 8-C1 | O | Me | Me | m.p. 107-109°C |
| c-31 | Me | t-Bu | H | - | S | Ph | H | m.p. 171-173°C |
| c-32 | Me | t-Bu | Et | - | O | 4-FPh | H | nD(21.1) 1.5964 |

(continued)

| Table 2 (continued) | | | | | | | | |
|---|---|---|---|---|---|---|---|---|
| Compound No. | $R^1$ | $R^2$ | $R^3$ | $(X^q)m$ | $B^1$ | $X^{b1}$ | $X^{b2}$ | Physical property |
| c-33 | Me | t-Bu | Et | - | O | $-CH_2CH_2CH_2CH_2-$ | | nD(21.1) 1.5794 |
| c-34 | Me | t-Bu | Me | - | S | Ph | H | amorphous |

Among the compounds described above, with respect to the compound in the form of an amorphous or a viscous oil, the NMR data thereof are shown below.

Compound No. (c-23): $^1$H-NMR (400 MHz, $CDCl_3$) δ ppm: 1.20 (t, 3H), 1.41 (m, 15H), 2.15 (d, 1H), 2.33 (d, 1H), 3.01 (s, 3H), 3.31 (q, 2H), 3.71 (s, 3H), 6.71 (d, 1H), 6.82 (m, 2H).

Compound No. (c-26): $^1$H-NMR (400 MHz, $CDCl_3$) δ ppm: 1.39 (m, 15H), 2.11 (d, 1H), 2.23 (d, 1H), 2.91 (s, 3H), 4.45 (q, 2H), 6.76 (d, 1H), 6.81 (d, 1H), 7.06 (d, 1H), 7.36 (m, 5H).

Compound (c-34): $^1$H-NMR (400 MHz, $CDCl_3$) δ ppm: 1.45 (s, 9H), 2.26 (m, 1H), 2.73 (m, 1H), 2.86 (s, 3H), 2.96 (s, 3H), 4.81 (m, 1H), 7.07 - 7.15 (m, 3H), 7.25 - 7.50 (m, 5H).

In addition, among the oxadiazoline compounds according to the present invention, some compounds represented by formula (II-c) are shown in Table 3, together with (melting point or characteristic).

In Table 3, Me represents a methyl group, Et represents an ethyl group, and t-Bu represents a t-butyl group.

[Chem. 62]

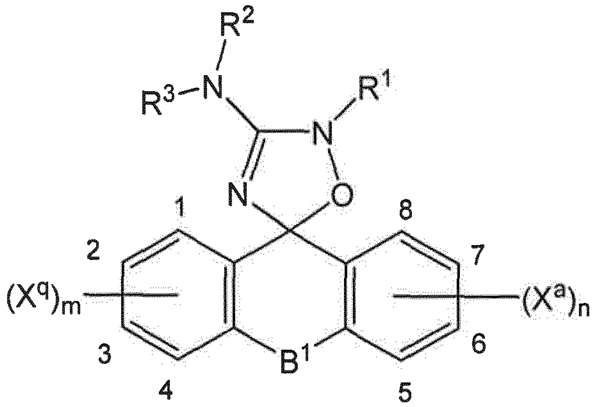

(II-c)

[Table 10]

| Table 3 | | | | | | | |
|---|---|---|---|---|---|---|---|
| Compound No. | $R^1$ | $R^2$ | $R^3$ | $(X^q)m$ | $(X^a)n$ | $B^1$ | Physical property |
| d-1 | Me | t-Bu | Me | - | - | S | m.p. 210-212°C |
| d-2 | Me | t-Bu | Me | - | - | S | m.p. 149-151°C |
| d-3 | Me | t-Bu | Et | - | - | S | amorphous |
| d-4 | Me | t-Bu | H | 2-Cl | - | S | m.p. 167-170°C |
| d-5 | Me | t-Bu | H | $2-CF_3$ | - | S | m.p. 151-153°C |
| d-6 | Me | t-Bu | H | $2,4-Et_2$ | - | S | amorphous |
| d-7 | Me | t-Bu | Et | 2-Cl | - | S | m.p. 110-113°C |

(continued)

| Table 3 | | | | | | | |
|---|---|---|---|---|---|---|---|
| Compound No. | $R^1$ | $R^2$ | $R^3$ | $(X^q)m$ | $(X^a)n$ | $B^1$ | Physical property |
| d-8 | Me | t-Bu | Et | 2-$CF_3$ | - | S | m.p. 98-101°C |
| d-9 | Me | t-Bu | Et | 2,4-$Et_2$ | - | S | amorphous |
| d-10 | Me | t-Bu | H | - | - | $C(Me)_2$ | m.p. 164-165°C |
| d-11 | Me | t-Bu | H | 4-F | - | S | m.p.212-214°C |
| d-12 | Me | t-Bu | Me | 4-F | - | S | m.p. 132-135°C |
| d-13 | Me | t-Bu | Et | 4-F | - | S | m.p. 106-108°C |

Among the compounds described above, with respect to the compounds in the form of an amorphous, the NMR data thereof are shown below.

Compound No. (d-3): $^1$H-NMR (400 MHz, $CDCl_3$) $\delta$ ppm: 0.87 (t, 3H), 1.69 (s, 9H), 2.74 (s, 2H), 3.38 (q, 2H), 7.24 - 7.27 (m, 4H), 7.46 - 7.49 (m, 2H), 7.79 - 7.81 (m, 2H).

Compound No. (d-6): $^1$H-NMR (400 MHz, $CDCl_3$) $\delta$ ppm: 1.23 (t, 3H), 1.30 (t, 3H), 1.67 (s, 9H), 2.65 (q, 2H), 2.76 (s, 3H), 2.83 (q, 2H), 3.90 (s, 1H), 7.04 (s, 1H), 7.26 - 7.28 (m, 2H), 7.52 (m, 1H), 7.57 (, 1H), 7.79 (m, 1H).

Compound (d-9): $^1$H-NMR (400 MHz, $CDCl_3$) $\delta$ ppm: 1.18 - 1.25 (m, 6H), 1.29 (t, 3H), 1.70 (s, 9H), 2.63 (q, 2H), 2.72 (s, 3H), 2.83 (q, 2H), 3.38 (q, 2H), 7.01 (s, 1H), 7.23 - 7.25 (m, 2H), 7.49 (m, 1H), 7.57 (s, 1H), 7.77 (m, 1H).

In addition, among the oxadiazoline compounds according to the present invention, some compounds represented by formula (1-2) are shown in Table 4, together with the physical properties (melting point or characteristic).

In Table 4, Me represents a methyl group, Et represents an ethyl group, n-Pr represents an n-propyl group, and t-Bu represents a t-butyl group.

[Chem. 63]

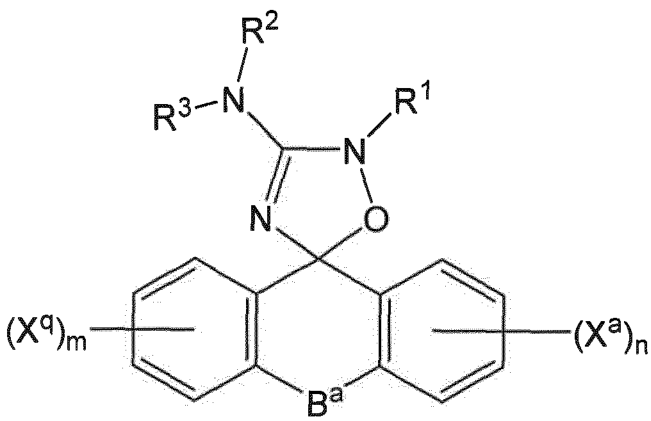

[Table 11]

| Table 4 | | | | | | | |
|---|---|---|---|---|---|---|---|
| Compound No. | $R^1$ | $R^2$ | $R^3$ | $(X^q)m$ | $(X^a)n$ | $B^a$ | Physical property |
| e-1 | Me | t-Bu | H | 4-F | - | CH=CH | m.p. 144-146°C |
| e-2 | Me | t-Bu | Me | 4-F | - | CH=CH | m.p. 143-146°C |
| e-3 | Me | t-Bu | Et | 4-F | - | CH=CH | m.p. 140-142°C |
| e-4 | Me | t-Bu | H | 4-F | - | $CH_2CH_2$ | m.p. 129-131°C |

(continued)

| Table 4 | | | | | | | |
|---|---|---|---|---|---|---|---|
| Compound No. | $R^1$ | $R^2$ | $R^3$ | $(X^q)m$ | $(X^a)n$ | $B^a$ | Physical property |
| e-5 | Me | t-Bu | Me | 4-F | - | $CH_2CH_2$ | m.p. 95-97°C |
| e-6 | Me | t-Bu | Et | 4-F | - | $CH_2CH_2$ | amorphous |
| e-7 | Me | t-Bu | H | - | - | CBr=CH | m.p. 96-98°C |
| e-8 | Me | t-Bu | Me | - | - | CBr=CH | m.p. 172-174°C |
| e-9 | Me | t-Bu | Et | - | - | CBr=CH | m.p. 70-72°C |
| e-10 | Me | t-Bu | H | - | - | C(Me)=CH | m.p. 81-83°C |
| e-11 | Me | t-Bu | Me | - | - | C(Me)=CH | m.p. 168-170°C |
| e-12 | Me | t-Bu | Et | - | - | C(Me)=CH | nD(20.7) 1.6132 |
| e-13 | Me | t-Bu | H | - | - | C(CN)=CH | m.p. 170-172°C |
| e-14 | Me | $C(Me)_2Et$ | H | - | - | $CH_2CH_2$ | m.p. 110-112°C |
| e-15 | Me | $C(Me)_2Et$ | Me | - | - | $CH_2CH_2$ | m.p. 116-118°C |
| e-16 | Me | $C(Me)_2Et$ | Et | - | - | $CH_2CH_2$ | amorphous |
| e-17 | Me | $C(Et)_2H$ | H | - | - | $CH_2CH_2$ | m.p. 96-98°C |
| e-18 | Me | $C(Et)_2H$ | Me | - | - | $CH_2CH_2$ | nD(21.3) 1.6012 |
| e-19 | Me | $C(Et)_2H$ | Et | - | - | $CH_2CH_2$ | m.p. 94-96°C |
| e-20 | Me | $C(Me)_2CH_2$(t-Bu) | H | - | - | $CH_2CH_2$ | m.p. 120-122°C |

[Table 12]

| Table 4 (continued) | | | | | | | |
|---|---|---|---|---|---|---|---|
| Compound No. | $R^1$ | $R^2$ | $R^3$ | $(X^q)m$ | $(X^a)n$ | $B^a$ | Physical property |
| e-21 | Me | $C(Me)_2Ph$ | H | - | - | $CH_2CH_2$ | m.p. 187-188°C |
| e-22 | Et | t-Bu | H | - | - | $CH_2CH_2$ | m.p. 148-150°C |
| e-23 | Et | t-Bu | Me | - | - | $CH_2CH_2$ | m.p. 109-111 °C |
| e-24 | Et | t-Bu | Et | - | - | $CH_2CH_2$ | m.p.58-60°C |
| e-25 | Et | t-Bu | n-Pr | - | - | $CH_2CH_2$ | nD(21.2) 1.6211 |
| e-26 | Me | $C(Et)_2Me$ | H | - | - | $CH_2CH_2$ | m.p. 54-56°C |
| e-27 | Me | $C(Et)_2Me$ | Me | - | - | $CH_2CH_2$ | nD(21.9) 1.6139 |
| e-28 | Me | $C(Et)_2Me$ | Et | - | - | $CH_2CH_2$ | nD(21.6) 1.6211 |
| e-29 | Me | $C(Me)_2Ph$ | Me | - | - | $CH_2CH_2$ | m.p. 164-166°C |
| e-30 | Me | $C(Me)_2Ph$ | Et | - | - | $CH_2CH_2$ | m.p. 131-132°C |
| e-31 | Me | $C(Me)_2Ph$ | n-Pr | - | - | $CH_2CH_2$ | m.p. 110-112°C |
| e-32 | Me | $C(Me)_2CH_2$(t-Bu) | Me | - | - | $CH_2CH_2$ | amorphous |
| e-33 | Me | $C(Me)_2CH_2$(t-Bu) | Et | - | - | $CH_2CH_2$ | amorphous |
| e-34 | Me | $C(Me)_2$(n-Pr) | H | - | - | $CH_2CH_2$ | m.p. 100-102°C |
| e-35 | Me | $C(Me)_2$(n-Pr) | Me | - | - | $CH_2CH_2$ | m.p. 90-92°C |
| e-36 | Me | $C(Me)_2$(n-Pr) | Et | - | - | $CH_2CH_2$ | nD(22) 1.5937 |

(continued)

| Table 4 (continued) | | | | | | | |
|---|---|---|---|---|---|---|---|
| Compound No. | $R^1$ | $R^2$ | $R^3$ | $(X^q)m$ | $(X^a)n$ | $B^a$ | Physical property |
| e-37 | Me | $C(Me)_2(n-Pr)$ | n-Pr | - | - | $CH_2CH_2$ | nD(22) 1.6029 |
| e-38 | Me | Adamantan-1-yl | H | - | - | $CH_2CH_2$ | m.p. 162-164°C |
| e-39 | Me | $C(Me)_2CH_2OMe$ | H | - | - | $CH_2CH_2$ | m.p. 124-126°C |
| e-40 | Me | $C(Me)_2CH_2OMe$ | Me | - | - | $CH_2CH_2$ | m.p. 83-85°C |
| e-41 | Me | $C(Me)_2CH_2OMe$ | n-Pr | - | - | $CH_2CH_2$ | m.p. 52-54°C |
| e-42 | Me | $C(Me)_2C(=O)O(t-Bu)$ | H | - | - | $CH_2CH_2$ | m.p. 132-134°C |
| e-43 | Me | $C(Me)_2CH_2OSiPh_2(t-Bu)$ | H | - | - | $CH_2CH_2$ | nD(22.2) 1.6311 |
| e-44 | Me | $C(Me)_2C(=O)O(t-Bu)$ | Me | - | - | $CH_2CH_2$ | nD(21.9) 1.5931 |
| e-45 | Me | Adamantan-1-yl | Me | - | - | $CH_2CH_2$ | m.p.85-87°C |
| e-46 | Me | Adamantan-1-yl | Et | - | - | $CH_2CH_2$ | nD(22) 1.5877 |
| e-47 | Me | 1-Me-c-Pr | H | - | - | $CH_2CH_2$ | m.p. 102-104°C |
| e-48 | Me | 1-Me-c-Pr | Me | - | - | $CH_2CH_2$ | m.p. 115-117°C |

Among the compounds described above, with respect to the compounds in the form of an amorphous, the NMR data thereof are shown below.

Compound No. (e-6): $^1H$-NMR (400 MHz, $CDCl_3$) $\delta$ ppm: 1.13 (t, 3H), 1.57 (s, 9H), 2.76 (s, 3H), 3.13 (m, 2H), 3.32 (q, 2H), 3.40 (m, 1H), 3.86 (m, 1H), 6.90 (m, 1H), 7.10 - 7.17 (m, 4H), 7.64 (d, 1H), 7.76 (d, 1H).
Compound No. (e-16): $^1H$-NMR (400 MHz, $CDCl_3$) $\delta$ ppm: 0.87 (t, 3H), 1.14 (t, 3H), 1.52 (s, 6H), 2.12 (q, 2H), 2.74 (s, 3H), 3.05 (m, 2H), 3.29 (q, 2H), 3.75 (m, 2H), 7.08-7.16 (m, 6H), 7.76-7.78 (d, 2H).
Compound No. (e-32): $^1H$-NMR (400 MHz, $CDCl_3$) $\delta$ ppm: 1.05 (s, 9H), 1.62 (s, 6H), 2.20 (s, 2H), 2.71 (s, 3H), 2.89 (s, 3H), 3.08 (m, 2H), 3.72 (m, 2H), 7.08-7.12 (m, 6H), 7.82 (m, 2H).
Compound No. (e-33):$^1H$-NMR(400MHz, $CDCl_3$) $\delta$ ppm: 1.04 (s, 9H), 1.14 (t, 3H), 1.63 (s, 6H), 2.19 (s, 2H), 2.74 (s, 3H), 3.05 (m, 2H), 3.31 (q, 2H), 3.74 (m, 2H), 7.07 - 7.14 (m, 6H), 7.83 (m, 2H).

In addition, Examples of other compounds are described.

[Reference Example 7]

Synthesis of 1-(tert-butyl)-3-(5,6,7,12-tetrahydrodibenzo[a,d][8]annulen-12-yl)thiourea

[Chem. 64]

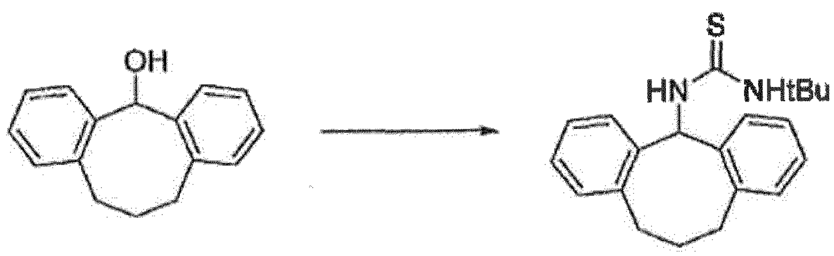

5,6,7,12-Tetrahydrodibenzo[a,d][8]annulen-12-ol was synthesized by the method described in J. Med. Chem. 1992, 35, 2481.
5,6,7,12-Tetrahydrodibenzo[a,d][8]annulen-12-ol (0.48 g) was dissolved in dichloromethane (7 ml). Subsequently, 1-(t-butyl)thiourea (0.32 g) and aluminum trifluoromethane sulfonate (0.10 g) were added thereto. The mixture was stirred for 2 hours, and subsequently, the reaction solvent was distilled off. The residue was purified by column chromatography with silica gel. Thereby, 0.47 g of the objective product was obtained.
$^1$H-NMR (400 MHz, $CDCl_3$) δ ppm: 1.39 (s, 9H), 2.38 (m, 2H), 2.97 (m, 2H), 3.35 (m, 2H), 5.74 (m, 1H), 6.42 (m, 1H), 6.68 (m, 1H), 7.05 (m, 4H), 7.33 (t, 2H), 7.42 (d, 2H).

[Reference Example 8]

Synthesis of N-tert-butyl-N-(5,6,7,12-tetrahydrodibenzo[a,d][8]annulen-12-yl)methanediimine

[Chem. 65]

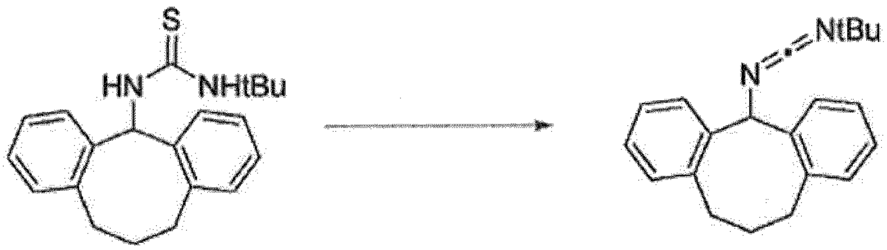

Triethylamine (0.58 ml) and 2-chloro-1-methylpyridinium iodide (0.54 g) were added to a solution of the thiourea (0.55 g) obtained in Reference Example 7 dissolved in acetonitrile (5 ml), and the mixture was stirred for 4 hours at room temperature. The insoluble material was filtered, and the filtrate was concentrated under reduced pressure. The residue was purified by column chromatography with silica gel. Thereby, 0.45 g of the objective product was obtained.
$^1$H-NMR (400 MHz, $CDCl_3$) δ ppm: 1.29 (s, 9H), 2.32 (m, 2H), 2.97 (m, 2H), 3.25 (m, 2H), 6.38 (m, 1H), 7.05 (m, 4H), 7.13 (t, 2H), 7.63 (d, 2H).

[Example 6]

Synthesis of N-(tert-butyl)-2'-methyl-6,7-dihydro-2'H,5H-spiro[dibenzo[a,d][8] annulene-12,5'-[1,2,4]oxadiazol]-3'-amine (Compound No. A-1)

[Chem. 66]

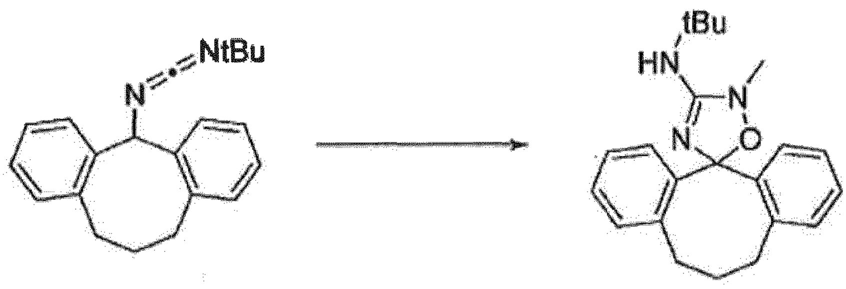

N-methylhydroxyamine hydrochloride (0.15 g) and trimethylamine (0.25 ml) were added to a solution of the carbodimide (0.45 g) obtained in Reference Example 8 dissolved in acetonitrile (8 ml), and the mixture was stirred for 2 hours at room temperature. The reaction solution was concentrated under reduced pressure. Subsequently, dichloromethane (8 ml) and manganese dioxide (0.13 g) were added thereto. The reaction solution was filtered using celite, and the insoluble material was washed with ethyl acetate. The filtrate was concentrated under reduced pressure. The crude product was washed with hexane. Thereby, 0.5 g of the objective product was obtained. The physical properties (melting point and NMR) thereof are shown below.

Melting point: 153 - 155°C
$^1$H-NMR (400 MHz, $CDCl_3$) δ ppm: 1.42 (s, 9H), 2.58 (m, 4H), 2.76 (s, 3H), 2.95 (m, 2H), 3.64 (m, 1H), 7.05 (m, 4H), 7.23 (m, 2H), 7.62 (m, 2H).

[Example 7]

Synthesis of N-(tert-butyl)-N,2'-dimethyl-6,7-dihydro-2'H,5H-spiro[dibenzo [a,d][8]annulene-12,5'-[1,2,4]oxadiazol]-3'-amine (Compound No. A-2)

[Chem. 67]

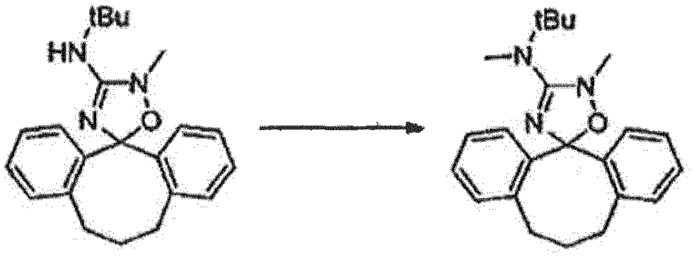

Sodium hydride (0.018 g) was added to a solution of the amine (0.1 g) obtained in Example 6 dissolved in DMF (1 ml) at 0°C, and the mixture was stirred for 30 minutes. Methyl iodide (0.04 mL) was added thereto, and the mixture was stirred for one hour at 0°C. The reaction solution was added to water with ice, and subjected to extraction with ethyl acetate. The organic layer was washed with a saturated aqueous solution of sodium chloride, dried over anhydrous magnesium sulfate, and filtered. Subsequently, the filtrate was concentrated under reduced pressure. The obtained crude product was purified by column chromatography with silica gel. Thereby, 0.075 g of the objective product was obtained. The physical properties (melting point and NMR) are shown below.
Melting point: 111 - 113°C
$^1$H-NMR (400 MHz, $CDCl_3$) δ ppm: 1.49 (s, 9H), 2.51 - 2.98 (m, 12H), 6.91 - 7.23 (m, 5H), 7.61 - 7.88 (m, 3H).

The oxadiazoline compounds according to the present invention (Compound No. A-3 to Compound No. A-16) produced by the same methods as those described in the Examples mentioned above are shown below together with the physical properties (refractive index or melting point).

[Chem. 68]

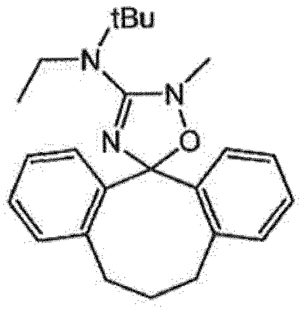

(A-3)

$n_D^{21.2°C}$=1.6214

[Chem. 69]

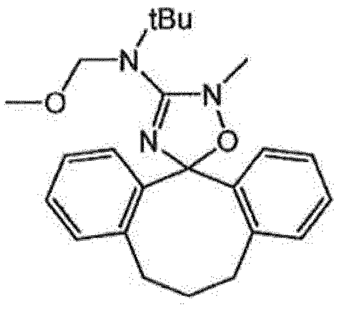

(A-4)

$n_D^{21.2°C}$=1.6311

[Chem. 70]

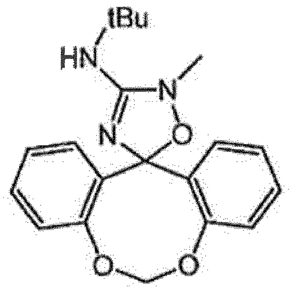

(A-5)

m.p. 165 - 167°C

[Chem. 71]

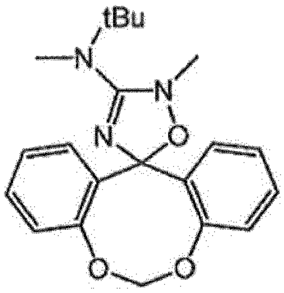

(A-6)

m.p. 162 - 164°C

[Chem. 72]

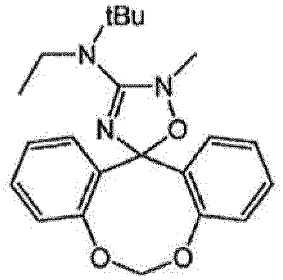

(A-7)

m.p. 76 - 78°C

[Chem. 73]

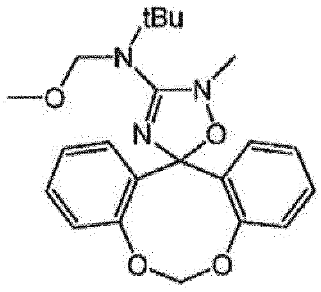

(A-8)

$n_D^{21.7°C}$=1.6398

[Chem. 74]

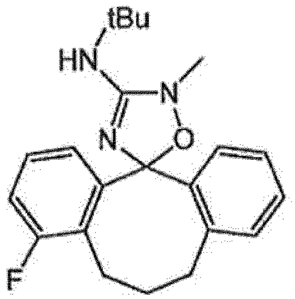

(A-9)

m.p. 113 - 115°C

[Chem. 75]

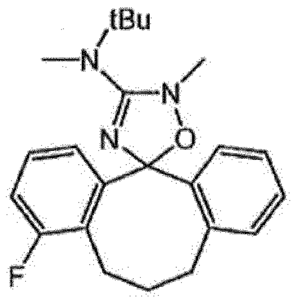

(A-10)

m.p. 129 - 131°C

[Chem. 76]

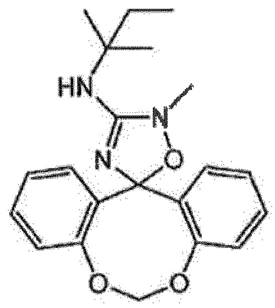

(A-11)

m.p. 138 - 140°C

[Chem. 77]

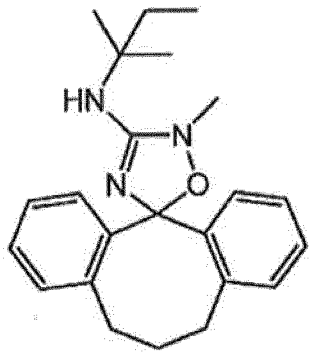

(A-12)

m.p. 134-136°C

[Chem. 78]

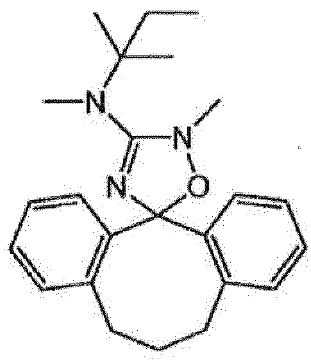

(A-13)

$n_D^{21.9°C}$ 1.6097

[Chem. 79]

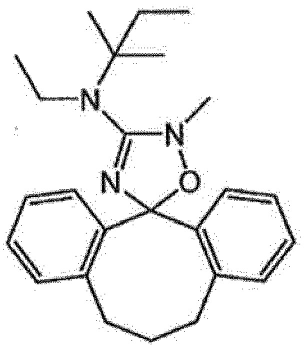

(A-14)

$n_D^{21.9°C}$ 1.613

[Chem. 80]

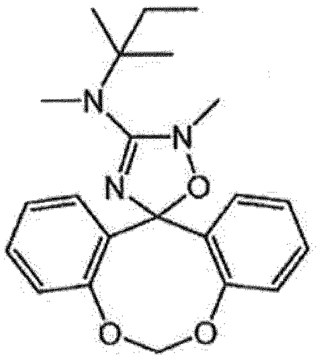

(A-15)

m.p. 98 - 100°C

[Chem. 81]

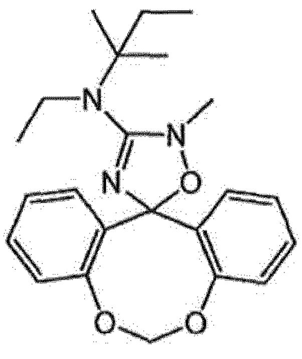

(A-16)

$n_D^{22°C}$ 1.6097

[Biological tests]

The following Test Examples demonstrate that the oxadiazoline compounds of the present invention (hereinafter, referred to as "the compounds of the present invention") are useful as active ingredients of the formulations for controlling harmful organisms. The term "part" is based on weight.

(Preparation of emulsion for test)

5 parts of the compound of the present invention, 93.6 parts of dimethylformamide and 1.4 parts of polyoxyethylene alkyl aryl ether were mixed and dissolved to prepare Emulsion (I) including 5% of an active ingredient.
For the control, Emulsion (II) was prepared by mixing and dissolving 93.6 parts of dimethylformamide and 1.4 parts of polyoxyethylene alkyl aryl ether.
An insect mortality rate was calculated by the numerical equation shown below.

$$\text{Insect mortality rate (\%)} = \{(\text{Number of dead insects}) / (\text{Number of sample insects})\} \times 100$$

(Test Example 1) Efficacy Test against *Mythimna separata*

Emulsion (I) was diluted with water so that the concentration of the compound of the present invention was 125 ppm. Corn leaves were soaked in the diluted liquid for 30 seconds. Subsequently, the corn leaves were put on Petri dishes, followed by inoculating 5 second-instar larvae of *Mythimna separata.* The Petri dishes were placed in a thermostatic chamber at a temperature of 25°C and humidity of 60%. Mortality was investigated 6 days after inoculation, and the insect mortality rate was calculated.
The efficacy test against *Mythimna separata* was carried out for the compounds shown in Table 13. All of the compounds shown in Table 13 demonstrated an 80% or more insect mortality rate against *Mythimna separata.*

[Table 13]

Table 13

| Compound No. | Compound No. | Compound No. | Compound No. | Compound No. | Compound No. |
|---|---|---|---|---|---|
| a-6 | b-1 | b-10 | b-84 | c-6 | c-16 |
| a-8 | b-2 | b-11 | b-85 | c-7 | c-17 |
| a-9 | b-3 | b-12 | b-86 | c-8 | c-19 |
| a-10 | b-4 | b-13 | a-25 | c-9 | c-20 |
| a-11 | b-5 | b-14 | a-29 | c-12 | c-24 |
| a-18 | b-6 | b-15 | a-30 | c-13 | c-25 |
| a-19 | b-7 | b-16 | c-2 | c-14 | c-29 |
| a-20 | b-9 | b-81 | c-5 | c-15 | c-30 |

(Test Example 2) Efficacy test against *Aphis gossypii*

Cucumber seedlings were inoculated with nymphs of *Aphis gossypii.* Emulsion (I) was diluted with water so that the concentration of the compound of the present invention was 125 ppm. Subsequently, the aforementioned diluted liquid was sprayed on the cucumber plants on which the nymphs of *Aphis gossypii* were parasitic. The aforementioned cucumber plants were then placed in a thermostatic chamber with a temperature of 25°C and humidity of 60%. Mortality was investigated 6 days after spraying was carried out, and the insect mortality rate of *Aphis gossypii* was calculated.
The efficacy test against *Aphis gossypii* was carried out for the compounds shown in Table 14. All of the compounds shown in Table 14 demonstrated an 80% or more insect mortality rate against *Aphis gossypii.*

[Table 14]

Table 14

| Compound No. | Compound No. | Compound No. | Compound No. | Compound No. |
|---|---|---|---|---|
| a-5 | b-3 | a-24 | c-8 | A-1 |
| a-8 | b-4 | a-25 | c-9 | A-2 |
| a-10 | b-10 | a-26 | c-10 | A-3 |
| a-12 | b-16 | c-2 | c-12 | |
| a-13 | b-54 | c-3 | c-24 | |
| a-20 | b-81 | c-5 | c-25 | |
| a-21 | b-100 | c-6 | c-29 | |
| b-1 | b-102 | c-7 | c-30 | |
| b-2 | b-104 | | | |

(Test Example 3) Efficacy test against *Thrips palmi* (*Frankliniella occidentalis*)

Cucumber seedlings were inoculated with 8 adults of *Thrips palmi.* Emulsion (I) was diluted with water so that the concentration of the compound of the present invention was 125 ppm. Subsequently, the aforementioned diluted liquid was sprayed on the cucumber seedlings, and then air dried. The number of the parasitic larvae was counted 7 days after spraying. The efficacy of the compounds was evaluated by the controlling rate described below.

$$\text{Controlling rate (\%)} = \{1 - (\text{Nt})/(\text{Nc})\} \times 100$$

In which Nt: number of parasites at the legion spray-treated.
Nc: number of parasites at the legion non-treated (control).

The efficacy test against *Thrips palmi* was carried out for the compounds shown in Table 15-1 and Table 15-2. All of the compounds shown in Table 15-1 and Table 15-2 demonstrated an 80% or more controlling rate against *Thrips palmi.*

[Table 15-1]

Table 15-1

| Compound No. | Compound No. | Compound No. | Compound No. | Compound No. | Compound No. | Compound No. | Compound No. |
|---|---|---|---|---|---|---|---|
| a-1 | a-16 | b-11 | b-33 | b-56 | b-80 | b-99 | a-38 |
| a-2 | a-17 | b-12 | b-34 | b-57 | b-81 | b-100 | c-5 |
| a-3 | a-22 | b-13 | b-35 | b-59 | b-82 | b-101 | c-12 |
| a-4 | a-23 | b-14 | b-39 | b-61 | b-83 | b-102 | c-24 |
| a-5 | b-1 | b-15 | b-45 | b-63 | b-84 | b-103 | c-31 |
| a-6 | b-2 | b-16 | b-46 | b-68 | b-85 | b-104 | d-1 |
| a-8 | b-3 | b-17 | b-47 | b-69 | b-86 | b-105 | d-2 |
| a-9 | b-4 | b-21 | b-48 | b-70 | b-92 | b-106 | d-3 |
| a-10 | b-5 | b-22 | b-49 | b-72 | b-93 | b-107 | d-4 |
| a-11 | b-6 | b-24 | b-50 | b-73 | b-94 | a-25 | d-5 |
| a-12 | b-7 | b-29 | b-51 | b-74 | b-95 | a-31 | d-6 |
| a-13 | b-8 | b-30 | b-52 | b-75 | b-96 | a-35 | d-7 |
| a-14 | b-9 | b-31 | b-53 | b-78 | b-97 | $\alpha$-36 | d-8 |
| a-15 | b-10 | b-32 | b-55 | b-79 | b-98 | a-37 | d-9 |

[Table 15-2]

Table 15-2

| Compound No. | | | | | | | |
|---|---|---|---|---|---|---|---|
| b-108 | b-127 | b-147 | b-165 | b-182 | e-1 | e-26 | A-1 |
| b-109 | b-128 | b-148 | b-166 | b-183 | e-2 | e-27 | A-2 |
| b-110 | b-129 | b-149 | b-167 | b-184 | e-3 | e-28 | A-3 |
| b-111 | b-130 | b-151 | b-168 | b-185 | e-4 | e-29 | A-4 |
| b-112 | b-135 | b-152 | b-170 | b-187 | e-5 | e-32 | A-5 |
| b-113 | b-136 | b-153 | b-171 | b-191 | e-6 | e-33 | A-6 |
| b-117 | b-137 | b-154 | b-172 | b-192 | e-11 | e-34 | A-7 |
| b-118 | b-138 | b-155 | b-173 | b-193 | e-14 | e-35 | A-8 |
| b-119 | b-139 | b-156 | b-174 | b-194 | e-15 | e-37 | A-9 |
| b-120 | b-140 | b-157 | b-175 | b-196 | e-16 | e-38 | A-10 |
| b-121 | b-141 | b-159 | b-176 | b-197 | e-20 | e-39 | A-11 |
| b-122 | b-142 | b-160 | b-177 | b-198 | e-21 | e-41 | A-12 |
| b-123 | b-143 | b-161 | b-178 | b-199 | e-22 | e-42 | A-13 |
| b-124 | b-144 | b-162 | b-179 | d-11 | e-23 | e-43 | A-14 |
| b-125 | b-145 | b-163 | b-180 | d-12 | e-24 | e-47 | A-16 |
| b-126 | b-146 | b-164 | b-181 | d-13 | e-25 | e-48 | |

In addition, the same test as described above was also carried out for Compound No. P1.4 described in Patent Document 1 (WO 2017/093409 A). The compound mentioned above exhibited an 80% or more controlling rate at 125 ppm.

On the other hand, the compounds of Compound No. b-198 and Compound No. b-184 demonstrated an 80% or more controlling rate even at 7.8 ppm. In addition, the compound of Compound No. b-160 demonstrated an 80% or more controlling rate even at 1.9 ppm.

[Chem. 82]

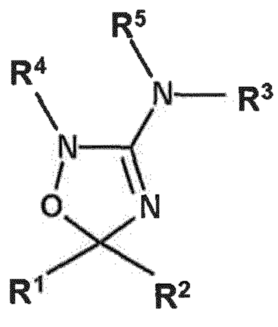

Compound No.P1.4

(In the formula, $R^1$ and $R^2$ are Ph, $R^3$ is t-Bu, and $R^4$ is Me.)

(Test Example 4) Efficacy test against *Thrips tabaci*

Leaf disks of *Phaseolus vulgaris* were inoculated with 8 nymphs of *Thrips tabaci.* Emulsion (I) was diluted with water so that the concentration of the compound of the present invention was 125 ppm. Subsequently, the aforementioned diluted liquid was sprayed on the leaf disks of *Phaseolus vulgaris,* and then air dried. The number of the parasitic larvae

was counted 7 days after spraying. The efficacy of the compounds was evaluated by the controlling rate described below.

$$\text{Controlling rate (\%)} = \{1 - (\text{Nt})/(\text{Nc})\} \times 100$$

In which Nt: number of parasites at the legion spray-treated.
Nc: number of parasites at the legion non-treated (control).

The efficacy test against *Thrips tabaci* was carried out for the compounds shown in Table 16-1 and Table 16-2. All of the compounds shown in Table 16-1 and Table 16-2 demonstrated an 80% or more controlling rate against *Thrips tabaci.*

[Table 16-1]

Table 16-1

| Compound No. | Compound No. | Compound No. |
|---|---|---|
| a-1 | a-15 | b-78 |
| a-4 | a-23 | b-100 |
| a-5 | b-21 | b-101 |
| a-8 | b-48 | b-102 |
| a-9 | b-52 | b-104 |
| a-10 | b-53 | |
| a-11 | b-54 | |
| a-13 | b-59 | |

[Table 16-2]

Tble 16-2

| Compound No. | | | | | |
|---|---|---|---|---|---|
| b-109 | b-141 | b-165 | e-14 | e-34 | A-5 |
| b-135 | b-142 | b-174 | e-15 | e-42 | A-6 |
| b-136 | b-143 | b-181 | e-16 | e-43 | A-7 |
| b-137 | b-159 | b-183 | e-20 | A-1 | A-8 |
| b-138 | b-160 | b-184 | e-21 | A-2 | A-9 |
| b-139 | b-161 | b-193 | e-22 | A-3 | A-10 |
| b-140 | b-162 | b-194 | e-23 | A-4 | A-14 |

(Test Example 5) Drenching test in cucumber seedling-planted pot against *Thrips palmi (Frankliniella occidentalis)*

Cucumber seedlings were inoculated with 8 adults of *Thrips palmi.* Emulsion (I) was diluted with water so that the concentration of the compound of the present invention was 500 ppm. Subsequently, 10 mL of the aforementioned diluted liquid was drenched into the cucumber seedlings, and then air dried. The number of the parasites was counted 7 days after spraying. The efficacy of the compounds was evaluated by the controlling rate described below.

$$\text{Controlling rate (\%)} = \{1 - (\text{Nt})/(\text{Nc})\} \times 100$$

In which Nt: number of parasites at the legion spray-treated.
Nc: number of parasites at the legion non-treated (control).

The efficacy test against *Thrips palmi* was carried out for the compound of Compound No. a-10. The compound mentioned above demonstrated an 80% or more controlling rate against *Thrips palmi.*

(Test Example 6) Efficacy test against *Scirtothrips dorsalis*

Leaf disks of tea leaves were inoculated with 8 larvae of *Scirtothrips dorsalis.* Emulsion (I) was diluted with water so that the concentration of the compound of the present invention was 125 ppm. Subsequently, the aforementioned diluted liquid was sprayed on the leaf disks of tea leaves, and then air dried. The number of the parasites was counted 3 days after spraying. The efficacy of the compounds was evaluated by the controlling rate described below.

$$\text{Controlling rate (\%)} = \{1 - (\text{Nt})/(\text{Nc})\} \times 100$$

In which Nt: number of parasites at the legion spray-treated.
Nc: number of parasites at the legion non-treated (control).

The efficacy test against *Scirtothrips dorsalis* was carried out for the compounds of Compound No. a-1 and Compound No. a-10. Both the compounds demonstrated an 80% or more controlling rate against *Scirtothrips dorsalis.*

(Test Example 7) Efficacy test against *Frankliniella intonsa*

Leaf disks of black-eyed pea plants were inoculated with 8 larvae of *Frankliniella intonsa.* Emulsion (I) was diluted with water so that the concentration of the compound of the present invention was 125 ppm. Subsequently, the aforementioned diluted liquid was sprayed on the leaf disks of black-eyed pea plants, and then air dried. The number of the parasites was counted 3 days after spraying. The efficacy of the compounds was evaluated by the controlling rate described below.

$$\text{Controlling rate (\%)} = \{1 - (\text{Nt})/(\text{Nc})\} \times 100$$

In which Nt: number of parasites at the legion spray-treated.
Nc: number of parasites at the legion non-treated (control).

The efficacy test against *Frankliniella intonsa* was carried out for the compound of Compound No. a-10. The compound mentioned above demonstrated an 80% or more controlling rate against *Frankliniella intonsa.*

(Test Example 8) Penetration test against *Thrips palmi* (*Frankliniella occidentalis*)

Emulsion (I) was diluted with water so that the concentration of the compound of the present invention was 125 ppm. Only the surface of the leaves of cucumber seedlings were sprayed with the diluted emulsion, and air dried. Subsequently, leaf disks thereof were prepared, and the rear face thereof was inoculated with 8 adults of *Thrips palmi.*

The number of the parasites was counted 2 days after inoculation. The efficacy of the compounds was evaluated by the controlling rate described below.

$$\text{Controlling rate (\%)} = \{1 - (\text{Nt})/(\text{Nc})\} \times 100$$

In which Nt: number of parasites at the legion spray-treated.
Nc: number of parasites at the legion non-treated (control).

The penetration test against *Thrips palmi* was carried out for the compounds of Compound No. a-10 and Compound No. a-12. Both the compounds mentioned above demonstrated an 80% or more controlling rate against *Thrips palmi.*

(Test Example 9) Efficacy test against *Thrips palmi* (*Frankliniella occidentalis*) using cotton seedlings planted in pots

Cotton seedlings planted in pots for raising seedlings were inoculated with 8 adults of *Thrips palmi.* Emulsion (I) was diluted with water so that the concentration of the compound of the present invention was 125 ppm. Subsequently,

the aforementioned diluted liquid was sprayed on the cotton seedlings mentioned above. The number of the parasitic larvae was counted 3 days after spraying. The efficacy of the compounds was evaluated by the controlling rate described below.

$$\text{Controlling rate (\%)} = \{1 - (\text{Nt})/(\text{Nc})\} \times 100$$

In which Nt: number of parasites at the legion spray-treated.
Nc: number of parasites at the legion non-treated (control).

The efficacy test against *Thrips palmi* was carried out for the compound of Compound No. a-10. The compound mentioned above demonstrated an 80% or more controlling rate against *Thrips palmi.*

(Test Example 10) Drenching test for cotton seedlings against *Thrips palmi* (*Frankliniella occidentalis*)

Cotton seedlings planted in a cell tray were inoculated with 10 adults of *Thrips palmi.* Emulsion (I) was diluted with water so that the concentration of the compound of the present invention was 500 ppm. Subsequently, 7 mL of the aforementioned diluted liquid was drenched into the cotton seedlings mentioned above. The number of the parasites was counted 7 days after inoculation. The efficacy of the compounds was evaluated by the controlling rate described below.

$$\text{Controlling rate (\%)} = \{1 - (\text{Nt})/(\text{Nc})\} \times 100$$

In which Nt: number of parasites at the legion spray-treated.
Nc: number of parasites at the legion non-treated (control).

The drenching test for cotton seedlings against *Thrips palmi* was carried out for the compound of Compound No. a-10. The compound mentioned above demonstrated an 80% or more controlling rate against *Thrips palmi.*

The compounds selected at random among the oxadiazoline compounds according to the present invention exhibit the effects described above. For this reason, it can be understood that the oxadiazoline compounds of the present invention including those which cannot be demonstrated above have effects of controlling harmful organisms, and in particular, acaricidal effects, insecticidal effects and the like. In addition, it can also be understood that the oxadiazoline compounds of the present invention have effects on ectoparasites and the like which harm humans and animals.

## Claims

1. A compound represented by formula (I) or a salt thereof

[Chem. 1]

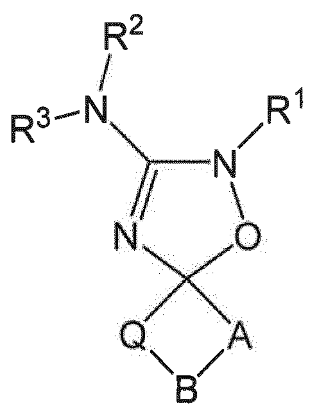

(I)

[in formula (I),

$R^1$ represents a hydrogen atom, a substituted or unsubstituted C1-6 alkyl group, a substituted or unsubstituted C2-6 alkenyl group, a substituted or unsubstituted C6-10 aryl C1-6 alkyl group, or a substituted or unsubstituted C1-6 alkyl carbonyl group,
$R^2$ represents a substituted or unsubstituted C1-8 alkyl group, a substituted or unsubstituted C3-10 cycloalkyl group, a substituted or unsubstituted C6-10 aryl group, a substituted or unsubstituted C6-10 aryl C1-6 alkyl group, or a substituted or unsubstituted C1-6 alkoxy carbonyl group,
$R^3$ represents a hydrogen atom, a substituted or unsubstituted C1-6 alkyl group, a substituted or unsubstituted C2-6 alkenyl group, a substituted or unsubstituted C2-6 alkynyl group, a substituted or unsubstituted C6-10 aryl C1-6 alkyl group, a substituted or unsubstituted C1-6 alkyl carbonyl group, or a substituted or unsubstituted C1-6 alkoxy carbonyl group,
$R^2$ and $R^3$ may bind together to form a substituted or unsubstituted C3-5 alkylene group,
A represents a substituted or unsubstituted o-phenylene group, a substituted or unsubstituted 5- to 6-membered heteroarylene group, a substituted or unsubstituted benzylene group, a substituted or unsubstituted dimethylene group, or a 1,2-cyclopropylene group,
B represents a single bond, an oxy group, a substituted or unsubstituted oxymethylene group, a substituted or unsubstituted methyleneoxy group, a substituted or unsubstituted thiomethylene group, a substituted or unsubstituted methylenethio group, a substituted or unsubstituted methylene group, a substituted or unsubstituted dimethylene group, or a substituted or unsubstituted vinylene group, a thio group, a substituted or unsubstituted sulfonylmethylene group, a substituted or unsubstituted methylenesulfonyl group, a substituted or unsubstituted trimethylene group, a substituted or unsubstituted oxyethylene group, a substituted or unsubstituted ethyleneoxy group, a substituted or unsubstituted propenylene group, a substituted or unsubstituted oxymethyleneoxy group, a group represented by a formula: $-NR^a-$, a group represented by a formula: $-CH_2-NR^a-$, or a group represented by a formula: $-NR^a-CH_2-$,
$R^a$ represents a hydrogen atom or a substituted or unsubstituted C1-6 alkyl group, and
Q represents a substituted or unsubstituted o-phenylene group.]

**2.** The compound according to Claim 1, wherein

A is a substituted or unsubstituted o-phenylene group, and
B is a substituted or unsubstituted oxymethylene group, a substituted or unsubstituted methyleneoxy group, a substituted or unsubstituted dimethylene group, or a substituted or unsubstituted vinylene group.

**3.** The compound according to Claim 1, wherein

A is a substituted or unsubstituted o-phenylene group, and
B is a substituted or unsubstituted oxymethylene group, or a substituted or unsubstituted methyleneoxy group.

**4.** A formulation for controlling harmful organisms, comprising at least one compound selected from the group consisting of the compounds as recited in any one of Claims 1 to 3, and salts thereof, as an active ingredient.

**5.** An insecticidal or acaricidal formulation, comprising at least one compound selected from the group consisting of the compounds as recited in any one of Claims 1 to 3, and salts thereof, as an active ingredient.

**6.** A formulation for controlling ectoparasites, comprising at least one compound selected from the group consisting of the compounds as recited in any one of Claims 1 to 3, and salts thereof, as an active ingredient.

**7.** A formulation for controlling *Thysanoptera* insect pests, comprising at least one compound selected from the group consisting of the compounds as recited in any one of Claims 1 to 3, and salts thereof, as an active ingredient.

## INTERNATIONAL SEARCH REPORT

International application No.
PCT/JP2018/022389

A. CLASSIFICATION OF SUBJECT MATTER
Int.Cl. C07D271/12(2006.01)i, A01N43/836(2006.01)i, A01P7/02(2006.01)i, A01P7/04(2006.01)i, A61K31/4245(2006.01)i, A61P33/14(2006.01)i, C07D498/10(2006.01)i

According to International Patent Classification (IPC) or to both national classification and IPC

B. FIELDS SEARCHED

Minimum documentation searched (classification system followed by classification symbols)
Int.Cl. C07D271/12, A01N43/836, A01P7/02, A01P7/04, A61K31/4245, A61P33/14, C07D498/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | WO 2017/093409 A1 (SYNGENTA PARTICIPATIONS AG) 08 June 2017, entire text (Family: none) | 1-7 |
| A | WO 2010/105179 A2 (VITAE PHARMACEUTICALS, INC.) 16 September 2010, entire text & JP 2012-520324 A & JP 2015-147792 A & US 2011/0071126 A1 & US 2014/0200223 A1 & EP 2801570 A1 & CN 102348698 A & KR 10-2017-0127048 A & KR 10-2012-0001756 A | 1-7 |
| A | WO 2010/021680 A2 (VITAE PHARMACEUTICALS, INC.) 25 February 2010, entire text & US 2011/0218192 A1 & US 2013/0317014 A1 & EP 2324032 A1 | 1-7 |

☐ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 September 2018 (03.09.2018) | 11 September 2018 (11.09.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017093409A PCT
- WO 2017093409 A


### Non-patent literature cited in the description


- **COLBY. S. R.** Calculating Synergistic and Antagonistic Responses of Herbicide Combinations. *Weeds,* 1967, vol. 15, 20-22
- *Chemical and Pharmaceutical Bulletin,* 1991, vol. 39 (0), 2564
- *Angewandte Chemie - International Edition,* 2015, vol. 54 (17), 5049
- *J. Med. Chem.,* 1992, vol. 35, 2481